# EUROPEAN PATENT APPLICATION

(11) **EP 4 796 564 A1**
(43) Date of publication of application: **26.08.2026**
(21) Application number: 24879070.1
(22) Date of filing: 17.10.2024
(51) Int. Cl.: C07K 16/46, C12N 15/13, A61K 39/395, A61P 35/00, A61P 35/02

(54) **BISPECIFIC ANTIBODY AGAINST CCR8 AND CTLA4**

(30) Priority: 17.10.2023 CN 202311346588
(71) Applicant: Biontech (Zhuhai) Pharmaceuticals R&D Co., Ltd, Zhuhai, Guangdong 519080 (CN)
(72) Inventor: CHEN, Liandi, Zhuhai, Guangdong 519080 (CN); LUO, Yi, Zhuhai, Guangdong 519080 (CN); MIAO, Xiaoniu, Zhuhai, Guangdong 519080 (CN); ZHAI, Tianhang, Zhuhai, Guangdong 519080 (CN); HUANG, Weifeng, Zhuhai, Guangdong 519080 (CN); TSUN, Andy, Zhuhai, Guangdong 519080 (CN)
(74) Representative: Maiwald GmbH
(86) International application number: PCT/CN2024/125440
(87) International publication number: WO 2025/082429

(57) **Abstract**

An antibody or an antigen-binding fragment thereof and a single-domain antibody or an antigen-binding fragment thereof that can specifically bind to CTLA4, and an immunoconjugate, a pharmaceutical composition and a multispecific molecule comprising the antibody or the antigen-binding fragment thereof. Furthermore, the present invention also relates to a multispecific molecule that can specifically bind to CCR8 and CTLA4 and the use thereof.

## Description

### CROSS-REFERENCE TO RELATED APPLICATIONS

The present application claims priority to Chinese Patent Application No. 202311346588.1 filed on October 17, 2023, the entire contents of which are incorporated herein by reference in their entirety.

### TECHNICAL FIELD

The present application belongs to the field of biomedical technology, and more specifically, relates to antibodies or antigen-binding fragments thereof and single domain antibodies or antigen-binding fragments thereof, capable of specifically binding to CTLA4. The present application also relates to immunoconjugates, pharmaceutical compositions, and multispecific molecules comprising the antibodies or antigen-binding fragments thereof. Furthermore, the present application also relates to multispecific molecules capable of specifically binding to CCR8 and CTLA4, and uses thereof.

### BACKGROUND

Regulatory T (Treg) cells play a crucial role in maintaining homeostasis and self-tolerance. A large number of Treg cells is present in the tumor microenvironment, which hinders anti-tumor immune responses and immune surveillance, thereby promoting tumor occurrence and development. Currently, many Treg-targeted therapies are in clinical studies. However, many drugs show limited efficacy and/or high systemic toxicity due to the difficulty in selectively targeting tumor-infiltrating Treg cells.

CCR8, a chemokine receptor, has been found to be mainly expressed on tumor-associated Tregs. However, other effector T cell populations have also reported to express CCR8, which increases uncertainty about the elimination of CCR8-positive cells. To specifically target tumor Tregs, CTLA-4, which is constitutively expressed on tumor-infiltrating Tregs, was selected as a target pair with CCR8 to generate an IgG-like bispecific antibody. The IgG-like bispecific antibody preferentially eliminates CCR8 x CTLA-4 double-positive tumor-infiltrating Treg cells with minimal interference to single-positive cells. Consequently, the bispecific antibody has stronger anti-tumor activity and limited immunotoxicity, and may be safer, more specific, and more efficient in clearing tumor-infiltrating Tregs, as compared to the corresponding monospecific antibody drugs.

In summary, there is an urgent need in the art to develop a bispecific antibody targeting CCR8 x CTLA-4 on Treg cells to improve antibody specificity and safety, and enhance tumor treatment efficacy.

### SUMMARY OF THE INVENTION

In summary, in order to specifically target tumor Tregs, the present application selects CTLA-4, which is constitutively expressed on Tregs, as a target pair with CCR8, and develops monoclonal antibodies/nanobodies. The present application further generates IgG-like bispecific antibodies to preferentially eliminate CCR8 x CTLA-4 double-positive tumor-infiltrating Treg cells with minimal interference to single-positive cells.

### CTLA4 Monoclonal Antibodies

In a first aspect, the present application provides an antibody or antigen-binding fragment thereof capable of specifically binding to CTLA4, comprising:
(a) a heavy chain variable region (VH) comprising the following three complementarity determining regions (CDRs):
   (i) a VH CDR1, consisting of the sequence set forth in SEQ ID NO: 48 or 54, or a sequence having one or several amino acid substitutions, deletions, or additions (e.g., 1, 2, or 3 amino acid substitutions, deletions, or additions) as compared to SEQ ID NO: 48 or 54,
   (ii) a VH CDR2, consisting of the sequence set forth in SEQ ID NO: 49 or 55, or a sequence having one or several amino acid substitutions, deletions, or additions (e.g., 1, 2, or 3 amino acid substitutions, deletions, or additions) as compared to SEQ ID NO: 49 or 55, and
   (iii) a VH CDR3, consisting of the sequence set forth in SEQ ID NO: 50 or 56, or a sequence having one or several amino acid substitutions, deletions, or additions (e.g., 1, 2, or 3 amino acid substitutions, deletions, or additions) as compared to SEQ ID NO: 50 or 56;
      and/or,
(b) a light chain variable region (VL) comprising the following three complementarity determining regions (CDRs):
   (iv) a VL CDR1, consisting of the sequence of SEQ ID NO: 51 or 57, or a sequence having one or several amino acid substitutions, deletions, or additions (e.g., 1, 2, or 3 amino acid substitutions, deletions, or additions) as compared to SEQ ID NO: 51 or 57,
   (v) a VL CDR2, consisting of the sequence of SEQ ID NO: 52 or 58, or a sequence having one or several amino acid substitutions, deletions, or additions (e.g., 1, 2, or 3 amino acid substitutions, deletions, or additions) as compared to SEQ ID NO: 52 or 58, and
   (vi) a VL CDR3, consisting of the sequence of SEQ ID NO: 53 or 59, or a sequence having one or several amino acid substitutions, deletions, or additions (e.g., 1, 2, or 3 amino acid substitutions, deletions, or additions) as compared to SEQ ID NO: 53 or 59.

In certain embodiments, the substitution in any one of (i)-(vi) is a conservative substitution.

In certain embodiments, the CDRs in any one of (i)-(vi) are defined according to the Kabat, IMGT, or Chothia numbering system.

In certain embodiments, the CDRs in any one of (i)-(vi) are defined according to the IMGT numbering system.

In certain embodiments, the antibody or antigen-binding fragment thereof as previously described comprises: the following three heavy chain CDRs: a VH CDR1 as set forth in SEQ ID NO: 48, a VH CDR2 as set forth in SEQ ID NO: 49, a VH CDR3 as set forth in SEQ ID NO: 50; and/or, the following three light chain CDRs: a VL CDR1 as set forth in SEQ ID NO: 51, a VL CDR2 as set forth in SEQ ID NO: 52, a VL CDR3 as set forth in SEQ ID NO: 53.

In certain embodiments, the antibody or antigen-binding fragment thereof as previously described comprises: the following three heavy chain CDRs: VH CDR1 as set forth in SEQ ID NO: 54, VH CDR2 as set forth in SEQ ID NO: 55, VH CDR3 as set forth in SEQ ID NO: 56; and/or, the following three light chain CDRs: VL CDR1 as set forth in SEQ ID NO: 57, VL CDR2 as set forth in SEQ ID NO: 58, VL CDR3 as set forth in SEQ ID NO: 59.

In certain embodiments, the antibody or antigen-binding fragment thereof further comprises a framework region of a human immunoglobulin.

In certain embodiments, the antibody or antigen-binding fragment thereof as previously described comprises:
(a) a heavy chain variable region (VH) comprising an amino acid sequence selected from the group consisting of:
   (i) the sequence set forth in SEQ ID NO: 3 or 5;
   (ii) a sequence having one or several amino acid substitutions, deletions, or additions (e.g., 1, 2, 3, 4, or 5 amino acid substitutions, deletions, or additions) as compared to the sequence set forth in SEQ ID NO: 3 or 5; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 3 or 5; and/or
(b) a light chain variable region (VL) comprising an amino acid sequence selected from the group consisting of:
   (iv) the sequence set forth in SEQ ID NO: 4 or 6;
   (v) a sequence having one or several amino acid substitutions, deletions, or additions (e.g., 1, 2, 3, 4, or 5 amino acid substitutions, deletions, or additions) as compared to the sequence set forth in SEQ ID NO: 4 or 6; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 4 or 6.

In certain embodiments, the substitution in (ii) or (v) is a conservative substitution.

In certain embodiments, the antibody or antigen-binding fragment thereof comprises:
(1) a VH having the sequence set forth in SEQ ID NO: 3 and a VL having the sequence set forth in SEQ ID NO: 4; or,
(2) a VH having the sequence set forth in SEQ ID NO: 5 and a VL having the sequence set forth in SEQ ID NO: 6.

### CTLA4 Single Domain Antibodies

In a second aspect, the present application provides a single domain antibody or antigen-binding fragment thereof capable of specifically binding to CTLA4, comprising:
(a) a CDR1 having: the sequence set forth in any one of SEQ ID NOs: 60, 63, 66, or 69, or a sequence having one or several amino acid substitutions, deletions, or additions (e.g., 1, 2, or 3 amino acid substitutions, deletions, or additions) as compared to the sequence set forth in any one of SEQ ID NOs: 60, 63, 66, or 69;
(b) a CDR2 having: the sequence set forth in any one of SEQ ID NOs: 61, 64, 67, or 70, or a sequence having one or several amino acid substitutions, deletions, or additions (e.g., 1, 2, or 3 amino acid substitutions, deletions, or additions) as compared to the sequence set forth in any one of SEQ ID NOs: 61, 64, 67, or 70; and
(c) a CDR3 having: the sequence set forth in any one of SEQ ID NOs: 62, 65, 68, or 71, or a sequence having one or several amino acid substitutions, deletions, or additions (e.g., 1, 2, or 3 amino acid substitutions, deletions, or additions) as compared to the sequence set forth in any one of SEQ ID NOs: 62, 65, 68, or 71.

In certain embodiments, the substitution is a conservative substitution. In certain embodiments, the CDRs are defined according to the Kabat, IMGT, or Chothia numbering system.

In certain embodiments, the CDRs are defined according to the IMGT numbering system.

In certain embodiments, the antibody or antigen-binding fragment thereof as previously described comprises: a CDR1 as set forth in SEQ ID NO: 60; a CDR2 as set forth in SEQ ID NO: 61; and a CDR3 as set forth in SEQ ID NO: 62.

In certain embodiments, the antibody or antigen-binding fragment thereof as previously described comprises: a CDR1 as set forth in SEQ ID NO: 63; a CDR2 as set forth in SEQ ID NO: 64; and a CDR3 as set forth in SEQ ID NO: 65.

In certain embodiments, the antibody or antigen-binding fragment thereof as previously described comprises: a CDR1 as set forth in SEQ ID NO: 66; a CDR2 as set forth in SEQ ID NO: 67; and a CDR3 as set forth in SEQ ID NO: 68.

In certain embodiments, the antibody or antigen-binding fragment thereof as previously described comprises: a CDR1 as set forth in SEQ ID NO: 69; a CDR2 as set forth in SEQ ID NO: 70; and a CDR3 as set forth in SEQ ID NO: 71.

In certain embodiments, the antibody or antigen-binding fragment thereof as previously described comprises an amino acid sequence selected from the group consisting of:
the sequence set forth in any one of SEQ ID NOs: 7, 8, 9, or 10;
a sequence having one or several amino acid substitutions, deletions, or additions (e.g., 1, 2, 3, 4, or 5 amino acid substitutions, deletions, or additions) as compared to the sequence set forth in any one of SEQ ID NOs: 7, 8, 9, or 10; or
a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in any one of SEQ ID NOs: 7, 8, 9, or 10.

In certain embodiments, the substitution is a conservative substitution.

### CCR8 Monoclonal Antibodies

In another aspect, the present application provides an antibody or antigen-binding fragment thereof capable of specifically binding to CCR8, comprising:
(a) a heavy chain variable region (VH) comprising the following three complementarity determining regions (CDRs):
   (i) a VH CDR1, consisting of the sequence set forth in SEQ ID NO: 137, or a sequence having one or several amino acid substitutions, deletions, or additions (e.g., 1, 2, or 3 amino acid substitutions, deletions, or additions) as compared to SEQ ID NO: 137,
   (ii) a VH CDR2, consisting of the sequence set forth in SEQ ID NO: 138, or a sequence having one or several amino acid substitutions, deletions, or additions (e.g., 1, 2, or 3 amino acid substitutions, deletions, or additions) as compared to SEQ ID NO: 138, and
   (iii) a VH CDR3, consisting of the sequence set forth in SEQ ID NO: 139, or a sequence having one or several amino acid substitutions, deletions, or additions (e.g., 1, 2, or 3 amino acid substitutions, deletions, or additions) as compared to SEQ ID NO: 139; and/or,
(b) a light chain variable region (VL) comprising the following three complementarity determining regions (CDRs):
   (iv) a VL CDR1, consisting of the sequence of SEQ ID NO: 75, or a sequence having one or several amino acid substitutions, deletions, or additions (e.g., 1, 2, or 3 amino acid substitutions, deletions, or additions) as compared to SEQ ID NO: 75,
   (v) a VL CDR2, consisting of the sequence of SEQ ID NO: 76, or a sequence having one or several amino acid substitutions, deletions, or additions (e.g., 1, 2, or 3 amino acid substitutions, deletions, or additions) as compared to SEQ ID NO: 76, and
   (vi) a VL CDR3, consisting of the sequence of SEQ ID NO: 77, or a sequence having one or several amino acid substitutions, deletions, or additions (e.g., 1, 2, or 3 amino acid substitutions, deletions, or additions) as compared to SEQ ID NO: 77.

In certain embodiments, the substitution in any one of (i)-(vi) is a conservative substitution.

In certain embodiments, the CDRs in any one of (i)-(vi) are defined according to the Kabat, IMGT, or Chothia numbering system.

In certain embodiments, the CDRs in any one of (i)-(vi) are defined according to the IMGT numbering system.

In certain embodiments, the antibody or antigen-binding fragment thereof as previously described comprises:
the following three heavy chain CDRs: a VH CDR1 as set forth in SEQ ID NO: 137, a VH CDR2 as set forth in SEQ ID NO: 138, a VH CDR3 as set forth in SEQ ID NO: 139; and/or, the following three light chain CDRs: a VL CDR1 as set forth in SEQ ID NO: 75, a VL CDR2 as set forth in SEQ ID NO: 76, a VL CDR3 as set forth in SEQ ID NO: 77.

In certain embodiments, the antibody or antigen-binding fragment thereof further comprises a framework region of a human immunoglobulin.

In certain embodiments, the antibody or antigen-binding fragment thereof as previously described comprises:
(a) a heavy chain variable region (VH) comprising an amino acid sequence selected from the group consisting of:
   (i) the sequence set forth in SEQ ID NO: 78 or 1;
   (ii) a sequence having one or several amino acid substitutions, deletions, or additions (e.g., 1, 2, 3, 4, or 5 amino acid substitutions, deletions, or additions) as compared to the sequence set forth in SEQ ID NO: 78 or 1; or
   (iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 78 or 1; and/or
(b) a light chain variable region (VL) comprising an amino acid sequence selected from the group consisting of:
   (iv) a sequence set forth in SEQ ID NO: 2;
   (v) a sequence having one or several amino acid substitutions, deletions, or additions (e.g., 1, 2, 3, 4, or 5 amino acid substitutions, deletions, or additions) as compared to the sequence set forth in SEQ ID NO: 2; or
   (vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 2.

In certain embodiments, the substitution in (ii) or (v) is a conservative substitution.

In certain embodiments, the antibody or antigen-binding fragment thereof comprises: a VH having the sequence set forth in SEQ ID NO: 78 or 1 and a VL having the sequence set forth in SEQ ID NO: 2.

In certain embodiments, the antibody or antigen-binding fragment thereof as previously described further comprises a constant region derived from a human immunoglobulin.

In certain embodiments, the heavy chain of the antibody or antigen-binding fragment thereof comprises a heavy chain constant region derived from a human immunoglobulin (e.g., IgG1, IgG2, IgG3, or IgG4) or a variant thereof.

In certain embodiments, the heavy chain constant region has a LALA mutation and/or a knob-into-hole modification.

In certain embodiments, the heavy chain constant region has the sequence set forth in any one of SEQ ID NOs: 12-17, 79-82.

In certain embodiments, the light chain of the antibody or antigen-binding fragment thereof comprises a light chain constant region derived from a human immunoglobulin (e.g., κ or λ).

In certain embodiments, the light chain constant region has the sequence set forth in any one of SEQ ID NOs: 11, 81, 82.

In certain embodiments, the antibody or antigen-binding fragment thereof is hypofucosylated or afucosylated.

In certain embodiments, the antibody or antigen-binding fragment thereof as previously described, wherein the antigen-binding fragment is selected from Fab, Fab', (Fab')2, Fv, disulfide-linked Fv, scFv, diabody, and single domain antibody (sdAb); and/or, the antibody is a murine antibody, a chimeric antibody, a humanized antibody, or a multispecific antibody.

In another aspect, the present application provides an isolated nucleic acid molecule encoding the antibody or antigen-binding fragment thereof as previously described or the single domain antibody or antigen-binding fragment thereof as previously described.

In another aspect, the present application provides a vector comprising the nucleic acid molecule as previously described. In certain embodiments, the vector is a cloning vector or an expression vector.

In another aspect, the present application provides a host cell comprising the nucleic acid molecule as previously described or the vector as previously described.

In certain embodiments, the host cell is a mammalian cell.

In certain embodiments, the host cell has low or no fucosylation activity, for example selected from a mammalian cell (e.g., CHO cell) lacking expression of a gene encoding fucosyltransferase.

In another aspect, the present application provides a method of preparing the antibody or antigen-binding fragment thereof as previously described or the single domain antibody or antigen-binding fragment thereof as previously described, comprising culturing the host cell as previously described under conditions that allow expression of the antibody or antigen-binding fragment thereof, and recovering the antibody or antigen-binding fragment thereof from the cultured host cell culture.

In certain embodiments, the host cell has low or no fucosylation activity, for example selected from a mammalian cell (e.g., CHO cell) lacking expression of a gene encoding fucosyltransferase.

### Bispecific Antibodies

In another aspect, the present application provides a multispecific antibody (e.g., a bispecific antibody) comprising the antibody or antigen-binding fragment thereof as previously described.

To specifically target tumor Tregs, CTLA-4, which is constitutively expressed on tumor-infiltrating Tregs, is selected as a target pair with CCR8 to generate an IgG-like bispecific antibody. The IgG-like bispecific antibody preferentially eliminates CCR8 x CTLA-4 double-positive tumor-infiltrating Treg cells with minimal interference to single-positive cells. Therefore, the bispecific antibody has stronger anti-tumor activity and limited immunotoxicity, and may be safer, more specific, and more efficient in clearing tumor-infiltrating Tregs, as compared to the corresponding monospecific antibody drugs.

In another aspect, the present application provides a multispecific antibody (e.g., a bispecific antibody) that specifically binds to CCR8 and CTLA4, comprising a first antigen-binding domain specific for CTLA4 and a second antigen-binding domain specific for CCR8.

In certain embodiments, the first antigen-binding domain comprises: the antibody or antigen-binding fragment thereof that specifically binds to CTLA4 as previously described.

In certain embodiments, the first antigen-binding domain comprises: the single domain antibody or antigen-binding fragment thereof that specifically binds to CTLA4 as previously described.

In certain embodiments, the second antigen-binding domain comprises the antibody or antigen-binding fragment thereof as previously described that specifically binds to CCR8.

In certain embodiments, the first antigen-binding domain is a Fab fragment, a scFv, or a VHH, and the second antigen-binding domain is a Fab fragment, a scFv, or a VHH.

In certain embodiments, the first antigen-binding domain is a Fab fragment or a VHH, and the second antigen-binding domain is a Fab fragment.

In certain embodiments, the multispecific antibody as previously described further comprises an Fc domain comprising a first monomer and a second monomer.

In certain embodiments, the first antigen-binding domain and the second antigen-binding domain are each linked to the N-terminus of the first monomer and the second monomer of the Fc domain, optionally via a linker.

In certain embodiments, the Fc domain comprises a modification to promote dimerization of the first monomer and the second monomer.

In certain embodiments, the modification comprises an amino acid substitution in the CH3 domain of the Fc domain.

In certain embodiments, the Fc domain comprises a modification to promote controlled Fab-arm Exchange (cFAE).

In certain embodiments, the modification comprises an amino acid substitution in the CH3 domain of the Fc domain.

In certain embodiments, the modification comprises a "knob" modification in one of the two monomers of the Fc domain and a "hole" modification in the other of the two monomers of the Fc domain to form a "knob-into-hole" modification.

In certain embodiments, the two monomers of the Fc domain comprise the amino acid sequences set forth in SEQ ID NOs: 16 and 17, respectively.

In certain embodiments, the modification comprises a substitution selected from F405L and/or R411T in one of the two monomers of the Fc domain and a substitution selected from T370K and/or K409R in the other of the two monomers of the Fc domain to form a modification that promotes cFAE.

In certain embodiments, the two monomers of the Fc domain comprise the amino acid sequences set forth in SEQ ID NOs: 13 and 14, respectively, or comprise the amino acid sequences set forth in SEQ ID NOs: 79 and 80, respectively.

In certain embodiments, the multispecific antibody is hypofucosylated or afucosylated.

In certain embodiments, the first antigen-binding domain is a Fab fragment, the second antigen-binding domain is a Fab fragment, and the multispecific antibody comprises:
(i) a first peptide chain comprising a VL of the first antigen-binding domain and a first light chain constant region (CL); preferably, the first CL is kappa;
(ii) a second peptide chain comprising a VH of the first antigen-binding domain and a first heavy chain constant region (CH); preferably, the first CH is IgG, e.g., IgG1, IgG2, IgG3, or IgG4;
(iii) a third peptide chain comprising a VH of the second antigen-binding domain and a second heavy chain constant region (CH); preferably, the second CH is IgG, e.g., IgG1, IgG2, IgG3, or IgG4;
(iv) a fourth peptide chain comprising a VL of the second antigen-binding domain and a second light chain constant region (CL); preferably, the second CL is kappa.

In certain embodiments, the Fc domain monomers of the first CH and the second CH form a dimer. In certain embodiments, the Fc domain comprises a modification as defined above.

In certain embodiments, the first CH and first CL, and the second CH and second CL are each capable of forming a dimer.

In certain embodiments, the multispecific antibody comprises:
(i) a first peptide chain comprising a VL of the sequence set forth in SEQ ID NO: 4 and a CL of the sequence set forth in SEQ ID NO: 11, 82, 81, 143;
(ii) a second peptide chain comprising a VH of the sequence set forth in SEQ ID NO: 3 and a CH of the sequence set forth in SEQ ID NO: 13, 80, 79, 140;
(iii) a third peptide chain comprising a VH of the sequence set forth in SEQ ID NO: 1, 78 and a CH of the sequence set forth in SEQ ID NO: 14, 79, 80, 141;
(iv) a fourth peptide chain comprising a VL of the sequence set forth in SEQ ID NO: 2 and a CL of the sequence set forth in SEQ ID NO: 11, 81, 82, 143.

In certain embodiments, the multispecific antibody comprises a first peptide chain comprising the sequence set forth in SEQ ID NO: 29, a second peptide chain comprising the sequence set forth in SEQ ID NO: 28, a third peptide chain comprising the sequence set forth in SEQ ID NO: 30, and a fourth peptide chain comprising the sequence set forth in SEQ ID NO: 31.

In certain embodiments, the multispecific antibody comprises a first peptide chain comprising the sequence set forth in SEQ ID NO: 86, a second peptide chain comprising the sequence set forth in SEQ ID NO: 85, a third peptide chain comprising the sequence set forth in SEQ ID NO: 87, and a fourth peptide chain comprising the sequence set forth in SEQ ID NO: 88.

In certain embodiments, the multispecific antibody comprises a first peptide chain comprising the sequence set forth in SEQ ID NO: 96, a second peptide chain comprising the sequence set forth in SEQ ID NO: 95, a third peptide chain comprising the sequence set forth in SEQ ID NO: 93, and a fourth peptide chain comprising the sequence set forth in SEQ ID NO: 94.

In certain embodiments, the multispecific antibody comprises a first peptide chain comprising the sequence set forth in SEQ ID NO: 100, a second peptide chain comprising the sequence set forth in SEQ ID NO: 99, a third peptide chain comprising the sequence set forth in SEQ ID NO: 97, and a fourth peptide chain comprising the sequence set forth in SEQ ID NO: 98.

In certain embodiments, the multispecific antibody comprises a first peptide chain comprising the sequence set forth in SEQ ID NO: 110, a second peptide chain comprising the sequence set forth in SEQ ID NO: 109, a third peptide chain comprising the sequence set forth in SEQ ID NO: 111, and a fourth peptide chain comprising the sequence set forth in SEQ ID NO: 112.

In certain embodiments, the multispecific antibody comprises a first peptide chain comprising the sequence set forth in SEQ ID NO: 114, a second peptide chain comprising the sequence set forth in SEQ ID NO: 113, a third peptide chain comprising the sequence set forth in SEQ ID NO: 115, and a fourth peptide chain comprising the sequence set forth in SEQ ID NO: 116.

In certain embodiments, the multispecific antibody comprises a first peptide chain comprising the sequence set forth in SEQ ID NO: 150, a second peptide chain comprising the sequence set forth in SEQ ID NO: 148, a third peptide chain comprising the sequence set forth in SEQ ID NO: 149, and a fourth peptide chain comprising the sequence set forth in SEQ ID NO: 151.

In certain embodiments, the multispecific antibody comprises a first peptide chain comprising the sequence set forth in SEQ ID NO: 154, a second peptide chain comprising the sequence set forth in SEQ ID NO: 152, a third peptide chain comprising the sequence set forth in SEQ ID NO: 153, and a fourth peptide chain comprising the sequence set forth in SEQ ID NO: 155.

In certain embodiments, the multispecific antibody comprises:
(i) a first peptide chain comprising a VL of the sequence set forth in SEQ ID NO: 6 and a CL of the sequence set forth in SEQ ID NO: 11, 82, 81, 143;
(ii) a second peptide chain comprising a VH of the sequence set forth in SEQ ID NO: 5 and a CH of the sequence set forth in SEQ ID NO: 13, 80, 79, 140;
(iii) a third peptide chain comprising a VH of the sequence set forth in SEQ ID NO: 1, 78 and a CH of the sequence set forth in SEQ ID NO: 14, 79, 80, 141;
(iv) a fourth peptide chain comprising a VL of the sequence set forth in SEQ ID NO: 2 and a CL of the sequence set forth in SEQ ID NO: 11, 81, 82, 143.

In certain embodiments, the multispecific antibody comprises a first peptide chain comprising the sequence set forth in SEQ ID NO: 33, a second peptide chain comprising the sequence set forth in SEQ ID NO: 32, a third peptide chain comprising the sequence set forth in SEQ ID NO: 34, and a fourth peptide chain comprising the sequence set forth in SEQ ID NO: 35.

In certain embodiments, the multispecific antibody comprises a first peptide chain comprising the sequence set forth in SEQ ID NO: 90, a second peptide chain comprising the sequence set forth in SEQ ID NO: 89, a third peptide chain comprising the sequence set forth in SEQ ID NO: 91, and a fourth peptide chain comprising the sequence set forth in SEQ ID NO: 92.

In certain embodiments, the multispecific antibody comprises a first peptide chain comprising the sequence set forth in SEQ ID NO: 104, a second peptide chain comprising the sequence set forth in SEQ ID NO: 103, a third peptide chain comprising the sequence set forth in SEQ ID NO: 101, and a fourth peptide chain comprising the sequence set forth in SEQ ID NO: 102.

In certain embodiments, the multispecific antibody comprises a first peptide chain comprising the sequence set forth in SEQ ID NO: 108, a second peptide chain comprising the sequence set forth in SEQ ID NO: 107, a third peptide chain comprising the sequence set forth in SEQ ID NO: 105, and a fourth peptide chain comprising the sequence set forth in SEQ ID NO: 106.

In certain embodiments, the multispecific antibody comprises a first peptide chain comprising the sequence set forth in SEQ ID NO: 118, a second peptide chain comprising the sequence set forth in SEQ ID NO: 117, a third peptide chain comprising the sequence set forth in SEQ ID NO: 119, and a fourth peptide chain comprising the sequence set forth in SEQ ID NO: 120.

In certain embodiments, the multispecific antibody comprises a first peptide chain comprising the sequence set forth in SEQ ID NO: 122, a second peptide chain comprising the sequence set forth in SEQ ID NO: 121, a third peptide chain comprising the sequence set forth in SEQ ID NO: 123, and a fourth peptide chain comprising the sequence set forth in SEQ ID NO: 124.

In certain embodiments, the multispecific antibody comprises a first peptide chain comprising the sequence set forth in SEQ ID NO: 158, a second peptide chain comprising the sequence set forth in SEQ ID NO: 156, a third peptide chain comprising the sequence set forth in SEQ ID NO: 157, and a fourth peptide chain comprising the sequence set forth in SEQ ID NO: 159.

In certain embodiments, the first antigen-binding domain is a VHH, the second antigen-binding domain is a Fab fragment, and the multispecific antibody comprises:
(i) a first peptide chain comprising a VL of the second antigen-binding domain and a light chain constant region (CL). In certain embodiments, the CL is a kappa light chain constant region.
(ii) a second peptide chain comprising a VH of the second antigen-binding domain and a heavy chain constant region (CH). In certain embodiments, the CH is IgG, e.g., IgG1, IgG2, IgG3, or IgG4.
   and
(iii) a third peptide chain comprising the first antigen-binding domain and an Fc domain monomer. In certain embodiments, the Fc domain monomer is IgG, e.g., IgG1, IgG2, IgG3, or IgG4. In certain embodiments, the Fc domain monomer comprises a hinge region, CH2, and CH3; preferably, the first antigen-binding domain is linked to the N-terminus of the Fc domain monomer via a linker (e.g., a peptide linker comprising one or more glycine (G) and/or serine (S) residues).

In certain embodiments, the Fc domain monomer of the third peptide chain is capable of forming a dimer with the Fc domain of the heavy chain constant region (CH) of the second peptide chain. In certain embodiments, the Fc domain comprises a modification as defined above.

In certain embodiments, the CL of the first peptide chain is capable of forming a dimer with the CH of the second peptide chain.

In certain embodiments, the multispecific antibody comprises:
(i) a first peptide chain comprising a VL of the sequence set forth in SEQ ID NO: 2 and a CL of the sequence set forth in SEQ ID NO: 11;
(ii) a second peptide chain comprising a VH of the sequence set forth in SEQ ID NO: 1, 78, and a CH of the sequence set forth in SEQ ID NO: 17; and
(iii) a third peptide chain comprising a VHH of the sequence set forth in SEQ ID NO: 7, 8, 9, 10, and an Fc domain monomer of the sequence set forth in SEQ ID NO: 16.

In certain embodiments, the multispecific antibody comprises:
(1) a first peptide chain comprising the sequence set forth in SEQ ID NO: 38, a second peptide chain comprising the sequence set forth in SEQ ID NO: 37, and a third peptide chain comprising the sequence set forth in SEQ ID NO: 36;
(2) a first peptide chain comprising the sequence set forth in SEQ ID NO: 41, a second peptide chain comprising the sequence set forth in SEQ ID NO: 40, and a third peptide chain comprising the sequence set forth in SEQ ID NO: 39;
(3) a first peptide chain comprising the sequence set forth in SEQ ID NO: 44, a second peptide chain comprising the sequence set forth in SEQ ID NO: 43, and a third peptide chain comprising the sequence set forth in SEQ ID NO: 42;
(4) a first peptide chain comprising the sequence set forth in SEQ ID NO: 47, a second peptide chain comprising the sequence set forth in SEQ ID NO: 46, and a third peptide chain comprising the sequence set forth in SEQ ID NO: 45.
(5) a first peptide chain comprising the sequence set forth in SEQ ID NO: 127, a second peptide chain comprising the sequence set forth in SEQ ID NO: 126, and a third peptide chain comprising the sequence set forth in SEQ ID NO: 125;
(6) a first peptide chain comprising the sequence set forth in SEQ ID NO: 130, a second peptide chain comprising the sequence set forth in SEQ ID NO: 129, and a third peptide chain comprising the sequence set forth in SEQ ID NO: 128;
(7) a first peptide chain comprising the sequence set forth in SEQ ID NO: 133, a second peptide chain comprising the sequence set forth in SEQ ID NO: 132, and a third peptide chain comprising the sequence set forth in SEQ ID NO: 131;
(8) a first peptide chain comprising the sequence set forth in SEQ ID NO: 136, a second peptide chain comprising the sequence set forth in SEQ ID NO: 135, and a third peptide chain comprising the sequence set forth in SEQ ID NO: 134.

In another aspect, the present application provides an isolated nucleic acid molecule comprising a nucleotide sequence encoding the multispecific antibody as previously described or at least one peptide chain thereof.

In another aspect, the present application provides a vector comprising the isolated nucleic acid molecule as previously described.

In certain embodiments, the vector comprises nucleotide sequences encoding the respective peptide chains of the multispecific antibody, and the nucleotide sequences encoding the respective peptide chains are present on the same or different vectors.

In another aspect, the present application provides a host cell comprising the isolated nucleic acid molecule as previously described or the vector as previously described.

In certain embodiments, the host cell has low or no fucosylation activity, for example, may be a mammalian cell lacking expression of a gene encoding fucosyltransferase (e.g., FUT8), such as a CHO cell.

In another aspect, the present application provides a method of preparing the multispecific antibody as previously described, comprising culturing the host cell as previously described under conditions that allow protein expression, and recovering the multispecific antibody from the cultured host cell culture.

In certain embodiments, the host cell has low or no fucosylation activity, thereby producing a hypofucosylated or afucosylated antibody. In certain embodiments, the host cell may be a mammalian cell such as a CHO cell lacking expression of a gene encoding fucosyltransferase (e.g., FUT8).

In another aspect, the present application provides an immunoconjugate comprising the antibody or antigen-binding fragment thereof as previously described or the single domain antibody or antigen-binding fragment thereof as previously described or the multispecific molecule as previously described, and a therapeutic agent linked to the antibody or antigen-binding fragment thereof or the multispecific molecule.

In certain embodiments, the therapeutic agent is selected from a cytotoxic agent.

In certain embodiments, the therapeutic agent is selected from an alkylating agent, a mitotic inhibitor, an anti-tumor antibiotic, an antimetabolite, a topoisomerase inhibitor, a tyrosine kinase inhibitor, a radionuclide agent, and any combination thereof.

In certain embodiments, the immunoconjugate is an antibody-drug conjugate (ADC).

In another aspect, the present application provides a pharmaceutical composition comprising the antibody or antigen-binding fragment thereof as previously described, or the single domain antibody or antigen-binding fragment thereof as previously described, or the multispecific molecule as previously described, and a pharmaceutically acceptable carrier and/or excipient.

In certain embodiments, the pharmaceutical composition further comprises an additional pharmaceutically active agent.

In certain embodiments, the additional pharmaceutically active agent is a drug having anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the pharmaceutical composition further comprises an anti-PD-1 antibody.

In certain embodiments, the pharmaceutical composition comprises the multispecific antibody as previously described and an anti-PD-1 antibody.

In another aspect, the present application provides a kit comprising the antibody or antigen-binding fragment thereof as previously described or the single domain antibody or antigen-binding fragment thereof as previously described or the multispecific antibody as previously described.

In certain embodiments, the antibody or antigen-binding fragment thereof has a detectable label, such as an enzyme (e.g., horseradish peroxidase), a radionuclide, a fluorescent dye, a luminescent substance (e.g., a chemiluminescent substance), or biotin.

In certain embodiments, the multispecific antibody has a detectable label, such as an enzyme (e.g., horseradish peroxidase), a radionuclide, a fluorescent dye, a luminescent substance (e.g., a chemiluminescent substance), or biotin.

In another aspect, the present application provides a method of inhibiting the growth of and/or killing a tumor cell expressing CCR8 and/or CTLA4, comprising contacting the tumor cell with an effective amount of the antibody or antigen-binding fragment thereof as previously described, or the multispecific molecule as previously described, or the immunoconjugate as previously described, or the pharmaceutical composition as previously described.

In another aspect, the present application provides use of the antibody or antigen-binding fragment thereof as previously described, or the single domain antibody or antigen-binding fragment thereof as previously described, or the multispecific molecule as previously described, or the immunoconjugate as previously described, or the pharmaceutical composition as previously described for the manufacture of a medicament for:
(1) increasing immune cell activity in vitro or in vivo in a subject;
(2) enhancing an immune response in a subject;
(3) preventing and/or treating a tumor in a subject; or
(4) preventing and/or treating an infection in a subject.

In certain embodiments, the tumor expresses CCR8 and/or CTLA4.

In certain embodiments, the CCR8 and/or CTLA4 are expressed on the surface of the tumor cell.

In certain embodiments, the tumor is selected from non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoides, Merkel cell carcinoma, and other hematologic malignancies, such as classical Hodgkin's lymphoma (CHL), primary mediastinal large B-cell lymphoma, T-cell/histiocyte rich B-cell lymphoma, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

In certain embodiments, the infection is selected from a viral infection, a bacterial infection, a fungal infection, and a parasitic infection.

In certain embodiments, the subject is a mammal, such as a human, cynomolgus monkey, or mouse.

In another aspect, the present application provides use of the antibody or antigen-binding fragment thereof as previously described, or the single domain antibody or antigen-binding fragment thereof as previously described, or the multispecific molecule as previously described, for the manufacture of a kit for detecting whether a tumor may be treated by an anti-tumor therapy targeting CCR8 and/or CTLA4;
(1) contacting a sample containing the tumor cells with the antibody or antigen-binding fragment thereof as previously described or the single domain antibody or antigen-binding fragment thereof as previously described;
(2) detecting the formation of a complex between the antibody or antigen-binding fragment thereof and CCR8 and/or CTLA4.

In certain embodiments, the antibody or antigen-binding fragment thereof has a detectable label.

In certain embodiments, the CCR8 and/or CTLA4 is mammalian (e.g., human, monkey) CCR8 and/or CTLA4.

In certain embodiments, the tumor is selected from non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoides, Merkel cell carcinoma, and other hematologic malignancies, such as classical Hodgkin's lymphoma (CHL), primary mediastinal large B-cell lymphoma, T-cell/histiocyte rich B-cell lymphoma, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.

### Definition of Terms

As used herein, the term "antibody" refers to an immunoglobulin molecule typically composed of two pairs of polypeptide chains, each having one light chain (LC) and one heavy chain (HC). Antibody light chains are classified as kappa (κ) and lambda (λ) light chains. The heavy chains are classified as µ, δ, γ, α, or ε, and the isotype of the antibody as IgM, IgD, IgG, IgA, and IgE, respectively. Within the light and heavy chains, the variable and constant regions are joined by a "J" region of about 12 or more amino acids, and the heavy chains further comprise a "D" region of about 3 or more amino acids. Each heavy chain is composed of a heavy chain variable region (VH) and a heavy chain constant region (CH). The heavy chain constant region is composed of three domains, CH1, CH2, and CH3. Each light chain is composed of a light chain variable region (VL) and a light chain constant region (CL). The light chain constant region is composed of one domain, CL. The constant domains are not directly involved in the binding of an antibody to an antigen but exhibit various effector functions, such as mediating the binding of immunoglobulin to host tissues or factors, including various cells of the immune system (e.g., effector cells) and the first component (C1q) of the classical complement system. The VH and VL regions are also subdivided into regions of hypervariability, termed complementarity determining regions (CDRs), interspersed with more conserved regions termed framework regions (FRs). Each VH and VL is composed of three CDRs and four FRs arranged from amino-terminus to carboxyl-terminus in the following order: FR1, CDR1, FR2, CDR2, FR3, CDR3, FR4. The variable regions (VH and VL) of each heavy/light chain pair form the antigen-binding site, respectively. The assignment of amino acids in each region or domain may follow the definition of Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to the amino acid residues in the variable region of an antibody that are responsible for antigen binding. The variable regions in a heavy chain and a light chain each contain three CDRs, designated CDR1, CDR2, and CDR3. The precise boundaries of these CDRs may be defined according to various numbering systems known in the art, for example, according to the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, those skilled in the art will readily identify the CDRs defined by each numbering system. Moreover, the correspondence between different numbering systems is well known to those skilled in the art (see, e.g., Lefranc et al., Dev. Comparat. Immunol. 27:55-77, 2003).

In the present disclosure, the CDRs contained in the antibody or antigen-binding fragment thereof of the present disclosure may be determined according to various numbering systems known in the art. In certain embodiments, the CDRs contained in the antibody or antigen-binding fragment thereof of the present disclosure are preferably determined by the Kabat, Chothia, or IMGT numbering system.

As used herein, the term "framework region" or "FR" residues refers to those amino acid residues in the variable region of an antibody other than the CDR residues defined above.

The term "antibody" is not limited by any specific method of producing the antibody. For example, the antibody includes a recombinant antibody, a monoclonal antibody, and a polyclonal antibody. The antibody may be of different isotypes, e.g., IgG (e.g., IgG1, IgG2, IgG3, or IgG4 subtypes), IgA1, IgA2, IgD, IgE, or IgM antibodies.

As used herein, the terms "monoclonal antibody," "McAb," and "mAb" have the same meaning and are used interchangeably, and refer to an antibody or a fragment of an antibody from a population of highly homologous antibody molecules, i.e., a population of identical antibody molecules except for possibly naturally occurring mutations that may arise spontaneously. Monoclonal antibodies have high specificity for a single epitope on an antigen. Polyclonal antibodies, in contrast to monoclonal antibodies, typically comprise at least 2 or more different antibodies that typically recognize different epitopes on an antigen. Furthermore, the modifier "monoclonal" merely indicates that the antibody is characterized by being obtained from a highly homologous population of antibodies and is not to be construed as requiring the preparation of the antibody by any particular method.

The monoclonal antibody of the present disclosure may be prepared by a variety of techniques, such as hybridoma technology (see, e.g., Kohler et al., Nature, 256:495, 1975), recombinant DNA technology (see, e.g., U.S. Patent Application No. 4,816,567), or phage antibody library technology (see, e.g., Clackson et al., Nature 352:624-628, 1991, or Marks et al. J. Mol. Biol. 222:581-597, 1991).

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specific binding to the antigen, which is also referred to as an "antigen-binding portion". See generally, Fundamental Immunology, Ch. 7 (Paul, W., ed., 2nd ed., Raven Press, N.Y. (1989), which is incorporated herein by reference in its entirety for all purposes. The antigen-binding fragment of an antibody may be produced by recombinant DNA techniques or by enzymatic or chemical cleavage of an intact antibody. Non-limiting examples of antigen-binding fragments include Fab, Fab', F(ab')2, Fd, Fv, complementarity determining region (CDR) fragments, scFv, diabodies, single domain antibodies, chimeric antibodies, linear antibodies, nanobodies (technology from Domantis), probodies, and polypeptides comprising at least a portion of an antibody sufficient to confer specific antigen binding ability to the polypeptide. Engineered antibody variants are reviewed in Holliger et al., 2005; Nat Biotechnol, 23:1126-1136.

As used herein, the term "full-length antibody" means an antibody consisting of two "full-length heavy chains" and two "full-length light chains". Here, a "full-length heavy chain" refers to a polypeptide chain consisting, in N-terminus to C-terminus direction, of a heavy chain variable region (VH), a heavy chain constant region CH1 domain, a hinge region (HR), a heavy chain constant region CH2 domain, and a heavy chain constant region CH3 domain. Also, the full-length antibody, when is of an IgE isotype, optionally also includes a heavy chain constant region CH4 domain. Preferably, the "full-length heavy chain" is a polypeptide chain consisting of VH, CH1, HR, CH2, and CH3 in the N-terminus to C-terminus direction. The "full-length light chain" is a polypeptide chain consisting of a light chain variable region (VL) and a light chain constant region (CL) in the N-terminus to C-terminus direction. Two pairs of full-length antibody chains are linked together by disulfide bonds between CL and CH1 and between the HRs of the two full-length heavy chains. The full-length antibody of the present disclosure may be from a single species, e.g., human, or may also be a chimeric antibody or a humanized antibody. The full-length antibody of the present disclosure comprises two antigen-binding sites formed by VH and VL pairs, both of which specifically recognize/bind to the same antigen.

As used herein, the term "Fd" means an antibody fragment consisting of the VH and CH1 domains. The term "dAb fragment" means an antibody fragment consisting of the VH domain (Ward et al., Nature 341:544 546 (1989)). The term "Fab fragment" means an antibody fragment consisting of the VL, VH, CL, and CH1 domains. The term "F(ab')2 fragment" means an antibody fragment comprising two Fab fragments linked by a disulfide bridge in the hinge region. The term "Fab' fragment" means a fragment obtained by reducing the disulfide bond linking the two heavy chain fragments in an F(ab')2 fragment, consisting of an intact light chain and an Fd fragment of the heavy chain (consisting of the VH and CH1 domains).

As used herein, the term "Fv" means an antibody fragment consisting of the VL and VH domains of a single arm of an antibody. The Fv fragment is generally considered to be the smallest antibody fragment capable of forming an intact antigen-binding site. It is generally believed that six CDRs confer the antigen-binding specificity of an antibody. However, even a single variable region (e.g., an Fd fragment, which contains only three CDRs specific for an antigen) is capable of recognizing and binding an antigen, although with potentially lower affinity than the intact binding site.

As used herein, the term "Fc" means an antibody fragment formed by the disulfide bonding of the second and third constant regions of the first heavy chain of an antibody with the second and third constant regions of the second heavy chain. The Fc fragment of an antibody has various functions but does not participate in antigen binding.

As used herein, the term "scFv" refers to a single polypeptide chain comprising VL and VH domains, wherein the VL and VH are linked by a linker (see, e.g., Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH2-VL-linker-VH-COOH or NH2-VH-linker-VL-COOH. Suitable prior art linkers consist of repeating GGGGS amino acid sequence or variants thereof. For example, a linker having the amino acid sequence (GGGGS)₄ may be used, but variants thereof may also be used (Holliger et al. (1993), Proc. Natl. Acad. Sci. USA 90:6444-6448). Other linkers useful in the present disclosure are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between VH and VL of the scFv. In certain embodiments of the present disclosure, scFv may form a di-scFv, which refers to an antibody formed by linking two or more single scFv molecules in tandem. In certain embodiments of the present disclosure, scFv may form (scFv)2, which refers to an antibody formed by linking two or more single scFv molecules in parallel.

As used herein, the term "single-domain antibody (sdAb)" has the meaning commonly understood by those skilled in the art, and refers to an antibody fragment consisting of a single monomeric variable antibody domain (e.g., a single heavy chain variable region) that retains the ability to specifically bind to the same antigen to which the full-length antibody binds. The single domain antibody is also referred to as nanobody.

Each of the above antibody fragments retains the ability to specifically bind to the same antigen to which the full-length antibody binds, and/or competes with the full-length antibody for specific binding to the antigen.

The antigen-binding fragment of antibodies (e.g., the above antibody fragment) can be obtained from a given antibody (e.g., the antibody provided by the present disclosure) using conventional techniques known to those skilled in the art (e.g., recombinant DNA technology or enzymatic or chemical cleavage methods). The antigen-binding fragments of the antibodies are screened for specificity in the same manner as used for an intact antibody.

Herein, unless the context clearly indicates otherwise, when referring to the term "antibody", it includes not only an intact antibody but also an antigen-binding fragment of the antibody.

As used herein, the term "chimeric antibody" refers to an antibody in which a portion of the light chain and/or heavy chain is derived from one antibody (which may be derived from a particular species or belong to a particular antibody class or subclass), and the other portion of the light chain and/or heavy chain is derived from another antibody (which may be derived from the same or a different species or belong to the same or a different antibody class or subclass), but which in any case retains binding activity to the antigen of interest (U.S.P 4,816,567 to Cabilly et al.; Morrison et al., Proc. Natl. Acad. Sci. USA, 81:6851 6855 (1984)). In certain embodiments, the term "chimeric antibody" may include an antibody in which the heavy and light chain variable regions of the antibody are derived from a first antibody, and the heavy and light chain constant regions of the antibody are derived from a second antibody.

As used herein, the term "identity" is used to refer to the degree of sequence matching between two polypeptides or between two nucleic acids. To determine the percent identity of two amino acid sequences or two nucleic acid sequences, the sequences are aligned for optimal comparison purposes (e.g., gaps may be introduced in the first amino acid sequence or nucleic acid sequence for optimal alignment with the second amino acid sequence or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between the two sequences is a function of the number of identical positions shared by the sequences (i.e., percent identity = number of identical overlapping positions / total number of positions × 100%). In certain embodiments, the two sequences are the same length.

Determination of percent identity between two sequences may also be achieved using mathematical algorithms. A non-limiting example of a mathematical algorithm used for the comparison of two sequences is the algorithm of Karlin and Altschul, 1990, Proc. Natl. Acad. Sci. U.S.A. 87:2264-2268, as modified in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by introducing amino acid residue substitutions, deletions, or additions. In some cases, the term "variant" also refers to a polypeptide or peptide that has been modified (i.e., by covalently attaching any type of molecule to the polypeptide or peptide). For example, but not by way of limitation, a polypeptide may be modified, e.g., by glycosylation, acetylation, pegylation, phosphorylation, amidation, derivatization by known protecting/blocking groups, proteolytic cleavage, attachment to a cellular ligand or other protein, etc. Derivatized polypeptides or peptides may be generated by chemical modifications using techniques known to those skilled in the art, including but not limited to specific chemical cleavage, acetylation, formylation, metabolic synthesis of tunicamycin, etc. Furthermore, a variant has similar, identical, or improved functionality compared to the polypeptide or peptide from which it is derived.

As used herein, the term "specifically binding" and "specific binding" refers to a non-random binding reaction between two molecules, such as between an antibody and an antigen against which it is directed. The strength or affinity of a specific binding interaction may be represented by the equilibrium dissociation constant (KD) of the interaction. In the present disclosure, the term "KD" refers to the dissociation equilibrium constant for a particular antibody-antigen interaction, which is used to describe the binding affinity between an antibody and an antigen. The smaller the equilibrium dissociation constant, the tighter the antibody-antigen binding, and the higher the affinity between the antibody and the antigen.

As used herein, a detectable label described herein may be any substance detectable by fluorescence, spectroscopy, photochemistry, biochemistry, immunology, electricity, optics, or chemistry. Such labels are well known in the art, and examples include, but are not limited to, enzymes (e.g., horseradish peroxidase, alkaline phosphatase, β-galactosidase, urease, glucose oxidase, etc.), radionuclides (e.g., 3H, 125I, 35S, 14C, or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots, or cyanine dye derivatives (e.g., Cy7, Alexa 750)), luminescent substances (e.g., chemiluminescent substances such as acridinium esters, luminol and its derivatives thereof, ruthenium derivatives such as tris(bipyridine)ruthenium(II)), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels.

As used herein, the term "vector" refers to a nucleic acid vehicle into which a polynucleotide may be inserted. When a vector enables expression of a protein encoded by the inserted polynucleotide, the vector is referred to as an expression vector. The vector may be introduced into a host cell by transformation, transduction, or transfection, allowing the genetic material elements carried to be expressed in the host cell. Vectors are well known to those skilled in the art and include, but are not limited to: plasmids; phagemids; cosmids; artificial chromosomes, such as yeast artificial chromosomes (YACs), bacterial artificial chromosomes (BACs), or P1-derived artificial chromosomes (PACs); bacteriophages such as lambda phage or M13 phage, and animal viruses. The animal viruses useful as vectors include, but are not limited to, retroviruses (including lentiviruses), adenoviruses, adeno-associated viruses, herpesviruses (e.g., herpes simplex virus), poxviruses, baculoviruses, papillomaviruses, and papovaviruses (e.g., SV40). A vector may contain a variety of expression-controlling elements, including but not limited to, a promoter sequence, a transcription initiation sequence, an enhancer sequence, a selection element, and a reporter gene. Additionally, the vector may contain an origin of replication.

As used herein, the term "host cell" refers to a cell into which a vector may be introduced, including, but not limited to, prokaryotic cells such as *Escherichia coli* or Bacillu*s subtilis,* fungal cells such as yeast cells or *Aspergillus*, insect cells such as S2 *Drosophila* cells or Sf9 cells, or animal cells such as fibroblasts, CHO cells, COS cells, NSO cells, HeLa cells, BHK cells, HEK 293 cells, or human cells.

As used herein, the term "conservative substitution" refers to an amino acid substitution that does not adversely affect or alter the intended properties of the protein/polypeptide comprising an amino acid sequence. For example, the conservative substitution may be introduced by standard techniques known in the art, such as site-directed mutagenesis and PCR-mediated mutagenesis. The conservative amino acid substitution includes a substitution in which an amino acid residue is replaced with another amino acid residue having a similar side chain, for example, substitution with a residue that is physically or functionally similar to the corresponding amino acid residue (e.g., having similar size, shape, charge, chemical properties, including the ability to form covalent or hydrogen bonds, etc.). Families of amino acid residues having similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), beta-branched side chains (e.g., threonine, valine, and isoleucine), and aromatic side chains (e.g., tyrosine, phenylalanine, tryptophan, and histidine). Therefore, it is preferred to replace an amino acid residue with another corresponding amino acid residue from the same side chain family. Methods for identifying conservative amino acid substitutions are well known in the art (see, e.g., Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The abbreviations for the twenty conventional amino acids mentioned herein follow conventional usage. See, e.g., Immunology-A Synthesis (2nd Edition, E.S. Golub and D.R. Gren, Eds., Sinauer Associates, Sunderland, Mass. (1991)), which is incorporated herein by reference. In the present disclosure, the terms "polypeptide" and "protein" have the same meaning and are used interchangeably. Furthermore, in the present disclosure, amino acids are generally represented by single-letter and three-letter abbreviations well known in the art. For example, alanine may be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), including but not limited to: pH adjusting agents, surfactants, adjuvants, ionic strength enhancers, diluents, agents for maintaining osmotic pressure, agents for delaying absorption, preservatives. For example, pH adjusters include, but are not limited to, phosphate buffers. Surfactants include, but are not limited to, cationic, anionic, or nonionic surfactants, such as Tween-80. Ionic strength enhancers include, but are not limited to, sodium chloride. Preservatives include, but are not limited to, various antibacterial and antifungal agents, such as parabens, chlorobutanol, phenol, sorbic acid, etc. Agents for maintaining osmotic pressure include, but are not limited to, sugars, NaCl, and the like. Agents for delaying absorption include, but are not limited to, monostearates and gelatin. Diluents include, but are not limited to, water, aqueous buffers (e.g., buffered saline), alcohols, and polyols (e.g., glycerol), etc. Preservatives include, but are not limited to, various antibacterial and antifungal agents, such as thimerosal, 2-phenoxyethanol, parabens, chlorobutanol, phenol, sorbic acid, etc. Stabilizers have the meaning commonly understood by those skilled in the art and are capable of stabilizing the desired activity of an active ingredient in a drug, including but not limited to sodium glutamate, gelatin, SPGA, saccharides (e.g., sorbitol, mannitol, starch, sucrose, lactose, dextran, or glucose), amino acids (e.g., glutamic acid, glycine), proteins (e.g., dried whey, albumin, or casein), or degradation products thereof (e.g., lactalbumin hydrolysate), etc. In certain exemplary embodiments, the pharmaceutically acceptable carrier or excipient comprises a sterile injectable liquid (e.g., aqueous or non-aqueous suspensions or solutions). In certain exemplary embodiments, such sterile injectable liquid is selected from water for injection (WFI), bacteriostatic water for injection (BWFI), sodium chloride solution (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

As used herein, the term "prevention" refers to a method performed to prevent or delay the onset of a disease or disorder or symptom in a subject. As used herein, the term "treatment" refers to a method performed to obtain a beneficial or desired clinical outcome. For the purposes of the present disclosure, beneficial or desired clinical outcomes include (but are not limited to) alleviating symptoms, reducing the extent of the disease, stabilizing (i.e., not worsening) the state of the disease, delaying or slowing the progression of the disease, ameliorating or alleviating the state of the disease, and relieving symptoms (whether partially or totally), whether detectable or undetectable. Furthermore, "treatment" may also refer to prolonging survival compared to expected survival if not treated.

As used herein, the term "subject" refers to a mammal, such as a human, a cynomolgus monkey, or a mouse.

As used herein, the term "effective amount" refers to an amount sufficient to obtain, or at least partially obtain, a desired effect. For example, an effective amount for preventing a disease refers to an amount sufficient to prevent, arrest, or delay the occurrence of the disease. A therapeutically effective amount refers to an amount sufficient to cure or at least partially arrest the disease and its complications in a patient already suffering from the disease. Determination of such an effective amount is well within the capability of those skilled in the art. For example, the amount effective for therapeutic use will depend on the severity of the disease to be treated, the overall state of the immune system of the patient, the general condition of the patient such as age, weight, and sex, the mode of administration of a drug, and other treatments administered concurrently, etc.

### Beneficial Effects of the Invention

The present application has obtained monoclonal antibodies/single domain antibodies capable of specifically binding to CTLA4. Furthermore, bispecific antibodies capable of specifically binding to CCR8 and CTLA4, and defucosylated bispecific antibodies are prepared. Compared with benchmark antibodies, the bispecific antibodies or defucosylated bispecific antibodies of the present application are capable of: specifically binding to CCR8/CTLA4 co-expressing cells; specifically inducing ADCC effects against CCR8/CTLA4 co-expressing cells; inducing the killing of PBMC to CCR8/CTLA4 co-expressing cells; specifically inducing internalization effects specific to CCR8/CTLA4 co-expressing cells; inhibiting tumor growth *in vivo*, with a tumor growth inhibitory activity significantly superior to that of control monoclonal antibodies; having better thermal stability and hydrophilicity; having higher safety. Therefore, the antibodies of the present application have significant clinical value.

The embodiments of the present disclosure will be described in detail below with reference to the accompanying drawings and examples, but it will be understood by those skilled in the art that the following drawings and examples are intended to illustrate the present disclosure solely and are not intended to limit the scope of the present disclosure. Various objects and advantageous aspects of the present disclosure will become apparent to those skilled in the art from the following detailed description of the drawings and preferred embodiments.

### DESCRIPTION OF THE DRAWINGS

Fig. 1 shows a schematic diagram of the structures of the CCR8 monoclonal antibody, CTLA4 monoclonal antibody, and anti-CTLA4xCCR8 bispecific antibody synthesized in the present disclosure.
Figs. 2-4 show binding curves of the monoclonal antibodies and anti-CTLA4xCCR8 bispecific antibodies of the present disclosure to cells overexpressing human CCR8, cells overexpressing human CTLA4, and cells overexpressing both human CCR8 and human CTLA4.
Figs. 5-7 show ADCC effects of the monoclonal antibodies and anti-CTLA4xCCR8 bispecific antibodies of the present disclosure detected based on a luciferase reporter gene system.
Figs. 8-10 show the killing effects of PBMCs on cells overexpressing human CCR8, cells overexpressing human CTLA4, and cells overexpressing both human CCR8 and human CTLA4 induced by the monoclonal antibodies and anti-CTLA4xCCR8 bispecific antibodies of the present disclosure.
Figs. 11-13 show the effects of antibody internalization upon binding of the monoclonal antibodies and anti-CTLA4xCCR8 bispecific antibodies of the present disclosure to cells overexpressing human CCR8, cells overexpressing human CTLA4, and cells overexpressing both human CCR8 and human CTLA4.
Fig. 14 shows the blocking results of CTLA4 binding to human CD80/CD86 proteins by the monoclonal antibodies and anti-CTLA4xCCR8 bispecific antibodies of the present disclosure.
Fig. 15 shows the results of PTM studies after high temperature treatment of the anti-CTLA4xCCR8 bispecific antibodies of the present disclosure.
Fig. 16 shows the study results of the hydrophilic and hydrophobic properties of the monoclonal antibodies and anti-CTLA4xCCR8 bispecific antibodies of the present disclosure.
Fig. 17 shows binding curves of the monoclonal antibodies and anti-CTLA4xCCR8 bispecific antibodies of the present disclosure to cells overexpressing human CCR8, cells overexpressing human CTLA4, and cells overexpressing both human CCR8 and human CTLA4.
Fig. 18 shows ADCC effects of the monoclonal antibodies and anti-CTLA4xCCR8 bispecific antibodies of the present disclosure detected based on a luciferase reporter gene system.
Fig. 19 shows the killing effects of PBMCs on cells overexpressing human CCR8, cells overexpressing human CTLA4, and cells overexpressing both human CCR8 and human CTLA4 induced by the monoclonal antibodies and anti-CTLA4xCCR8 bispecific antibodies of the present disclosure.
Fig. 20 shows the *in vivo* efficacy activity of the defucosylated anti-CTLA4xCCR8 bispecific antibody of the present disclosure.
Fig. 21 shows the *in vivo* efficacy activity of the anti-CTLA4xCCR8 bispecific antibody (with the Fc of murine IgG2a) of the present disclosure.
Fig. 22 shows the safety evaluation results of the anti-CTLA4xCCR8 bispecific antibody of the present disclosure.
Fig. 23 shows the results of cell line screening of the anti-CTLA4xCCR8 bispecific antibody of the present disclosure, wherein A in Fig. 23 shows the SEC results of the anti-CTLA4xCCR8 of the present disclosure, and B and C in Fig. 23 show the high-performance liquid chromatography mass spectrometry detection results of the anti-CTLA4xCCR8 of the present disclosure.
Fig. 24 shows binding curves of the anti-CTLA4xCCR8 bispecific antibody (cell line-derived) of the present disclosure to cells overexpressing human CCR8, cells overexpressing human CTLA4, and cells overexpressing both human CCR8 and human CTLA4.
Fig. 25 shows ADCC effects of the (from cell lines) anti-CTLA4xCCR8 bispecific antibody of the present disclosure detected based on a luciferase reporter gene system.
Fig. 26 shows the killing effect of PBMCs on cells overexpressing human CCR8, cells overexpressing human CTLA4, and cells overexpressing both human CCR8 and human CTLA4 induced by the anti-CTLA4xCCR8 bispecific antibody (from cell lines) of the present disclosure.

### Sequence Information

Information on some sequences involved in the present disclosure is provided in Table 1 below.

**Table 1: Description of Sequences**

| SEQ ID NO. | Name | Sequences |
|---|---|---|
| 1 | Anti-CCR8(ADI6 8741) mAb heavy chain variable region | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSS |
| 2 | Anti-CCR8(ADI6 8741) mAb light chain variable region | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIK |
| 3 | Anti-CTLA4 (ip-05) mAb heavy chain variable region | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SS |
| 4 | Anti- CTLA4 (ip-05) mAb light chain variable region | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIK |
| 5 | Anti-CTLA4 (ip-0508) mAb heavy chain variable region | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SS |
| 6 | Anti-CTLA4 (ip-0508) mAb light chain variable region | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIK |
| 7 | CTLA4-binding region of Anti-CTLA4 Nanobody VHH-9 | EVQLQESGGGLVQAGGSLTLSCAASGSIFSLNGMEWYRQ FPGQQRELVAAITVGGSTIYADSVKGRFTISRDNAKNTVY LQMSSLKPEDTAVYYCHKFHFTLGQPYWGQGTQVTVSS |
| 8 | CTLA4-binding region of Anti-CTLA4 Nanobody VHH-16 | EVQLQESGGGLVQAGGALRLSCAAPIDIFRISIMGWYRQA PGQQRELVARISKAGTTTYADSVKGRFTISRDNAKNTVIL QMNSLKPEDTAVYYCNADRLRQHDLWGQGTQVTVSS |
| 9 | CTLA4-binding region of Anti-CTLA4 Nanobody VHH-23 | EVQLQESGGGLVQPGGSLRLSCAASGIIFSIYHMGWYRQA PGKQRELVAVITSGGSTNYADSVKGRFTISRDNAKNTVYL QMNSLKPEDTAVYYCNSYHYGATRDYDAWGQGTQVTV SS |
| 10 | CTLA4-binding region of Anti-CTLA4 Nanobody VHH-43 | EVQLQESGGGLVQPGGSLRLSCVASGSIFRPYVMGWYRQ APGKQRELVATNSKGGSTIYADAVKGRFTISSDNDENTV YLQMNSLKPEDTAVYYCNLKELGIYLRGDYWGQGTQVT VSS |
| 11 | Human κ light chain constant region (CL) amino acid sequence | |
| 12 | Human IgG1 amino acid sequence | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSR DELTKNQVSLTCLVKGFYPSDIAVEWESNGQPENNYKTT PPVLDSDGSFFLYSKLTVDKSRWQQGNVFSCSVMHEALH NHYTQKSLSLSPG |
| 13 | Human IgG1 amino acid sequence (with a FL mutation introduced) | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSR DELTKNQVSLTCLVKGFYPSDIAVEWESNGQPENNYKTT PPVLDSDGSFLLYSKLTVDKSRWQQGNVFSCSVMHEALH NHYTQKSLSLSPG |
| 14 | Human IgG1 amino acid sequence (with a KR mutation introduced) | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSR DELTKNQVSLTCLVKGFYPSDIAVEWESNGQPENNYKTT PPVLDSDGSFFLYSRLTVDKSRWQQGNVFSCSVMHEALH NHYTQKSLSLSPG |
| 15 | Human IgG1 Fc amino acid sequence | DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG |
| 16 | Human IgG1 Fc amino acid sequence (with a Knob mutation introduced) | DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPCRDELTKNQVSLWCLVKGFYPSDI AVEWESNGQPENNYKTTPPVLDSDGSFFLYSKLTVDKSR WQQGNVFSCSVMHEALHNHYTQKSLSLSPG |
| 17 | Human IgG1 amino acid sequence (with a Hole mutation introduced) | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVCTLPPSR DELTKNQVSLSCAVKGFYPSDIAVEWESNGQPENNYKTT PPVLDSDGSFFLVSKLTVDKSRWQQGNVFSCSVMHEALH NRFTQKSLSLSPG |
| 18 | Anti-CCR8(ADI6 8741) heavy chain | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPSRDELTKNQVSLTCLVKGFYPSDIAVEWESNGQPENN YKTTPPVLDSDGSFFLYSKLTVDKSRWQQGNVFSCSVMH EALHNHYTQKSLSLSPG |
| 19 | Anti-CCR8(ADI6 8741) light chain | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 20 | Anti-CTLA4 (ip-05) mAb heavy chain | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYK TTPPVLDSDGSFFLYSKLTVDKSRWQQGNVFSCSVMHEA LHNHYTQKSLSLSPG |
| 21 | Anti-CTLA4 (ip-05) mAb light chain | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 22 | Anti-CTLA4 (ip-0508) mAb heavy chain | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYK TTPPVLDSDGSFFLYSKLTVDKSRWQQGNVFSCSVMHEA LHNHYTQKSLSLSPG |
| 23 | Anti-CTLA4 (ip-0508) mAb light chain | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 24 | Anti-CTLA4 (VHH-9) | EVQLQESGGGLVQAGGSLTLSCAASGSIFSLNGMEWYRQ FPGQQRELVAAITVGGSTIYADSVKGRFTISRDNAKNTVY LQMSSLKPEDTAVYYCHKFHFTLGQPYWGQGTQVTVSS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG |
| 25 | Anti-CTLA4 (VHH-16) | EVQLQESGGGLVQAGGALRLSCAAPIDIFRISIMGWYRQA PGQQRELVARISKAGTTTYADSVKGRFTISRDNAKNTVIL QMNSLKPEDTAVYYCNADRLRQHDLWGQGTQVTVSSD KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPG |
| 26 | Anti-CTLA4 (VHH-23) | EVQLQESGGGLVQPGGSLRLSCAASGIIFSIYHMGWYRQA PGKQRELVAVITSGGSTNYADSVKGRFTISRDNAKNTVYL QMNSLKPEDTAVYYCNSYHYGATRDYDAWGQGTQVTV SSDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTI SKAKGQPREPQVYTLPPSRDELTKNQVSLTCLVKGFYPSD IAVEWESNGQPENNYKTTPPVLDSDGSFFLYSKLTVDKSR WQQGNVFSCSVMHEALHNHYTQKSLSLSPG |
| 27 | Anti-CTLA4 (VHH-43) | EVQLQESGGGLVQPGGSLRLSCVASGSIFRPYVMGWYRQ APGKQRELVATNSKGGSTIYADAVKGRFTISSDNDENTV YLQMNSLKPEDTAVYYCNLKELGIYLRGDYWGQGTQVT VSSDKTHTCPPCPAPELLGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALPAPIEKT ISKAKGQPREPQVYTLPPSRDELTKNQVSLTCLVKGFYPS DIAVEWESNGQPENNYKTTPPVLDSDGSFFLYSKLTVDKS RWQQGNVFSCSVMHEALHNHYTQKSLSLSPG |
| 28 | Anti-CTLA4 (ip-05) × CCR8 -1 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYK TTPPVLDSDGSFLLYSKLTVDKSRWQQGNVFSCSVMHEA LHNHYTQKSLSLSPG |
| 29 | Anti-CTLA4 (ip-05) × CCR8 -1 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 30 | Anti-CTLA4 (ip-05) × CCR8 -1 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPSRDELTKNQVSLTCLVKGFYPSDIAVEWESNGQPENN YKTTPPVLDSDGSFFLYSRLTVDKSRWQQGNVFSCSVMH EALHNHYTQKSLSLSPG |
| 31 | Anti-CTLA4 (ip-05) × CCR8 -1 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 32 | Anti-CTLA4 (ip-0508) × CCR8 -1 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYK TTPPVLDSDGSFLLYSKLTVDKSRWQQGNVFSCSVMHEA LHNHYTQKSLSLSPG |
| 33 | Anti-CTLA4 (ip-0508) × CCR8 -1 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 34 | Anti-CTLA4 (ip-0508) × CCR8 -1 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPSRDELTKNQVSLTCLVKGFYPSDIAVEWESNGQPENN YKTTPPVLDSDGSFFLYSRLTVDKSRWQQGNVFSCSVMH EALHNHYTQKSLSLSPG |
| 35 | Anti-CTLA4 (ip-0508) × CCR8 -1 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 36 | Anti-CTLA4 (VHH-9) × CCR8 -1 heavy chain 1 | EVQLQESGGGLVQAGGSLTLSCAASGSIFSLNGMEWYRQ FPGQQRELVAAITVGGSTIYADSVKGRFTISRDNAKNTVY LQMSSLKPEDTAVYYCHKFHFTLGQPYWGQGTQVTVSS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPCRDELTKNQVSLWCLVKGFYPSDI AVEWESNGQPENNYKTTPPVLDSDGSFFLYSKLTVDKSR WQQGNVFSCSVMHEALHNHYTQKSLSLSPG |
| 37 | Anti-CTLA4 (VHH-9) × CCR8 -1 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 38 | Anti-CTLA4 (VHH-9) × CCR8 -1 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 39 | Anti-CTLA4 (VHH-16) × CCR8 -1 heavy chain 1 | EVQLQESGGGLVQAGGALRLSCAAPIDIFRISIMGWYRQA PGQQRELVARISKAGTTTYADSVKGRFTISRDNAKNTVIL QMNSLKPEDTAVYYCNADRLRQHDLWGQGTQVTVSSD KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPCRDELTKNQVSLWCLVKGFYPSDIA VEWESNGQPENNYKTTPPVLDSDGSFFLYSKLTVDKSRW QQGNVFSCSVMHEALHNHYTQKSLSLSPG |
| 40 | Anti-CTLA4 (VHH-16) × CCR8 -1 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 41 | Anti-CTLA4 (VHH-16) × CCR8 -1 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 42 | Anti-CTLA4 (VHH-23) × CCR8 -1 heavy chain 1 | EVQLQESGGGLVQPGGSLRLSCAASGIIFSIYHMGWYRQA PGKQRELVAVITSGGSTNYADSVKGRFTISRDNAKNTVYL QMNSLKPEDTAVYYCNSYHYGATRDYDAWGQGTQVTV SSDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTI SKAKGQPREPQVYTLPPCRDELTKNQVSLWCLVKGFYPS DIAVEWESNGQPENNYKTTPPVLDSDGSFFLYSKLTVDKS RWQQGNVFSCSVMHEALHNHYTQKSLSLSPG |
| 43 | Anti-CTLA4 (VHH-23) × CCR8 -1 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 44 | Anti-CTLA4 (VHH-23) × CCR8 -1 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 45 | Anti-CTLA4 (VHH-43) × CCR8 -1 heavy chain 1 | EVQLQESGGGLVQPGGSLRLSCVASGSIFRPYVMGWYRQ APGKQRELVATNSKGGSTIYADAVKGRFTISSDNDENTV YLQMNSLKPEDTAVYYCNLKELGIYLRGDYWGQGTQVT VSSDKTHTCPPCPAPELLGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALPAPIEKT ISKAKGQPREPQVYTLPPCRDELTKNQVSLWCLVKGFYPS DIAVEWESNGQPENNYKTTPPVLDSDGSFFLYSKLTVDKS RWQQGNVFSCSVMHEALHNHYTQKSLSLSPG |
| 46 | Anti-CTLA4 (VHH-43) × CCR8 -1 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 47 | Anti-CTLA4 (VHH-43) × CCR8 -1 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 48 | ip-05 VH CDR1 | GFTFSSYT |
| 49 | ip-05 VH CDR2 | ISEDGNNK |
| 50 | ip-05 VH CDR3 | ARTGWLGPFDY |
| 51 | ip-05 VL CDR1 | QSVGSSY |
| 52 | ip-05 VL CDR2 | GAF |
| 53 | ip-05 VL CDR3 | QQYGSSPWT |
| 54 | ip-0508 VH CDR1 | GFTFSSYT |
| 55 | ip-0508 VH CDR2 | ISEDGNNK |
| 56 | ip-0508 VH CDR3 | ARTGWLGPEDY |
| 57 | ip-0508 VL CDR1 | QSVGSSY |
| 58 | ip-0508 VL CDR2 | GAF |
| 59 | ip-0508 VL CDR3 | QQYGSSPWT |
| 60 | VHH-9 HCDR1 | GSIFSLNG |
| 61 | VHH-9 HCDR2 | ITVGGST |
| 62 | VHH-9 HCDR3 | HKFHFTLGQPY |
| 63 | VHH-16 HCDR1 | IDIFRISI |
| 64 | VHH-16 HCDR2 | ISKAGTT |
| 65 | VHH-16 HCDR3 | NADRLRQHDL |
| 66 | VHH-23 HCDR1 | GIIFSIYH |
| 67 | VHH-23 HCDR2 | ITSGGST |
| 68 | VHH-23 HCDR3 | NSYHYGATRDYDA |
| 69 | VHH-43 HCDR1 | GSIFRPYV |
| 70 | VHH-43 HCDR2 | NSKGGST |
| 71 | VHH-43 HCDR3 | NLKELGIYLRGDY |
| 72 | ADI68741 VH CDR1 | GYTFTSYY |
| 73 | ADI68741 VH CDR2 | INPGTGST |
| 74 | ADI68741 VH CDR3 | ARDGAFRHKYFDL |
| 75 | ADI68741 VL CDR1 | QSVSSY |
| 76 | ADI68741 VL CDR2 | DAS |
| 77 | ADI68741 VL CDR3 | QQYSAWPLT |
| 78 | Anti-CCR8(ADI6 8741-R2-21-DA) mAb heavy chain variable region | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSS |
| 79 | CH1/CL-preferen tially mutated human IgG1 Fc amino acid sequence CH SET1 (with a Knob mutation introduced) | ASTKGPSVFPLAPSSKSTSGGTAALGCQVEDYFPEPVTVS WNSGALTSGVHTFPAVLQSSGLYELSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPCR DELTKNQVSLWCLVKGFYPSDIAVEWESNGQPENNYKTT PPVLDSDGSFFLYSKLTVDKSRWQQGNVFSCSVMHEALH NHYTQKSLSLSPG |
| 80 | CH1/CL-preferen tially mutated human IgG1 Fc amino acid sequence CH SET2 (with a Hole mutation introduced) | ASTKGPSVFPRAPSSKSTSGGTAALGCLVRDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVCTLPPSR DELTKNQVSLSCAVKGFYPSDIAVEWESNGQPENNYKTT PPVLDSDGSFFLVSKLTVDKSRWQQGNVFSCSVMHEALH NHYTQKSLSLSPG |
| 81 | CH1/CL-preferen tially mutated human κ light chain constant region (CL) amino acid sequence CL SET1 | RTVAAPSVFIFPPSDEQLKSGRASVVCLLNNFYPREAKVQ WKVDNALQSGNSQESVTEQDSKDSTYSLSSRLQLSKADY EKHKVYACEVTHQGLSSPVTKSFNRGEC |
| 82 | CH1/CL-preferen tially mutated human κ light chain constant region (CL) amino acid sequence CL SET2 | RTVAAPSVFIFPPSDEELKSGTASVQCLLNNFYPREAKVQ WKVDNALQSGNSQESVTEQDSKDSTYSLSSELTLSKADY EKHKVYACEVTHQGLSSPVTKSFNRGEC |
| 83 | Anti-CCR8(ADI6 8741-R2-21-DA) mAb heavy chain | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPSRDELTKNQVSLTCLVKGFYPSDIAVEWESNGQPENN YKTTPPVLDSDGSFFLYSKLTVDKSRWQQGNVFSCSVMH EALHNHYTQKSLSLSPG |
| 84 | Anti-CCR8(ADI6 8741-R2-21-DA) light chain | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 85 | Anti-CTLA4 (ip-05) × CCR8 -2 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYK TTPPVLDSDGSFLLYSKLTVDKSRWQQGNVFSCSVMHEA LHNHYTQKSLSLSPG |
| 86 | Anti-CTLA4 (ip-05) × CCR8 -2 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 87 | Anti-CTLA4 (ip-05) × CCR8 -2 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPSRDELTKNQVSLTCLVKGFYPSDIAVEWESNGQPENN YKTTPPVLDSDGSFFLYSRLTVDKSRWQQGNVFSCSVMH EALHNHYTQKSLSLSPG |
| 88 | Anti-CTLA4 (ip-05) × CCR8 -2 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 89 | Anti-CTLA4 (ip-0508) × CCR8 -2 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPLAPSSKSTSGGTAALGCLVKDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP SRDELTKNQVSLTCLVKGFYPSDIAVEWESNGQPENNYK TTPPVLDSDGSFLLYSKLTVDKSRWQQGNVFSCSVMHEA LHNHYTQKSLSLSPG |
| 90 | Anti-CTLA4 (ip-0508) × CCR8 -2 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |
| 91 | Anti-CTLA4 (ip-0508) × CCR8 -2 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPSRDELTKNQVSLTCLVKGFYPSDIAVEWESNGQPENN YKTTPPVLDSDGSFFLYSRLTVDKSRWQQGNVFSCSVMH EALHNHYTQKSLSLSPG |
| 92 | Anti-CTLA4 (ip-0508) × CCR8 -2 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 93 | Anti-CTLA4 (ip-05) × CCR8 -3 heavy chain 1 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCQVEDYFPEP VTVSWNSGALTSGVHTFPAVLQSSGLYELSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPG |
| 94 | Anti-CTLA4 (ip-05) × CCR8 -3 light chain 1 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGRASVVCLLNNFYPREAKVQWKVDNALQSGNSQ ESVTEQDSKDSTYSLSSRLQLSKADYEKHKVYACEVTHQ GLSSPVTKSFNRGEC |
| 95 | Anti-CTLA4 (ip-05) × CCR8 -3 heavy chain 2 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPRAPSSKSTSGGTAALGCLVRDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVCTLPP SRDELTKNQVSLSCAVKGFYPSDIAVEWESNGQPENNYK TTPPVLDSDGSFFLVSKLTVDKSRWQQGNVFSCSVMHEA LHNHYTQKSLSLSPG |
| 96 | Anti-CTLA4 (ip-05) × CCR8 -3 light chain 2 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEELKSGTASVQCLLNNFYPREAKVQWKVDNALQSGNS QESVTEQDSKDSTYSLSSELTLSKADYEKHKVYACEVTH QGLSSPVTKSFNRGEC |
| 97 | Anti-CTLA4 (ip-05) × CCR8 -4 heavy chain 1 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCQVEDYFPEP VTVSWNSGALTSGVHTFPAVLQSSGLYELSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPG |
| 98 | Anti-CTLA4 (ip-05) × CCR8 -4 light chain 1 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGRASVVCLLNNFYPREAKVQWKVDNALQSGNSQ ESVTEQDSKDSTYSLSSRLQLSKADYEKHKVYACEVTHQ GLSSPVTKSFNRGEC |
| 99 | Anti-CTLA4 (ip-05) × CCR8 -4 heavy chain 2 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPRAPSSKSTSGGTAALGCLVRDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVCTLPP SRDELTKNQVSLSCAVKGFYPSDIAVEWESNGQPENNYK TTPPVLDSDGSFFLVSKLTVDKSRWQQGNVFSCSVMHEA LHNHYTQKSLSLSPG |
| 100 | Anti-CTLA4 (ip-05) × CCR8 -4 light chain 2 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEELKSGTASVQCLLNNFYPREAKVQWKVDNALQSGNS QESVTEQDSKDSTYSLSSELTLSKADYEKHKVYACEVTH QGLSSPVTKSFNRGEC |
| 101 | Anti-CTLA4 (ip-0508) × CCR8 -3 heavy chain 1 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPLAPSSKSTSGGTAALGCQVEDYFPEP VTVSWNSGALTSGVHTFPAVLQSSGLYELSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPG |
| 102 | Anti-CTLA4 (ip-0508) × CCR8 -3 light chain 1 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGRASVVCLLNNFYPREAKVQWKVDNALQSGNSQ ESVTEQDSKDSTYSLSSRLQLSKADYEKHKVYACEVTHQ GLSSPVTKSFNRGEC |
| 103 | Anti-CTLA4 (ip-0508) × CCR8 -3 heavy chain 2 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPRAPSSKSTSGGTAALGCLVRDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVCTLPP SRDELTKNQVSLSCAVKGFYPSDIAVEWESNGQPENNYK TTPPVLDSDGSFFLVSKLTVDKSRWQQGNVFSCSVMHEA LHNHYTQKSLSLSPG |
| 104 | Anti-CTLA4 (ip-0508) × CCR8 -3 light chain 2 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEELKSGTASVQCLLNNFYPREAKVQWKVDNALQSGNS QESVTEQDSKDSTYSLSSELTLSKADYEKHKVYACEVTH QGLSSPVTKSFNRGEC |
| 105 | Anti-CTLA4 (ip-0508) × CCR8 -4 heavy chain 1 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCQVEDYFPEP VTVSWNSGALTSGVHTFPAVLQSSGLYELSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVYT LPPCRDELTKNQVSLWCLVKGFYPSDIAVEWESNGQPEN NYKTTPPVLDSDGSFFLYSKLTVDKSRWQQGNVFSCSVM HEALHNHYTQKSLSLSPG |
| 106 | Anti-CTLA4 (ip-0508) × CCR8 -4 light chain 1 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGRASVVCLLNNFYPREAKVQWKVDNALQSGNSQ ESVTEQDSKDSTYSLSSRLQLSKADYEKHKVYACEVTHQ GLSSPVTKSFNRGEC |
| 107 | Anti-CTLA4 (ip-0508) × CCR8 -4 heavy chain 2 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPRAPSSKSTSGGTAALGCLVRDYFPEPVTV SWNSGALTSGVHTFPAVLQSSGLYSLSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVCTLPP SRDELTKNQVSLSCAVKGFYPSDIAVEWESNGQPENNYK TTPPVLDSDGSFFLVSKLTVDKSRWQQGNVFSCSVMHEA LHNHYTQKSLSLSPG |
| 108 | Anti-CTLA4 (ip-0508) × CCR8 -4 light chain 2 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEELKSGTASVQCLLNNFYPREAKVQWKVDNALQSGNS QESVTEQDSKDSTYSLSSELTLSKADYEKHKVYACEVTH QGLSSPVTKSFNRGEC |
| 109 | Anti-CTLA4 (ip-05) × CCR8 -5 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPLAPSSKSTSGGTAALGCQVEDYFPEPVTV SWNSGALTSGVHTFPAVLQSSGLYELSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP CRDELTKNQVSLWCLVKGFYPSDIAVEWESNGQPENNYK TTPPVLDSDGSFFLYSKLTVDKSRWQQGNVFSCSVMHEA LHNHYTQKSLSLSPG |
| 110 | Anti-CTLA4 (ip-05) × CCR8 -5 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGRASVVCLLNNFYPREAKVQWKVDNALQSGNS QESVTEQDSKDSTYSLSSRLQLSKADYEKHKVYACEVTH QGLSSPVTKSFNRGEC |
| 111 | Anti-CTLA4 (ip-05) × CCR8 -5 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPRAPSSKSTSGGTAALGCLVRDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNHYTQKSLSLSPG |
| 112 | Anti-CTLA4 (ip-05) × CCR8 -5 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EELKSGTASVQCLLNNFYPREAKVQWKVDNALQSGNSQ ESVTEQDSKDSTYSLSSELTLSKADYEKHKVYACEVTHQ GLSSPVTKSFNRGEC |
| 113 | Anti-CTLA4 (ip-05) × CCR8 -6 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSASTKGPSVFPLAPSSKSTSGGTAALGCQVEDYFPEPVTV SWNSGALTSGVHTFPAVLQSSGLYELSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP CRDELTKNQVSLWCLVKGFYPSDIAVEWESNGQPENNYK TTPPVLDSDGSFFLYSKLTVDKSRWQQGNVFSCSVMHEA LHNHYTQKSLSLSPG |
| 114 | Anti-CTLA4 (ip-05) × CCR8 -6 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGRASVVCLLNNFYPREAKVQWKVDNALQSGNS QESVTEQDSKDSTYSLSSRLQLSKADYEKHKVYACEVTH QGLSSPVTKSFNRGEC |
| 115 | Anti-CTLA4 (ip-05) × CCR8 -6 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPRAPSSKSTSGGTAALGCLVRDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNHYTQKSLSLSPG |
| 116 | Anti-CTLA4 (ip-05) × CCR8 -6 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EELKSGTASVQCLLNNFYPREAKVQWKVDNALQSGNSQ ESVTEQDSKDSTYSLSSELTLSKADYEKHKVYACEVTHQ GLSSPVTKSFNRGEC |
| 117 | Anti-CTLA4 (ip-0508) × CCR8 -5 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPLAPSSKSTSGGTAALGCQVEDYFPEPVTV SWNSGALTSGVHTFPAVLQSSGLYELSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP CRDELTKNQVSLWCLVKGFYPSDIAVEWESNGQPENNYK TTPPVLDSDGSFFLYSKLTVDKSRWQQGNVFSCSVMHEA LHNHYTQKSLSLSPG |
| 118 | Anti-CTLA4 (ip-0508) × CCR8 -5 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGRASVVCLLNNFYPREAKVQWKVDNALQSGNS QESVTEQDSKDSTYSLSSRLQLSKADYEKHKVYACEVTH QGLSSPVTKSFNRGEC |
| 119 | Anti-CTLA4 (ip-0508) × CCR8 -5 heavy chain 2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSASTKGPSVFPRAPSSKSTSGGTAALGCLVRDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNHYTQKSLSLSPG |
| 120 | Anti-CTLA4 (ip-0508) × CCR8 -5 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EELKSGTASVQCLLNNFYPREAKVQWKVDNALQSGNSQ ESVTEQDSKDSTYSLSSELTLSKADYEKHKVYACEVTHQ GLSSPVTKSFNRGEC |
| 121 | Anti-CTLA4 (ip-0508) × CCR8 -6 heavy chain 1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSASTKGPSVFPLAPSSKSTSGGTAALGCQVEDYFPEPVTV SWNSGALTSGVHTFPAVLQSSGLYELSSVVTVPSSSLGTQ TYICNVNHKPSNTKVDKKVEPKSCDKTHTCPPCPAPELLG GPSVFLFPPKPKDTLMISRTPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLPP CRDELTKNQVSLWCLVKGFYPSDIAVEWESNGQPENNYK TTPPVLDSDGSFFLYSKLTVDKSRWQQGNVFSCSVMHEA LHNHYTQKSLSLSPG |
| 122 | Anti-CTLA4 (ip-0508) × CCR8 -6 light chain 1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRTVAAPSVFIFPPS DEQLKSGRASVVCLLNNFYPREAKVQWKVDNALQSGNS QESVTEQDSKDSTYSLSSRLQLSKADYEKHKVYACEVTH QGLSSPVTKSFNRGEC |
| 123 | Anti-CTLA4 (ip-0508) × CCR8 -6 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPRAPSSKSTSGGTAALGCLVRDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNHYTQKSLSLSPG |
| 124 | Anti-CTLA4 (ip-0508) × CCR8 -6 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EELKSGTASVQCLLNNFYPREAKVQWKVDNALQSGNSQ ESVTEQDSKDSTYSLSSELTLSKADYEKHKVYACEVTHQ GLSSPVTKSFNRGEC |
| 125 | Anti-CTLA4 (VHH-9) × CCR8 -2 heavy chain 1 | EVQLQESGGGLVQAGGSLTLSCAASGSIFSLNGMEWYRQ FPGQQRELVAAITVGGSTIYADSVKGRFTISRDNAKNTVY LQMSSLKPEDTAVYYCHKFHFTLGQPYWGQGTQVTVSS DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPCRDELTKNQVSLWCLVKGFYPSDI AVEWESNGQPENNYKTTPPVLDSDGSFFLYSKLTVDKSR WQQGNVFSCSVMHEALHNHYTQKSLSLSPG |
| 126 | Anti-CTLA4 (VHH-9) × CCR8 -2 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 127 | Anti-CTLA4 (VHH-9) × CCR8 -2 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 128 | Anti-CTLA4 (VHH-16) × CCR8 -2 heavy chain 1 | EVQLQESGGGLVQAGGALRLSCAAPIDIFRISIMGWYRQA PGQQRELVARISKAGTTTYADSVKGRFTISRDNAKNTVIL QMNSLKPEDTAVYYCNADRLRQHDLWGQGTQVTVSSD KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPCRDELTKNQVSLWCLVKGFYPSDIA VEWESNGQPENNYKTTPPVLDSDGSFFLYSKLTVDKSRW QQGNVFSCSVMHEALHNHYTQKSLSLSPG |
| 129 | Anti-CTLA4 (VHH-16) × CCR8 -2 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 130 | Anti-CTLA4 (VHH-16) × CCR8 -2 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 131 | Anti-CTLA4 (VHH-23) × CCR8 -2 heavy chain 1 | EVQLQESGGGLVQPGGSLRLSCAASGIIFSIYHMGWYRQA PGKQRELVAVITSGGSTNYADSVKGRFTISRDNAKNTVYL QMNSLKPEDTAVYYCNSYHYGATRDYDAWGQGTQVTV SSDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTI SKAKGQPREPQVYTLPPCRDELTKNQVSLWCLVKGFYPS DIAVEWESNGQPENNYKTTPPVLDSDGSFFLYSKLTVDKS RWQQGNVFSCSVMHEALHNHYTQKSLSLSPG |
| 132 | Anti-CTLA4 (VHH-23) × CCR8 -2 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 133 | Anti-CTLA4 (VHH-23) × CCR8 -2 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 134 | Anti-CTLA4 (VHH-43) × CCR8 -2 heavy chain 1 | EVQLQESGGGLVQPGGSLRLSCVASGSIFRPYVMGWYRQ APGKQRELVATNSKGGSTIYADAVKGRFTISSDNDENTV YLQMNSLKPEDTAVYYCNLKELGIYLRGDYWGQGTQVT VSSDKTHTCPPCPAPELLGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALPAPIEKT ISKAKGQPREPQVYTLPPCRDELTKNQVSLWCLVKGFYPS DIAVEWESNGQPENNYKTTPPVLDSDGSFFLYSKLTVDKS RWQQGNVFSCSVMHEALHNHYTQKSLSLSPG |
| 135 | Anti-CTLA4 (VHH-43) × CCR8 -2 heavy chain 2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKKVEPKSCDKTHTCPPCPAPE LLGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPAPIEKTISKAKGQPREPQVCT LPPSRDELTKNQVSLSCAVKGFYPSDIAVEWESNGQPENN YKTTPPVLDSDGSFFLVSKLTVDKSRWQQGNVFSCSVMH EALHNRFTQKSLSLSPG |
| 136 | Anti-CTLA4 (VHH-43) × CCR8 -2 light chain 2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| 137 | Anti-CCR8(6874 1-R2-21-DA) VH CDR1 | GYTFTSYY |
| 138 | Anti-CCR8(6874 1-R2-21-DA) VH CDR2 | INPMTGSA |
| 139 | Anti-CCR8(6874 1-R2-21-DA) VH CDR3 | VRDAAARHKYFAF |
| 140 | Murine IgG2a heavy chain amino acid sequence (with a LT mutation introduced) | AKTTAPSVYPLAPVCGDTTGSSVTLGCLVKGYFPEPVTLT WNSGSLSSGVHTFPAVLQSDLYTLSSSVTVTSSTWPSQSIT CNVAHPASSTKVDKKIEPRGPTIKPCPPCKCPAPNLLGGPS VFIFPPKIKDVLMISLSPIVTCVVVDVSEDDPDVQISWFVN NVEVHTAQTQTHREDYNSTLRVVSALPIQHQDWMSGKE FKCKVNNKDLPAPIERTISKPKGSVRAPQVYVLPPPEEEM TKKQVTLTCMVTDFMPEDIYVEWTNNGKTELNYKNTEP VLDSDGSYLMYSKLTVEKKNWVERNSYSCSVVHEGLHN HHTTKSFSRTPG |
| 141 | Murine IgG2a heavy chain amino acid sequence (with a KR/RF mutation introduced) | AKTTAPSVYPLAPVCGDTTGSSVTLGCLVKGYFPEPVTLT WNSGSLSSGVHTFPAVLQSDLYTLSSSVTVTSSTWPSQSIT CNVAHPASSTKVDKKIEPRGPTIKPCPPCKCPAPNLLGGPS VFIFPPKIKDVLMISLSPIVTCVVVDVSEDDPDVQISWFVN NVEVHTAQTQTHREDYNSTLRVVSALPIQHQDWMSGKE FKCKVNNKDLPAPIERTISKPKGSVRAPQVYVLPPPEEEM TKKQVTLTCMVKDFMPEDIYVEWTNNGKTELNYKNTEP VLDSDGSYFMYSRLRVEKKNWVERNSYSCSVVHEGLHN RFTTKSFSRTPG |
| 142 | Murine IgG2a heavy constant chain amino acid sequence | AKTTAPSVYPLAPVCGDTTGSSVTLGCLVKGYFPEPVTLT WNSGSLSSGVHTFPAVLQSDLYTLSSSVTVTSSTWPSQSIT CNVAHPASSTKVDKKIEPRGPTIKPCPPCKCPAPNLLGGPS VFIFPPKIKDVLMISLSPIVTCVVVDVSEDDPDVQISWFVN NVEVHTAQTQTHREDYNSTLRVVSALPIQHQDWMSGKE FKCKVNNKDLPAPIERTISKPKGSVRAPQVYVLPPPEEEM TKKQVTLTCMVTDFMPEDIYVEWTNNGKTELNYKNTEP VLDSDGSYFMYSKLRVEKKNWVERNSYSCSVVHEGLHN HHTTKSFSRTPG |
| 143 | Murine IgG2a light constant chain amino acid sequence | RADAAPTVSIFPPSSEQLTSGGASVVCFLNNFYPKDINVK WKIDGSERQNGVLNSWTDQDSKDSTYSMSSTLTLTKDEY ERHNSYTCEATHKTSTSPIVKSFNRNEC |
| 144 | Anti-CCR8(ADI6 8741)-2 mAb heavy chain | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSAKTTAPSVYPLAPVCGDTTGSSVTLGCLVKGYFPE PVTLTWNSGSLSSGVHTFPAVLQSDLYTLSSSVTVTSSTW PSQSITCNVAHPASSTKVDKKIEPRGPTIKPCPPCKCPAPNL LGGPSVFIFPPKIKDVLMISLSPIVTCVVVDVSEDDPDVQIS WFVNNVEVHTAQTQTHREDYNSTLRVVSALPIQHQDWM SGKEFKCKVNNKDLPAPIERTISKPKGSVRAPQVYVLPPPE EEMTKKQVTLTCMVTDFMPEDIYVEWTNNGKTELNYKN TEPVLDSDGSYFMYSKLRVEKKNWVERNSYSCSVVHEGL HNHHTTKSFSRTPG |
| 145 | Anti-CCR8(ADI6 8741)-2 mAb light chain | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRADAAPTVSIFPPSS EQLTSGGASVVCFLNNFYPKDINVKWKIDGSERQNGVLN SWTDQDSKDSTYSMSSTLTLTKDEYERHNSYTCEATHKT STSPIVKSFNRNEC |
| 146 | Anti-CTLA4(ip-0 5)-2 mAb heavy chain | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSAKTTAPSVYPLAPVCGDTTGSSVTLGCLVKGYFPEPVT LTWNSGSLSSGVHTFPAVLQSDLYTLSSSVTVTSSTWPSQ SITCNVAHPASSTKVDKKIEPRGPTIKPCPPCKCPAPNLLG GPSVFIFPPKIKDVLMISLSPIVTCVVVDVSEDDPDVQISWF VNNVEVHTAQTQTHREDYNSTLRVVSALPIQHQDWMSG KEFKCKVNNKDLPAPIERTISKPKGSVRAPQVYVLPPPEEE MTKKQVTLTCMVTDFMPEDIYVEWTNNGKTELNYKNTE PVLDSDGSYFMYSKLRVEKKNWVERNSYSCSVVHEGLH NHHTTKSFSRTPG |
| 147 | Anti-CTLA4(ip-0 5)-2 mAb light chain | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRADAAPTVSIFPPS SEQLTSGGASVVCFLNNFYPKDINVKWKIDGSERQNGVL NSWTDQDSKDSTYSMSSTLTLTKDEYERHNSYTCEATHK TSTSPIVKSFNRNEC |
| 148 | Anti-CTLA4(ip-0 5)×CCR8-7 mAb heavy chain-1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSAKTTAPSVYPLAPVCGDTTGSSVTLGCLVKGYFPEPVT LTWNSGSLSSGVHTFPAVLQSDLYTLSSSVTVTSSTWPSQ SITCNVAHPASSTKVDKKIEPRGPTIKPCPPCKCPAPNLLG GPSVFIFPPKIKDVLMISLSPIVTCVVVDVSEDDPDVQISWF VNNVEVHTAQTQTHREDYNSTLRVVSALPIQHQDWMSG KEFKCKVNNKDLPAPIERTISKPKGSVRAPQVYVLPPPEEE MTKKQVTLTCMVTDFMPEDIYVEWTNNGKTELNYKNTE PVLDSDGSYLMYSKLTVEKKNWVERNSYSCSVVHEGLH NHHTTKSFSRTPG |
| 149 | Anti-CTLA4(ip-0 5)×CCR8-7 mAb heavy chain-2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSAKTTAPSVYPLAPVCGDTTGSSVTLGCLVKGYFPE PVTLTWNSGSLSSGVHTFPAVLQSDLYTLSSSVTVTSSTW PSQSITCNVAHPASSTKVDKKIEPRGPTIKPCPPCKCPAPNL LGGPSVFIFPPKIKDVLMISLSPIVTCVVVDVSEDDPDVQIS WFVNNVEVHTAQTQTHREDYNSTLRVVSALPIQHQDWM SGKEFKCKVNNKDLPAPIERTISKPKGSVRAPQVYVLPPPE EEMTKKQVTLTCMVKDFMPEDIYVEWTNNGKTELNYKN TEPVLDSDGSYFMYSRLRVEKKNWVERNSYSCSVVHEGL HNRFTTKSFSRTPG |
| 150 | Anti-CTLA4(ip-0 5)×CCR8-7 mAb light chain-1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRADAAPTVSIFPPS SEQLTSGGASVVCFLNNFYPKDINVKWKIDGSERQNGVL NSWTDQDSKDSTYSMSSTLTLTKDEYERHNSYTCEATHK TSTSPIVKSFNRNEC |
| 151 | Anti-CTLA4(ip-0 5)×CCR8-7 mAb light chain-2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRADAAPTVSIFPPSS EQLTSGGASVVCFLNNFYPKDINVKWKIDGSERQNGVLN SWTDQDSKDSTYSMSSTLTLTKDEYERHNSYTCEATHKT STSPIVKSFNRNEC |
| 152 | Anti-CTLA4(ip-0 5)×CCR8-8 mAb heavy chain-1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPFDYWGQGTLVTV SSAKTTAPSVYPLAPVCGDTTGSSVTLGCLVKGYFPEPVT LTWNSGSLSSGVHTFPAVLQSDLYTLSSSVTVTSSTWPSQ SITCNVAHPASSTKVDKKIEPRGPTIKPCPPCKCPAPNLLG GPSVFIFPPKIKDVLMISLSPIVTCVVVDVSEDDPDVQISWF VNNVEVHTAQTQTHREDYNSTLRVVSALPIQHQDWMSG KEFKCKVNNKDLPAPIERTISKPKGSVRAPQVYVLPPPEEE MTKKQVTLTCMVTDFMPEDIYVEWTNNGKTELNYKNTE PVLDSDGSYLMYSKLTVEKKNWVERNSYSCSVVHEGLH NHHTTKSFSRTPG |
| 153 | Anti-CTLA4(ip-0 5)×CCR8-8 mAb heavy chain-2 | EVQLVQSGAEVKKPGASVKVSCKASGYTFTSYYMHWVR QAPGQGLEWMGIINPMTGSASHAQKFQGRVTMTRDTSTS TVYMELSSLRSEDTAVYYCVRDAAARHKYFAFWGRGTL VTVSS AKTTAPSVYPLAPVCGDTTGSSVTLGCLVKGYFPEPVTLT WNSGSLSSGVHTFPAVLQSDLYTLSSSVTVTSSTWPSQSIT CNVAHPASSTKVDKKIEPRGPTIKPCPPCKCPAPNLLGGPS VFIFPPKIKDVLMISLSPIVTCVVVDVSEDDPDVQISWFVN NVEVHTAQTQTHREDYNSTLRVVSALPIQHQDWMSGKE FKCKVNNKDLPAPIERTISKPKGSVRAPQVYVLPPPEEEM TKKQVTLTCMVKDFMPEDIYVEWTNNGKTELNYKNTEP VLDSDGSYFMYSRLRVEKKNWVERNSYSCSVVHEGLHN RFTTKSFSRTPG |
| 154 | Anti-CTLA4(ip-0 5)×CCR8-8 mAb light chain-1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRADAAPTVSIFPPS SEQLTSGGASVVCFLNNFYPKDINVKWKIDGSERQNGVL NSWTDQDSKDSTYSMSSTLTLTKDEYERHNSYTCEATHK TSTSPIVKSFNRNEC |
| 155 | Anti-CTLA4(ip-0 5)×CCR8-8 mAb light chain-2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRADAAPTVSIFPPSS EQLTSGGASVVCFLNNFYPKDINVKWKIDGSERQNGVLN SWTDQDSKDSTYSMSSTLTLTKDEYERHNSYTCEATHKT STSPIVKSFNRNEC |
| 156 | Anti-CTLA4(ip-0 508)×CCR8-7 mAb heavy chain-1 | EVQLVESGGGVVQPGRSLRLSCAASGFTFSSYTMHWVRQ APGKGLEWVTFISEDGNNKYYADSVKGRFTISRDNSKNT LYLQMNSLRAEDTAIYYCARTGWLGPEDYWGQGTLVTV SSAKTTAPSVYPLAPVCGDTTGSSVTLGCLVKGYFPEPVT LTWNSGSLSSGVHTFPAVLQSDLYTLSSSVTVTSSTWPSQ SITCNVAHPASSTKVDKKIEPRGPTIKPCPPCKCPAPNLLG GPSVFIFPPKIKDVLMISLSPIVTCVVVDVSEDDPDVQISWF VNNVEVHTAQTQTHREDYNSTLRVVSALPIQHQDWMSG KEFKCKVNNKDLPAPIERTISKPKGSVRAPQVYVLPPPEEE MTKKQVTLTCMVTDFMPEDIYVEWTNNGKTELNYKNTE PVLDSDGSYLMYSKLTVEKKNWVERNSYSCSVVHEGLH NHHTTKSFSRTPG |
| 157 | Anti-CTLA4(ip-0 508)×CCR8-7 mAb heavy chain-2 | EVQLVQSGAEVKKPGASVKVSCKASGYTFTSYYMHWVR QAPGQGLEWMGIINPGTGSTSHAQKFQGRVTMTRDTSTS TVYMELSSLRSEDTAVYYCARDGAFRHKYFDLWGRGTL VTVSSAKTTAPSVYPLAPVCGDTTGSSVTLGCLVKGYFPE PVTLTWNSGSLSSGVHTFPAVLQSDLYTLSSSVTVTSSTW PSQSITCNVAHPASSTKVDKKIEPRGPTIKPCPPCKCPAPNL LGGPSVFIFPPKIKDVLMISLSPIVTCVVVDVSEDDPDVQIS WFVNNVEVHTAQTQTHREDYNSTLRVVSALPIQHQDWM SGKEFKCKVNNKDLPAPIERTISKPKGSVRAPQVYVLPPPE EEMTKKQVTLTCMVKDFMPEDIYVEWTNNGKTELNYKN TEPVLDSDGSYFMYSRLRVEKKNWVERNSYSCSVVHEGL HNRFTTKSFSRTPG |
| 158 | Anti-CTLA4(ip-0 508)×CCR8-7 mAb light chain-1 | DIVLTQSPGTLSLSPGERATLSCRASQSVGSSYLAWYQQK PGQAPRLLIYGAFSRATGIPDRFSGSGSGTDFTLTISRLEPE DFAVYYCQQYGSSPWTFGQGTKVEIKRADAAPTVSIFPPS SEQLTSGGASVVCFLNNFYPKDINVKWKIDGSERQNGVL NSWTDQDSKDSTYSMSSTLTLTKDEYERHNSYTCEATHK TSTSPIVKSFNRNEC |
| 159 | Anti-CTLA4(ip-0 508)×CCR8-7 mAb light chain-2 | EIVLTQSPATLSLSPGERATLSCRASQSVSSYLAWYQQKP GQAPRLLIYDASNRATGIPARFSGSGSGTDFTLTISSLEPED FAVYYCQQYSAWPLTFGGGTKVEIKRADAAPTVSIFPPSS EQLTSGGASVVCFLNNFYPKDINVKWKIDGSERQNGVLN SWTDQDSKDSTYSMSSTLTLTKDEYERHNSYTCEATHKT STSPIVKSFNRNEC |
| 160 | ONC-392 Analog heavy chain | QVQLQESGPGLVKPSETLSLTCTVSGFSLTSYGLSWIRQPP GKGLEWIGYIWYDGNTNFHSPLKSRVTISVDTSKNQFSLK LSSVTAADTAVYYCAKTEGHYYGSNYGYYALDYWGQG TLVTVSSASTKGPSVFPLAPSSKSTSGGTAALGCLVKDYF PEPVTVSWNSGALTSGVHTFPAVLQSSGLYSLSSVVTVPS SSLGTQTYICNVNHKPSNTKVDKKVEPKSCDKTHTCPPCP APELLGGPSVFLFPPKPKDTLYITREPEVTCVVVDVSHEDP EVKFNWYVDGVEVHNAKTKPREEQYNATYRVVSVLTVL HQDWLNGKEYKCKVSNKALPAPIAATISKAKGQPREPQV YTLPPSRDELTKNQVSLTCLVKGFYPSDIAVEWESNGQPE NNYKTTPPVLDSDGSFFLYSKLTVDKSRWQQGNVFSCSV MHEALHNHYTQKSLSLSPG |
| 161 | ONC-392 Analog light chain | DIQMTQSPSSLSASVGDRVTITCRASENIYSNLAWYQQKQ GKAPKLLLYAATNLQDGVPSRFSGSGSGTDYTLTISSLQP EDFATYFCQHLWGTPYTFGQGTKLEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC |

### DETAILED DESCRIPTION

The present disclosure is now described with reference to the following Examples intended to illustrate, but not to limit, the present disclosure.

Unless otherwise specified, the experiments and methods described in the examples were performed substantially according to conventional methods well known in the art and described in various references. For example, conventional techniques in immunology, biochemistry, chemistry, molecular biology, microbiology, cell biology, genomics, and recombinant DNA used in the present disclosure may be found in Sambrook, Fritsch, and Maniatis, MOLECULAR CLONING: A LABORATORY MANUAL, 2nd edition (1989); CURRENT PROTOCOLS IN MOLECULAR BIOLOGY (F.M. Ausubel et al., eds., (1987)); the METHODS IN ENZYMOLOGY series (Academic Press, Inc.); PCR 2: A PRACTICAL APPROACH (M.J. MacPherson, B.D. Hames, and G.R. Taylor, eds. (1995)), and ANIMAL CELL CULTURE (R.I. Freshney, ed. (1987)).

Furthermore, where conditions are not specified in the Examples, the procedures are carried out according to conventional conditions or conditions recommended by the manufacturer. The reagents or instruments used, if the manufacturer is not indicated, are conventional products commercially available. It will be appreciated by those skilled in the art that the Examples illustrate the present disclosure by way of example and are not intended to limit the scope of the claimed inventions. All publications and other references mentioned herein are incorporated by reference in their entirety.

### Example 1. Cloning and Expression of Bispecific Antibodies

In this example, three CCR8 monoclonal antibodies, seven CTLA4 monoclonal antibodies, and 23 CTLA4xCCR8 bispecific antibodies were constructed. Details are as follows.

### CCR8 Monoclonal Antibodies

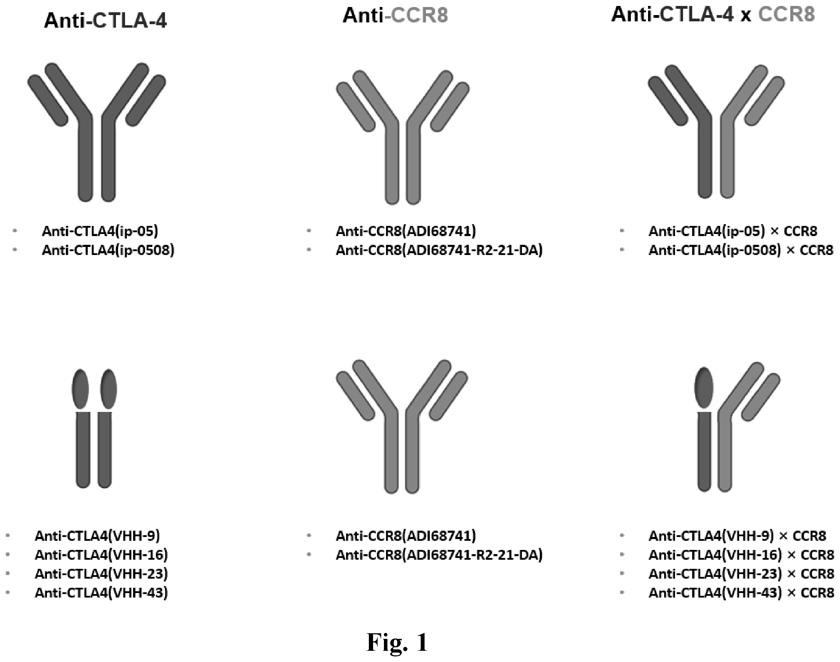

Anti-CCR8(ADI68741)-1: It is composed of 4 polypeptide chains with a structural schematic shown in Fig. 1. The heavy chain has the amino acid sequence set forth in SEQ ID NO: 18, comprising the amino acid sequence of the heavy chain variable region (SEQ ID NO: 1) of the anti-CCR8 monoclonal antibody ADI68741 and the human IgG1 amino acid sequence (SEQ ID NO: 12). The light chain has the amino acid sequence set forth in SEQ ID NO: 19, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CCR8(ADI68741)-2: It is composed of 4 polypeptide chains with a structural schematic shown in Fig. 1. The heavy chain has the amino acid sequence set forth in SEQ ID NO: 144, comprising the amino acid sequence of the heavy chain variable region (SEQ ID NO: 1) of the anti-CCR8 monoclonal antibody ADI68741 and the amino acid sequence of the murine IgG2a heavy chain constant region (SEQ ID NO: 142). The light chain has the amino acid sequence set forth in SEQ ID NO: 145, comprising the amino acid sequence of the light chain variable region (SEQ ID NO: 2) of the anti-CCR8 monoclonal antibody ADI68741, and the amino acid sequence of the murine IgG2a light chain constant region (SEQ ID NO: 143) at the C-terminus of the VL amino acid sequence.

Anti-CCR8(ADI68741-R2-21-DA): It is composed of 4 polypeptide chains with a structural schematic shown in Fig. 1. The heavy chain has the amino acid sequence set forth in SEQ ID NO: 83, comprising the amino acid sequence of the heavy chain variable region (SEQ ID NO: 78) of the anti-CCR8 monoclonal antibody ADI68741-R2-21-DA and the human IgG1 amino acid sequence (SEQ ID NO: 12). The light chain has the amino acid sequence set forth in SEQ ID NO: 84, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

### CTLA4 Monoclonal Antibodies

Anti-CTLA4(ip-05)-1: It is composed of 4 polypeptide chains with a structural schematic shown in Fig. 1. The heavy chain has the amino acid sequence set forth in SEQ ID NO: 20, comprising the amino acid sequence of the heavy chain variable region (SEQ ID NO: 3) of the anti-CTLA4 monoclonal antibody ip-05 and the human IgG1 amino acid sequence (SEQ ID NO: 12). The light chain has the amino acid sequence set forth in SEQ ID NO: 21, comprising the amino acid sequence of the light chain variable region (SEQ ID NO: 4) of the anti-CTLA4 monoclonal antibody ip-05, and the amino acid sequence of the human κ light chain constant region (CL) (SEQ ID NO: 11) at the C-terminus of the VL amino acid sequence.

Anti-CTLA4(ip-05)-2: It is composed of 4 polypeptide chains with a structural schematic shown in Fig. 1. The heavy chain has the amino acid sequence set forth in SEQ ID NO: 146, comprising the amino acid sequence of the heavy chain variable region (SEQ ID NO: 3) of the anti-CTLA4 monoclonal antibody ip-05 and the amino acid sequence of the murine IgG2a heavy chain constant region (SEQ ID NO: 142). The light chain has the amino acid sequence set forth in SEQ ID NO: 147, comprising the amino acid sequence of the light chain variable region (SEQ ID NO: 4) of the anti-CTLA4 monoclonal antibody ip-05, and the amino acid sequence of the murine IgG2a light chain constant region (SEQ ID NO: 143) at the C-terminus of the VL amino acid sequence.

Anti-CTLA4(ip-0508): It is composed of 4 polypeptide chains with a structural schematic shown in Fig. 1. The heavy chain has the amino acid sequence set forth in SEQ ID NO: 22, comprising the amino acid sequence of the heavy chain variable region (SEQ ID NO: 5) of the anti-CTLA4 monoclonal antibody ip-0508 and the human IgG1 amino acid sequence (SEQ ID NO: 12). The light chain has the amino acid sequence set forth in SEQ ID NO: 23, comprising the amino acid sequence of the light chain variable region (SEQ ID NO: 6) of the anti-CTLA4 monoclonal antibody ip-0508, and the amino acid sequence of the human κ light chain constant region (CL) (SEQ ID NO: 11) at the C-terminus of the VL amino acid sequence.

Anti-CTLA4(VHH-9): It is composed of 2 polypeptide chains with a structural schematic shown in Fig. 1. The antibody has the amino acid sequence set forth in SEQ ID NO: 24, comprising the CTLA4 binding region amino acid sequence (SEQ ID NO: 7) of the anti-CTLA4 single domain antibody VHH-9 and the human IgG1 Fc amino acid sequence (SEQ ID NO: 15).

Anti-CTLA4(VHH-16): It is composed of 2 polypeptide chains with a structural schematic shown in Fig. 1. The antibody has the amino acid sequence set forth in SEQ ID NO: 25, comprising the CTLA4 binding region amino acid sequence (SEQ ID NO: 8) of the anti-CTLA4 single domain antibody VHH-16 and the human IgG1 Fc amino acid sequence (SEQ ID NO: 15).

Anti-CTLA4(VHH-23): It is composed of 2 polypeptide chains with a structural schematic shown in Fig. 1. The antibody has the amino acid sequence set forth in SEQ ID NO: 26, comprising the CTLA4 binding region amino acid sequence (SEQ ID NO: 9) of the anti-CTLA4 single domain antibody VHH-23 and the human IgG1 Fc amino acid sequence (SEQ ID NO: 15).

Anti-CTLA4(VHH-43): It is composed of 2 polypeptide chains with a structural schematic shown in Fig. 1. The antibody has the amino acid sequence set forth in SEQ ID NO: 27, comprising the CTLA4 binding region amino acid sequence (SEQ ID NO: 10) of the anti-CTLA4 single domain antibody VHH-43 and the human IgG1 Fc amino acid sequence (SEQ ID NO: 15).

### CTLA4xCCR8 Bispecific Antibodies

Anti-CTLA4(ip-05)xCCR8-1: It is composed of 4 polypeptide chains with a structural schematic shown in Fig. 1. Peptide chain #1 was synthesized according to the method described in patent (Patent Application No.: WO2011/131746 A2) and has the amino acid sequence set forth in SEQ ID NO: 28, which comprises the amino acid sequence of the heavy chain variable region (SEQ ID NO: 3) of the anti-CTLA4 monoclonal antibody ip-05 and the human IgG1 amino acid sequence capable of forming a heterodimer (SEQ ID NO: 13, Patent Application No.: WO2011/131746 A2). Peptide chain #2 has the amino acid sequence set forth in SEQ ID NO: 29, comprising the amino acid sequence of the light chain variable region (SEQ ID NO: 4) of the anti-CTLA4 monoclonal antibody ip-05, and the amino acid sequence of the human κ light chain constant region (CL) (SEQ ID NO: 11) at the C-terminus of the VL amino acid sequence. Peptide chain #3 was synthesized according to the method described in patent (Patent Application No.: WO2011/131746 A2) and has the amino acid sequence set forth in SEQ ID NO: 30, which comprises the amino acid sequence of the heavy chain variable region (SEQ ID NO: 1) of the anti-CCR8 monoclonal antibody ADI68741 and the human IgG1 amino acid sequence capable of forming a heterodimer (SEQ ID NO: 14, Patent Application No.: WO2011/131746 A2). Peptide chain #4 has the amino acid sequence set forth in SEQ ID NO: 31, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(ip-05)xCCR8-2: It is composed of 4 polypeptide chains with a structural schematic shown in Fig. 1. Peptide chain #1 was synthesized according to the method described in patent (Patent Application No.: WO2011/131746 A2) and has the amino acid sequence set forth in SEQ ID NO: 85, which comprises the amino acid sequence of the heavy chain variable region (SEQ ID NO: 3) of the anti-CTLA4 monoclonal antibody ip-05 and the human IgG1 amino acid sequence capable of forming a heterodimer (SEQ ID NO: 13, Patent Application No.: WO2011/131746 A2). Peptide chain #2 has the amino acid sequence set forth in SEQ ID NO: 86, comprising the amino acid sequence of the light chain variable region (SEQ ID NO: 4) of the anti-CTLA4 monoclonal antibody ip-05, and the amino acid sequence of the human κ light chain constant region (CL) (SEQ ID NO: 11) at the C-terminus of the VL amino acid sequence. Peptide chain #3 was synthesized according to the method described in patent (Patent Application No.: WO2011/131746 A2) and has the amino acid sequence set forth in SEQ ID NO: 87, which comprises the amino acid sequence of the heavy chain variable region (SEQ ID NO: 78) of the anti-CCR8 monoclonal antibody ADI68741-R2-21-DA and the human IgG1 amino acid sequence capable of forming a heterodimer (SEQ ID NO: 14, Patent Application No.: WO2011/131746 A2). Peptide chain #4 has the amino acid sequence set forth in SEQ ID NO: 88, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(ip-05)xCCR8-3: It is composed of 4 polypeptide chains with a structural schematic shown in Fig. 1. Peptide chain #1 has the amino acid sequence set forth in SEQ ID NO: 95, comprising the amino acid sequence of the heavy chain variable region (SEQ ID NO: 3) of the anti-CTLA-4 monoclonal antibody ip-05 and the human IgG1 amino acid sequence with CH3 Hole mutation and CH1/CL-preferential mutation CH SET2 introduced (Patent Application No.: WO2021067404A2, SEQ ID NO: 80). Peptide chain #2 has the amino acid sequence set forth in SEQ ID NO: 96, comprising the amino acid sequence of the light chain variable region (SEQ ID NO: 4) of the anti-CTLA4 monoclonal antibody ip-05, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence. Peptide chain #3 has the amino acid sequence set forth in SEQ ID NO: 93, comprising the amino acid sequence of the heavy chain variable region (SEQ ID NO: 1) of the anti-CCR8 monoclonal antibody ADI68741 and the human IgG1 amino acid sequence with CH3 Knob mutation and CH1/CL-preferential mutation CH SET1 introduced (Patent Application No.: WO2021067404A2, SEQ ID NO: 79). Peptide chain #4 has the amino acid sequence set forth in SEQ ID NO: 94, comprising the amino acid sequence of the light chain variable region of the anti-CCR8 monoclonal antibody ADI68741 (SEQ ID NO: 2), and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence.

Anti-CTLA4(ip-05)xCCR8-4: It is composed of 4 polypeptide chains with a structural schematic shown in Fig. 1. Peptide chain #1 has the amino acid sequence set forth in SEQ ID NO: 99, comprising the amino acid sequence of the heavy chain variable region (SEQ ID NO: 3) of the anti-CTLA4 monoclonal antibody ip-05 and the human IgG1 amino acid sequence with CH3 Hole mutation and CH1/CL-preferential mutation CH SET2 introduced (Patent Application No.: WO2021067404A2, SEQ ID NO: 80). Peptide chain #2 has the amino acid sequence set forth in SEQ ID NO: 100, comprising the amino acid sequence of the light chain variable region (SEQ ID NO: 4) of the anti-CTLA4 monoclonal antibody ip-05, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence. Peptide chain #3 has the amino acid sequence set forth in SEQ ID NO: 97, comprising the amino acid sequence of the heavy chain variable region (SEQ ID NO: 78) of the anti-CCR8 monoclonal antibody ADI68741-R2-21-DA and the human IgG1 amino acid sequence with CH3 Knob mutation and CH1/CL-preferential mutation CH SET1 introduced (Patent Application No.: WO2021067404A2, SEQ ID NO: 79). Peptide chain #4 has the amino acid sequence set forth in SEQ ID NO: 98, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence.

Anti-CTLA4(ip-05)xCCR8-5: It is composed of 4 polypeptide chains with a structural schematic shown in Fig. 1. Peptide chain #1 has the amino acid sequence set forth in SEQ ID NO: 109, comprising the amino acid sequence of the heavy chain variable region (SEQ ID NO: 3) of the anti-CTLA4 monoclonal antibody ip-05 and the human IgG1 amino acid sequence with CH3 Knob mutation and CH1/CL-preferential mutation CH SET1 introduced (Patent Application No.: WO2021067404A2, SEQ ID NO: 79). Peptide chain #2 has the amino acid sequence set forth in SEQ ID NO: 110, comprising the amino acid sequence of the light chain variable region (SEQ ID NO: 4) of the anti-CTLA4 monoclonal antibody ip-05, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence. Peptide chain #3 has the amino acid sequence set forth in SEQ ID NO: 111, comprising the amino acid sequence of the heavy chain variable region (SEQ ID NO: 1) of the anti-CCR8 monoclonal antibody ADI68741 and the human IgG1 amino acid sequence with CH3 Hole mutation and CH1/CL-preferential mutation CH SET2 introduced (Patent Application No.: WO2021067404A2, SEQ ID NO: 80). Peptide chain #4 has the amino acid sequence set forth in SEQ ID NO: 112, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence.

Anti-CTLA4(ip-05)xCCR8-6: It is composed of 4 polypeptide chains with a structural schematic shown in Fig. 1. Peptide chain #1 has the amino acid sequence set forth in SEQ ID NO: 113, comprising the amino acid sequence of the heavy chain variable region (SEQ ID NO: 3) of the anti-CTLA4 monoclonal antibody ip-05 and the human IgG1 amino acid sequence with CH3 Knob mutation and CH1/CL-preferential mutation CH SET1 introduced (Patent Application No.: WO2021067404A2, SEQ ID NO: 79). Peptide chain #2 has the amino acid sequence set forth in SEQ ID NO: 114, comprising the amino acid sequence of the light chain variable region (SEQ ID NO: 4) of the anti-CTLA4 monoclonal antibody ip-05, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence. Peptide chain #3 has the amino acid sequence set forth in SEQ ID NO: 115, comprising the amino acid sequence of the heavy chain variable region (SEQ ID NO: 78) of the anti-CCR8 monoclonal antibody ADI68741-R2-21-DA and the human IgG1 amino acid sequence with CH3 Hole mutation and CH1/CL-preferential mutation CH SET2 introduced (Patent Application No.: WO2021067404A2, SEQ ID NO: 80). Peptide chain #4 has the amino acid sequence set forth in SEQ ID NO: 116, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence.

Anti-CTLA4(ip-05)xCCR8-7: It is composed of 4 polypeptide chains with a structural schematic shown in Fig. 1. Peptide chain #1 has the amino acid sequence set forth in SEQ ID NO: 148, comprising the amino acid sequence of the heavy chain variable region (SEQ ID NO: 3) of the anti-CTLA4 monoclonal antibody ip-05 and the amino acid sequence of the murine IgG2a heavy chain constant region capable of forming a heterodimer (SEQ ID NO: 140). Peptide chain #2 has the amino acid sequence set forth in SEQ ID NO: 150, comprising the amino acid sequence of the light chain variable region (SEQ ID NO: 4) of the anti-CTLA4 monoclonal antibody ip-05, and the amino acid sequence of the murine IgG2a light chain constant region (SEQ ID NO: 143) at the C-terminus of the VL amino acid sequence. Peptide chain #3 has the amino acid sequence set forth in SEQ ID NO: 149, comprising the amino acid sequence of the heavy chain variable region (SEQ ID NO: 1) of the anti-CCR8 monoclonal antibody ADI68741 and the amino acid sequence of the murine IgG2a heavy chain constant region capable of forming a heterodimer (SEQ ID NO: 141). Peptide chain #4 has the amino acid sequence set forth in SEQ ID NO: 151, comprising the amino acid sequence of the light chain variable region (SEQ ID NO: 2) of the anti-CCR8 monoclonal antibody ADI68741, and the amino acid sequence of the murine IgG2a light chain constant region (SEQ ID NO: 143) at the C-terminus of the VL amino acid sequence.

Anti-CTLA4(ip-05)xCCR8-8: It is composed of 4 polypeptide chains with a structural schematic shown in Fig. 1. Peptide chain #1 has the amino acid sequence set forth in SEQ ID NO: 152, comprising the amino acid sequence of the heavy chain variable region (SEQ ID NO: 3) of the anti-CTLA4 monoclonal antibody ip-05 and the amino acid sequence of the murine IgG2a heavy chain constant region capable of forming a heterodimer (SEQ ID NO: 140). Peptide chain #2 has the amino acid sequence set forth in SEQ ID NO: 154, comprising the amino acid sequence of the light chain variable region (SEQ ID NO: 4) of the anti-CTLA4 monoclonal antibody ip-05, and the amino acid sequence of the murine IgG2a light chain constant region (SEQ ID NO: 143) at the C-terminus of the VL amino acid sequence. Peptide chain #3 has the amino acid sequence set forth in SEQ ID NO: 153, comprising the amino acid sequence of the heavy chain variable region (SEQ ID NO: 78) of the anti-CCR8 monoclonal antibody ADI68741-R2-21-DA and the murine IgG2a amino acid sequence capable of forming a heterodimer (SEQ ID NO: 141). Peptide chain #4 has the amino acid sequence set forth in SEQ ID NO: 155, comprising the amino acid sequence of the light chain variable region (SEQ ID NO: 2) of the anti-CCR8 monoclonal antibody ADI68741, and the amino acid sequence of the murine IgG2a light chain constant region (SEQ ID NO: 143) at the C-terminus of the VL amino acid sequence.

Anti-CTLA4(ip-0508)xCCR8-1: It is composed of 4 polypeptide chains with a structural schematic shown in Fig. 1. Peptide chain #1 was synthesized according to the method described in patent (Patent Application No.: WO2011/131746 A2) and has the amino acid sequence set forth in SEQ ID NO: 32, which comprises the amino acid sequence of the heavy chain variable region (SEQ ID NO: 5) of the anti-CTLA4 monoclonal antibody ip-0508 and the human IgG1 amino acid sequence capable of forming a heterodimer (SEQ ID NO: 13, Patent Application No.: WO2011/131746 A2). Peptide chain #2 has the amino acid sequence set forth in SEQ ID NO: 33, comprising the amino acid sequence of the light chain variable region (SEQ ID NO: 6) of the anti-CTLA4 monoclonal antibody ip-0508, and the amino acid sequence of the human κ light chain constant region (CL) (SEQ ID NO: 11) at the C-terminus of the VL amino acid sequence. Peptide chain #3 was synthesized according to the method described in patent (Patent Application No.: WO2011/131746 A2) and has the amino acid sequence set forth in SEQ ID NO: 34, which comprises the amino acid sequence of the heavy chain variable region (SEQ ID NO: 1) of the anti-CCR8 monoclonal antibody ADI68741 and the human IgG1 amino acid sequence capable of forming a heterodimer (SEQ ID NO: 14, Patent Application No.: WO2011/131746 A2). Peptide chain #4 has the amino acid sequence set forth in SEQ ID NO: 35, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(ip-0508)xCCR8-2: It is composed of 4 polypeptide chains with a structural schematic shown in Fig. 1. Peptide chain #1 was synthesized according to the method described in patent (Patent Application No.: WO2011/131746 A2) and has the amino acid sequence set forth in SEQ ID NO: 89, which comprises the amino acid sequence of the heavy chain variable region (SEQ ID NO: 5) of the anti-CTLA4 monoclonal antibody ip-0508 and the human IgG1 amino acid sequence capable of forming a heterodimer (SEQ ID NO: 13, Patent Application No.: WO2011/131746 A2). Peptide chain #2 has the amino acid sequence set forth in SEQ ID NO: 90, comprising the amino acid sequence of the light chain variable region (SEQ ID NO: 6) of the anti-CTLA4 monoclonal antibody ip-0508, and the amino acid sequence of the human κ light chain constant region (CL) (SEQ ID NO: 11) at the C-terminus of the VL amino acid sequence. Peptide chain #3 was synthesized according to the method described in patent (Patent Application No.: WO2011/131746 A2) and has the amino acid sequence set forth in SEQ ID NO: 91, which comprises the amino acid sequence of the heavy chain variable region (SEQ ID NO: 78) of the anti-CCR8 monoclonal antibody ADI68741-R2-21-DA and the human IgG1 amino acid sequence capable of forming a heterodimer (SEQ ID NO: 14, Patent Application No.: WO2011/131746 A2). Peptide chain #4 has the amino acid sequence set forth in SEQ ID NO: 92, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(ip-0508)xCCR8-3: It is composed of 4 polypeptide chains with a structural schematic shown in Fig. 1. Peptide chain #1 has the amino acid sequence set forth in SEQ ID NO: 103, comprising the amino acid sequence of the heavy chain variable region (SEQ ID NO: 5) of the anti-CTLA-4 monoclonal antibody ip-0508 and the human IgG1 amino acid sequence with CH3 Hole mutation and CH1/CL-preferential mutation CH SET2 introduced (Patent Application No.: WO2021067404A2, SEQ ID NO: 80). Peptide chain #2 has the amino acid sequence set forth in SEQ ID NO: 104, comprising the amino acid sequence of the light chain variable region (SEQ ID NO: 6) of the anti-CTLA4 monoclonal antibody ip-0508, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence. Peptide chain #3 has the amino acid sequence set forth in SEQ ID NO: 101, comprising the amino acid sequence of the heavy chain variable region (SEQ ID NO: 1) of the anti-CCR8 monoclonal antibody ADI68741 and the human IgG1 amino acid sequence with CH3 Knob mutation and CH1/CL-preferential mutation CH SET1 introduced (Patent Application No.: WO2021067404A2, SEQ ID NO: 79). Peptide chain #4 has the amino acid sequence set forth in SEQ ID NO: 102, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence.

Anti-CTLA4(ip-0508)xCCR8-4: It is composed of 4 polypeptide chains with a structural schematic shown in Fig. 1. Peptide chain #1 has the amino acid sequence set forth in SEQ ID NO: 107, comprising the amino acid sequence of the heavy chain variable region (SEQ ID NO: 5) of the anti-CTLA-4 monoclonal antibody ip-0508 and the human IgG1 amino acid sequence with CH3 Hole mutation and CH1/CL-preferential mutation CH SET2 introduced (Patent Application No.: WO2021067404A2, SEQ ID NO: 80). Peptide chain #2 has the amino acid sequence set forth in SEQ ID NO: 108, comprising the amino acid sequence of the light chain variable region (SEQ ID NO: 6) of the anti-CTLA4 monoclonal antibody ip-0508, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence. Peptide chain #3 has the amino acid sequence set forth in SEQ ID NO: 105, comprising the amino acid sequence of the heavy chain variable region (SEQ ID NO: 78) of the anti-CCR8 monoclonal antibody ADI68741-R2-21-DA and the human IgG1 amino acid sequence with CH3 Knob mutation and CH1/CL-preferential mutation CH SET1 introduced (Patent Application No.: WO2021067404A2, SEQ ID NO: 79). Peptide chain #4 has the amino acid sequence set forth in SEQ ID NO: 106, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence.

Anti-CTLA4(ip-0508)xCCR8-5: It is composed of 4 polypeptide chains with a structural schematic shown in Fig. 1. Peptide chain #1 has the amino acid sequence set forth in SEQ ID NO: 117, comprising the amino acid sequence of the heavy chain variable region (SEQ ID NO: 5) of the anti-CTLA4 monoclonal antibody ip-0508 and the human IgG1 amino acid sequence with CH3 Knob mutation and CH1/CL-preferential mutation CH SET1 introduced (Patent Application No.: WO2021067404A2, SEQ ID NO: 79). Peptide chain #2 has the amino acid sequence set forth in SEQ ID NO: 118, comprising the amino acid sequence of the light chain variable region (SEQ ID NO: 6) of the anti-CTLA4 monoclonal antibody ip-0508, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence. Peptide chain #3 has the amino acid sequence set forth in SEQ ID NO: 119, comprising the amino acid sequence of the heavy chain variable region (SEQ ID NO: 1) of the anti-CCR8 monoclonal antibody ADI68741 and the human IgG1 amino acid sequence with CH3 Hole mutation and CH1/CL-preferential mutation CH SET2 introduced (Patent Application No.: WO2021067404A2, SEQ ID NO: 80). Peptide chain #4 has the amino acid sequence set forth in SEQ ID NO: 120, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence.

Anti-CTLA4(ip-0508)xCCR8-6: It is composed of 4 polypeptide chains with a structural schematic shown in Fig. 1. Peptide chain #1 has the amino acid sequence set forth in SEQ ID NO: 121, comprising the amino acid sequence of the heavy chain variable region (SEQ ID NO: 5) of the anti-CTLA4 monoclonal antibody ip-0508 and the human IgG1 amino acid sequence with CH3 Knob mutation and CH1/CL-preferential mutation CH SET1 introduced (Patent Application No.: WO2021067404A2, SEQ ID NO: 79). Peptide chain #2 has the amino acid sequence set forth in SEQ ID NO: 122, comprising the amino acid sequence of the light chain variable region (SEQ ID NO: 6) of the anti-CTLA4 monoclonal antibody ip-0508, and the amino acid sequence of the human κ light chain constant region (CL) with CH1/CL-preferential mutation CL SET1 introduced (Patent Application No.: WO2021067404A2, SEQ ID NO: 81) at the C-terminus of the VL amino acid sequence. Peptide chain #3 has the amino acid sequence set forth in SEQ ID NO: 123, comprising the amino acid sequence of the heavy chain variable region (SEQ ID NO: 78) of the anti-CCR8 monoclonal antibody ADI68741-R2-21-DA and the human IgG1 amino acid sequence with CH3 Hole mutation and CH1/CL-preferential mutation CH SET2 introduced (Patent Application No.: WO2021067404A2, SEQ ID NO: 80). Peptide chain #4 has the amino acid sequence set forth in SEQ ID NO: 124, comprising the amino acid sequence of the light chain variable region (SEQ ID NO: 2) of the anti-CCR8 monoclonal antibody ADI68741, and the amino acid sequence of the human κ light chain constant region (CL) with CH1/CL-preferential mutation CL SET2 introduced (Patent Application No.: WO2021067404A2, SEQ ID NO: 82) at the C-terminus of the VL amino acid sequence.

Anti-CTLA4(ip-0508)xCCR8-7: It is composed of 4 polypeptide chains with a structural schematic shown in Fig. 1. Peptide chain #1 has the amino acid sequence set forth in SEQ ID NO: 156, comprising the amino acid sequence of the heavy chain variable region (SEQ ID NO: 5) of the anti-CTLA4 monoclonal antibody ip-0508 and the amino acid sequence of the murine IgG2a heavy chain constant region capable of forming a heterodimer (SEQ ID NO: 140). Peptide chain #2 has the amino acid sequence set forth in SEQ ID NO: 158, comprising the amino acid sequence of the light chain variable region (SEQ ID NO: 6) of the anti-CTLA4 monoclonal antibody ip-0508, and the amino acid sequence of the murine IgG2a light chain constant region (SEQ ID NO: 143) at the C-terminus of the VL amino acid sequence. Peptide chain #3 has the amino acid sequence set forth in SEQ ID NO: 157, comprising the amino acid sequence of the heavy chain variable region (SEQ ID NO: 1) of the anti-CCR8 monoclonal antibody ADI68741 and the amino acid sequence of the murine IgG2a heavy chain constant region capable of forming a heterodimer (SEQ ID NO: 141). Peptide chain #4 has the amino acid sequence set forth in SEQ ID NO: 159, comprising the amino acid sequence of the light chain variable region (SEQ ID NO: 2) of the anti-CCR8 monoclonal antibody ADI68741, and the amino acid sequence of the murine IgG2a light chain constant region (SEQ ID NO: 143) at the C-terminus of the VL amino acid sequence.

Anti-CTLA4(VHH-9)xCCR8-1: It is composed of 3 polypeptide chains with a structural schematic shown in Fig. 1. Peptide chain #1 was synthesized according to the method described in the patent and has the amino acid sequence set forth in SEQ ID NO: 36, which comprises the CTLA4 binding region amino acid sequence (SEQ ID NO: 7) of the anti-CTLA4 single domain antibody VHH-9 and the human IgG1 Fc amino acid sequence with CH3 Knob mutation introduced (SEQ ID NO: 16). Peptide chain #2 has the amino acid sequence set forth in SEQ ID NO: 37, comprising the amino acid sequence of the heavy chain variable region (SEQ ID NO: 1) of the anti-CCR8 monoclonal antibody ADI68741 and the human IgG1 amino acid sequence with CH3 Hole mutation introduced (SEQ ID NO: 17). Peptide chain #3 has the amino acid sequence set forth in SEQ ID NO: 38, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-9)xCCR8-2: It is composed of 3 polypeptide chains with a structural schematic shown in Fig. 1. Peptide chain #1 was synthesized according to the method described in the patent and has the amino acid sequence set forth in SEQ ID NO: 125, which comprises the CTLA4 binding region amino acid sequence (SEQ ID NO: 7) of the anti-CTLA4 single domain antibody VHH-9 and the human IgG1 Fc amino acid sequence with CH3 Knob mutation introduced (SEQ ID NO: 16). Peptide chain #2 has the amino acid sequence set forth in SEQ ID NO: 126, comprising the amino acid sequence of the heavy chain variable region (SEQ ID NO: 78) of the anti-CCR8 monoclonal antibody ADI68741-R2-21-DA and the human IgG1 amino acid sequence with CH3 Hole mutation introduced (SEQ ID NO: 17). Peptide chain #3 has the amino acid sequence set forth in SEQ ID NO: 127, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-16)xCCR8-1: It is composed of 3 polypeptide chains with a structural schematic shown in Fig. 1. Peptide chain #1 was synthesized according to the method described in the patent and has the amino acid sequence set forth in SEQ ID NO: 39, which comprises the CTLA4 binding region amino acid sequence (SEQ ID NO: 8) of the anti-CTLA4 single domain antibody VHH-16 and the human IgG1 Fc amino acid sequence with CH3 Knob mutation introduced (SEQ ID NO: 16). Peptide chain #2 has the amino acid sequence set forth in SEQ ID NO: 40, comprising the amino acid sequence of the heavy chain variable region (SEQ ID NO: 1) of the anti-CCR8 monoclonal antibody ADI68741 and the human IgG1 amino acid sequence with CH3 Hole mutation introduced (SEQ ID NO: 17). Peptide chain #3 has the amino acid sequence set forth in SEQ ID NO: 41, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-16)xCCR8-2: It is composed of 3 polypeptide chains with a structural schematic shown in Fig. 1. Peptide chain #1 was synthesized according to the method described in the patent and has the amino acid sequence set forth in SEQ ID NO: 128, which comprises the CTLA4 binding region amino acid sequence (SEQ ID NO: 8) of the anti-CTLA4 single domain antibody VHH-16 and the human IgG1 Fc amino acid sequence with CH3 Knob mutation introduced (SEQ ID NO: 16). Peptide chain #2 has the amino acid sequence set forth in SEQ ID NO: 129, comprising the amino acid sequence of the heavy chain variable region (SEQ ID NO: 78) of the anti-CCR8 monoclonal antibody ADI68741-R2-21-DA and the human IgG1 amino acid sequence with CH3 Hole mutation introduced (SEQ ID NO: 17). Peptide chain #3 has the amino acid sequence set forth in SEQ ID NO: 130, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-23)xCCR8-1: It is composed of 3 polypeptide chains with a structural schematic shown in Fig. 1. Peptide chain #1 was synthesized according to the method described in the patent and has the amino acid sequence set forth in SEQ ID NO: 42, which comprises the CTLA4 binding region amino acid sequence (SEQ ID NO: 9) of the anti-CTLA4 single domain antibody VHH-23 and the human IgG1 Fc amino acid sequence with CH3 Knob mutation introduced (SEQ ID NO: 16). Peptide chain #2 has the amino acid sequence set forth in SEQ ID NO: 43, comprising the amino acid sequence of the heavy chain variable region (SEQ ID NO: 1) of the anti-CCR8 monoclonal antibody ADI68741 and the human IgG1 amino acid sequence with CH3 Hole mutation introduced (SEQ ID NO: 17). Peptide chain #3 has the amino acid sequence set forth in SEQ ID NO: 44, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-23)xCCR8-2: It is composed of 3 polypeptide chains with a structural schematic shown in Fig. 1. Peptide chain #1 was synthesized according to the method described in the patent and has the amino acid sequence set forth in SEQ ID NO: 131, which comprises the CTLA4 binding region amino acid sequence (SEQ ID NO: 9) of the anti-CTLA4 single domain antibody VHH-23 and the human IgG1 Fc amino acid sequence with CH3 Knob mutation introduced (SEQ ID NO: 16). Peptide chain #2 has the amino acid sequence set forth in SEQ ID NO: 132, comprising the amino acid sequence of the heavy chain variable region (SEQ ID NO: 78) of the anti-CCR8 monoclonal antibody ADI68741-R2-21-DA and the human IgG1 amino acid sequence with CH3 Hole mutation introduced (SEQ ID NO: 17). Peptide chain #3 has the amino acid sequence set forth in SEQ ID NO: 133, comprising the amino acid sequence (SEQ ID NO: 2) of the light chain variable region of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-43)xCCR8-1: It is composed of 3 polypeptide chains with a structural schematic shown in Fig. 1. Peptide chain #1 was synthesized according to the method described in the patent and has the amino acid sequence set forth in SEQ ID NO: 45, which comprises the CTLA4 binding region amino acid sequence (SEQ ID NO: 10) of the anti-CTLA4 single domain antibody VHH-43 and the human IgG1 Fc amino acid sequence with CH3 Knob mutation introduced (SEQ ID NO: 16). Peptide chain #2 has the amino acid sequence set forth in SEQ ID NO: 46, comprising the amino acid sequence of the heavy chain variable region (SEQ ID NO: 1) of the anti-CCR8 monoclonal antibody ADI68741 and the human IgG1 amino acid sequence with CH3 Hole mutation introduced (SEQ ID NO: 17). Peptide chain #3 has the amino acid sequence set forth in SEQ ID NO: 47, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Anti-CTLA4(VHH-43)xCCR8-2: It is composed of 3 polypeptide chains with a structural schematic shown in Fig. 1. Peptide chain #1 was synthesized according to the method described in the patent and has the amino acid sequence set forth in SEQ ID NO: 134, which comprises the CTLA4 binding region amino acid sequence (SEQ ID NO: 10) of the anti-CTLA4 single domain antibody VHH-43 and the human IgG1 Fc amino acid sequence with CH3 Knob mutation introduced (SEQ ID NO: 16). Peptide chain #2 has the amino acid sequence set forth in SEQ ID NO: 135, comprising the amino acid sequence of the heavy chain variable region (SEQ ID NO: 78) of the anti-CCR8 monoclonal antibody ADI68741-R2-21-DA and the human IgG1 amino acid sequence with CH3 Hole mutation introduced (SEQ ID NO: 17). Peptide chain #3 has the amino acid sequence set forth in SEQ ID NO: 136, comprising the amino acid sequence of the light chain variable region (SEQ ID NO: 2) of the anti-CCR8 monoclonal antibody ADI68741, and the amino acid sequence of the human κ light chain constant region (CL) (SEQ ID NO: 11) at the C-terminus of the VL amino acid sequence.

Control molecule ONC-392 Analog (Patent Application No.: WO2024059833A1) is composed of 2 polypeptide chains, in which the heavy chain has the amino acid sequence set forth in SEQ ID NO: 160, and the light chain has the amino acid sequence set forth in SEQ ID NO: 161.

### Example 2. Binding of CTLA4xCCR8 Bispecific Antibodies to CTLA4/CCR8 Cells

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were adjusted to a cell density of 2×10⁶ cells/ml with 2% BSA solution, and 100 µl/well was added to a 96-well flow cytometry plate, and centrifuged for later use. The antibodies after gradient dilution were added to the 96-well flow cytometry plate containing the above cells at 100 µl/well, and incubated at 4°C for 60 min. After washing twice with PBS, 100 µl/well of Goat anti-human IgG-Fc (PE) (Abcam, ab98596) diluted 1000-fold with 2% BSA solution was added, and incubated at 4°C for 60 min. After washing twice with PBS, the cells were finally resuspended by adding PBS at 100 µl/well, and detected on a CytoFlex (Beckman) flow cytometer, and the respective MFI was calculated.

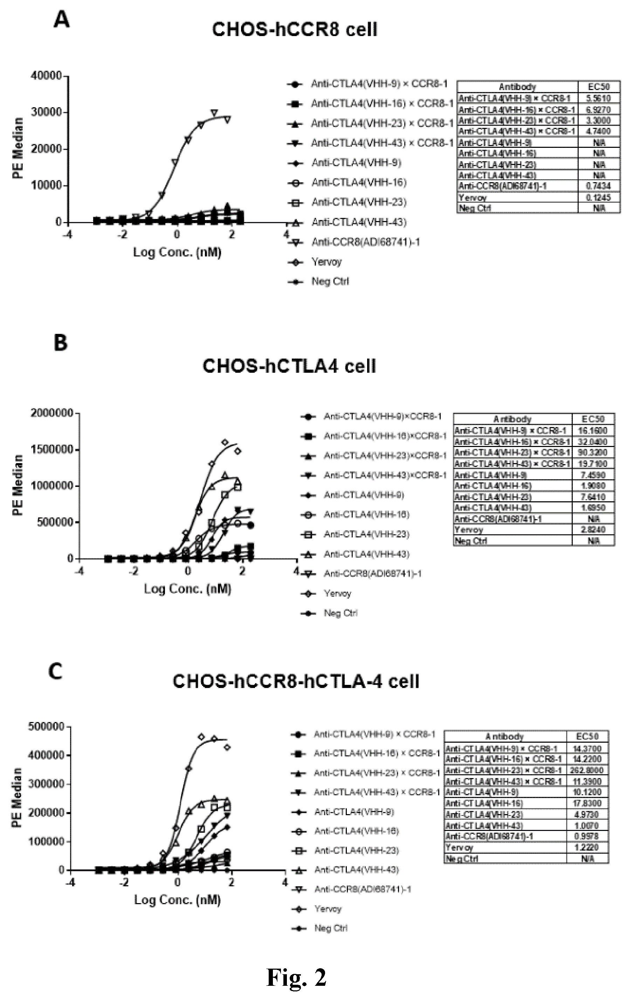

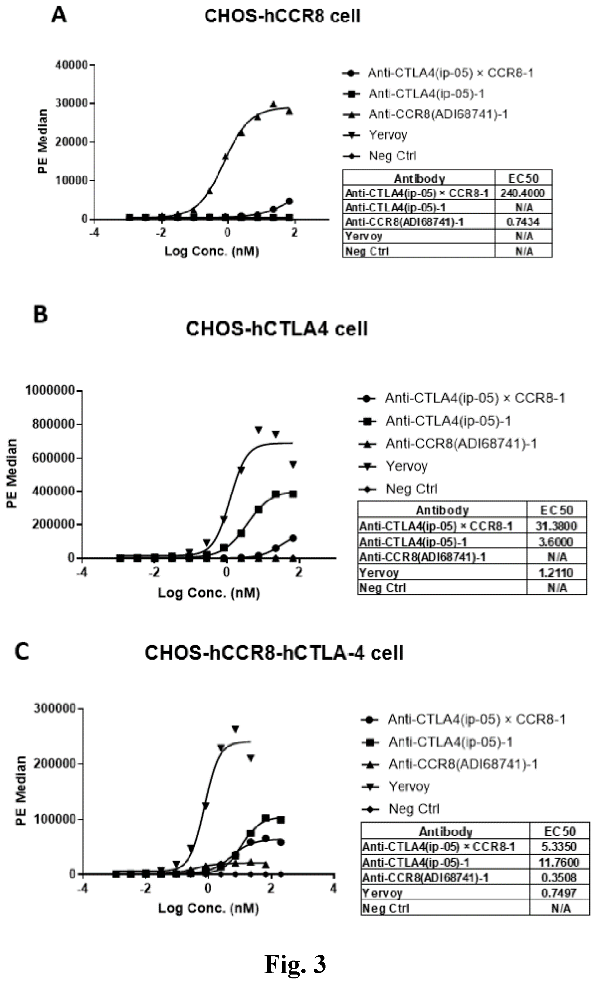

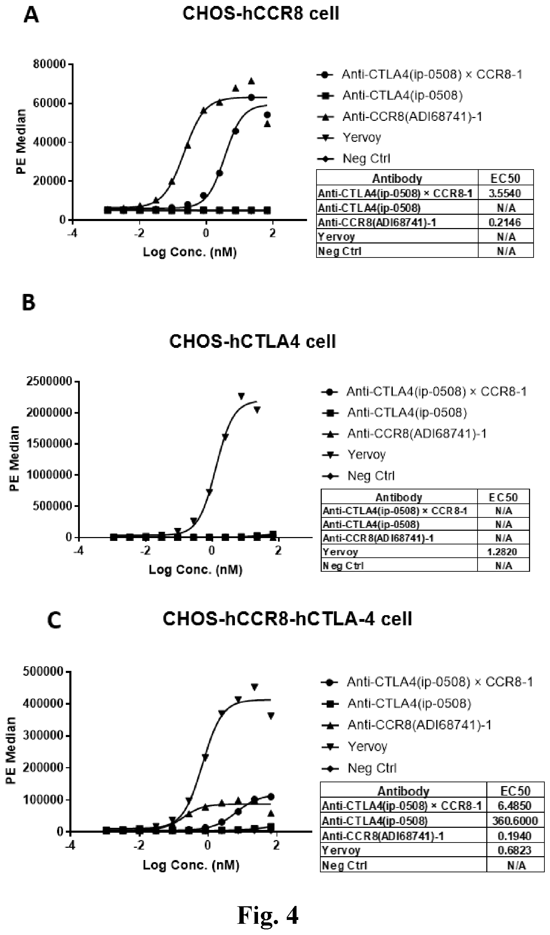

The experimental results are shown in Figs. 2-4. The CTLA4xCCR8 bispecific antibodies of the present disclosure could preferentially bind to CTLA4/CCR8 double-positive cells, and their binding to CTLA4 or CCR8 single-positive cells was weaker, significantly weaker than the corresponding CTLA4 or CCR8 monoclonal antibodies. In addition, compared to Yervoy (a monoclonal antibody targeting CTLA4; purchased from Bristol-Myers Squibb, Lot No.: 1336379A5 ABV5408), the affinity-optimized monoclonal antibody molecules and single domain molecules all showed varying degrees of reduced binding to CTLA4.

### Example 3. ADCC Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells at 3×10⁴ cells/well, respectively mixed with NFAT Luciferase/Jurkat CD16a (overexpressing CD16a and NFAT-Luc) effector cells at 1.2×10⁵ cells/well, were seeded into 96-well cell culture white bottom plates. Subsequently, the multispecific antibodies after gradient dilution were added to the 96-well plate and mixed, and incubated in a cell culture incubator for 6 hours. Chemiluminescence signals were collected by a microplate reader after color development using the Bio-Glo luciferase assay system (Promega, G7940) kit.

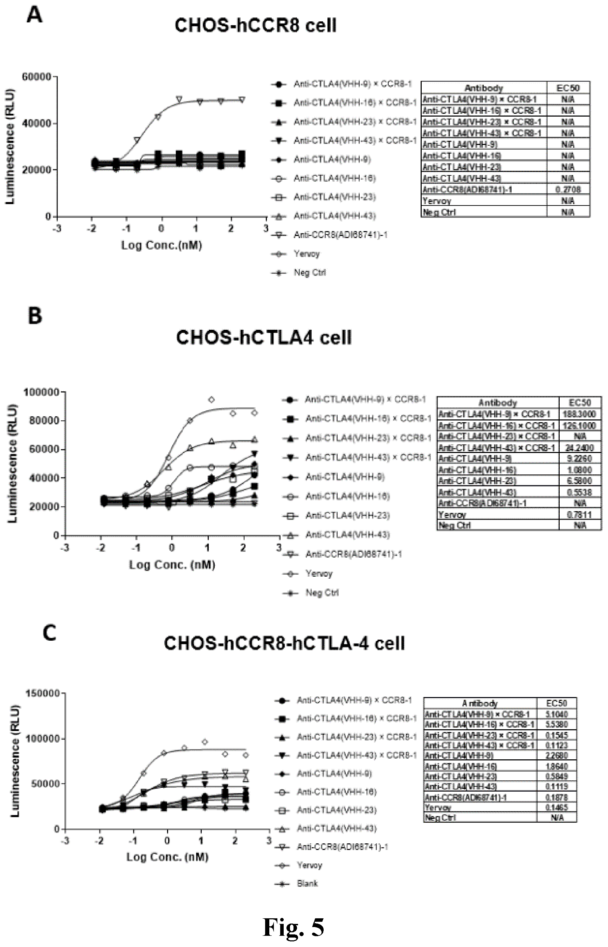

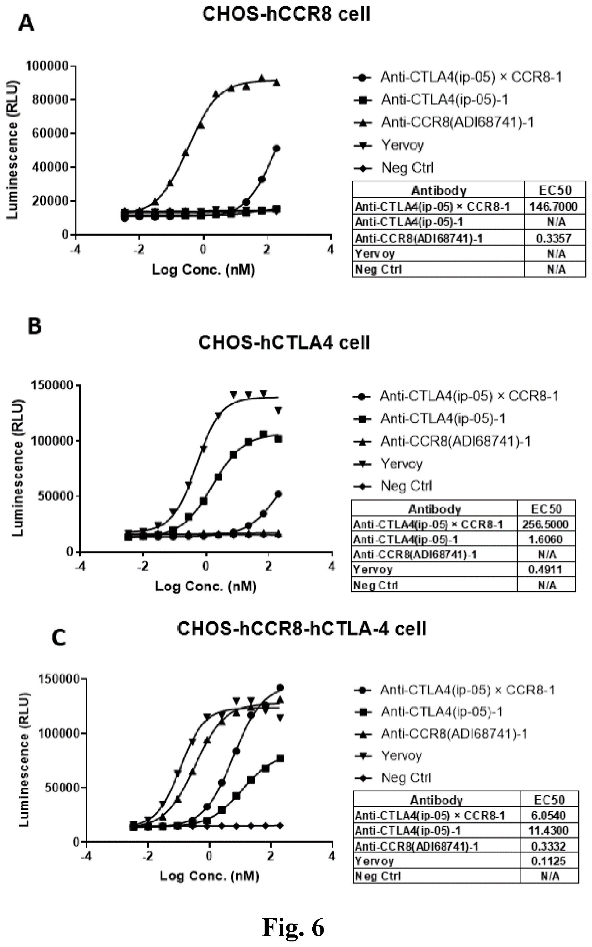

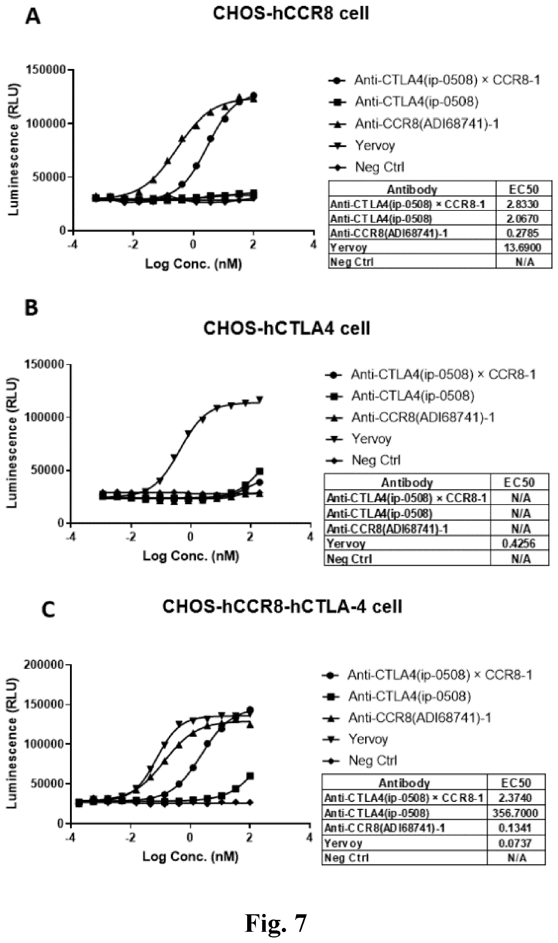

The experimental results are shown in Figs. 5-7. The CTLA4xCCR8 bispecific antibodies of the present disclosure could preferentially bind to CTLA4/CCR8 double-positive cells and mediate ADCC effects, thereby activating the CD16a-NFAT signaling pathway in Jurkat cells. However, due to the weak binding of the CTLA4xCCR8 bispecific antibodies to CTLA4 or CCR8 single-positive cells, the ADCC effect on single-positive cells was not apparent. In addition, compared to Yervoy, the affinity-optimized monoclonal antibody molecules and single domain molecules also showed varying degrees of reduced ADCC function against CTLA4-positive cells.

### Example 4. ADCC Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were stained using the CellTrace^{™} Violet kit and seeded into a 96-well clear-bottom black-walled cell culture plate at 1×10⁴ cells/well. PBMCs resuscitated one day in advance were collected and added to the target cell wells at 1×10⁵ cells/well. Subsequently, the bispecific antibodies after gradient dilution were added to the cell wells and co-incubated for 48 hours. After 48 hours, DAPI fluorescence signals were collected using a Cytation 5, and the respective killing intensity was calculated.

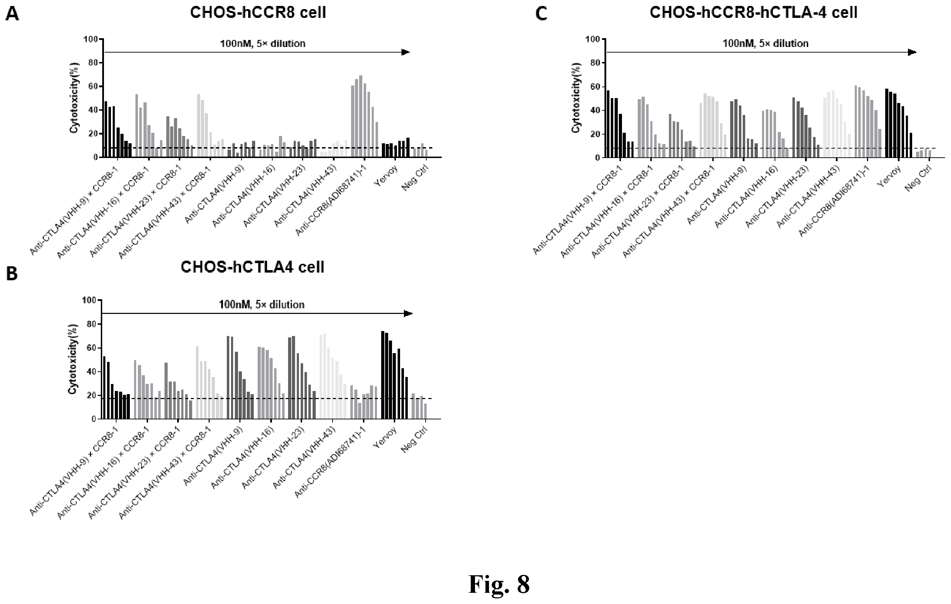

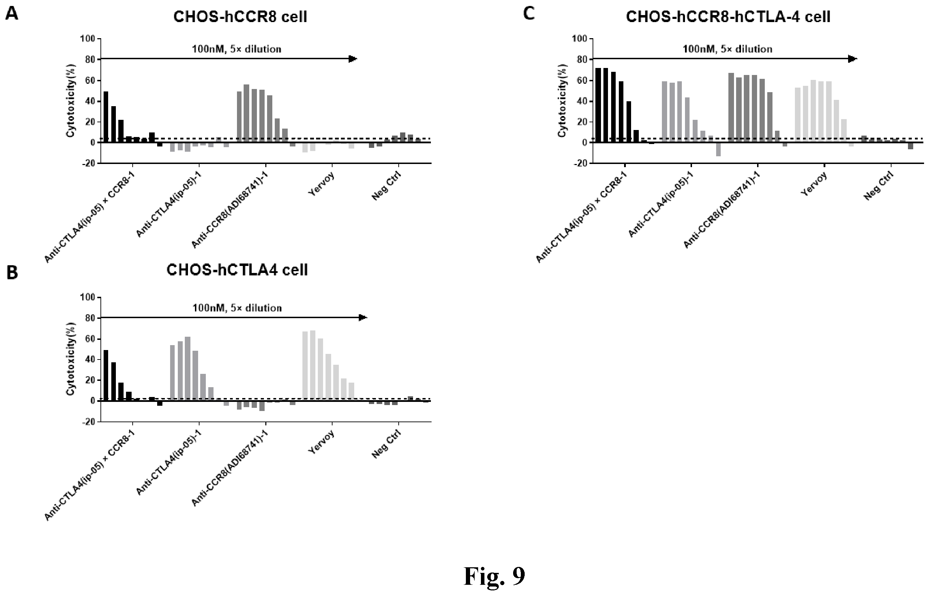

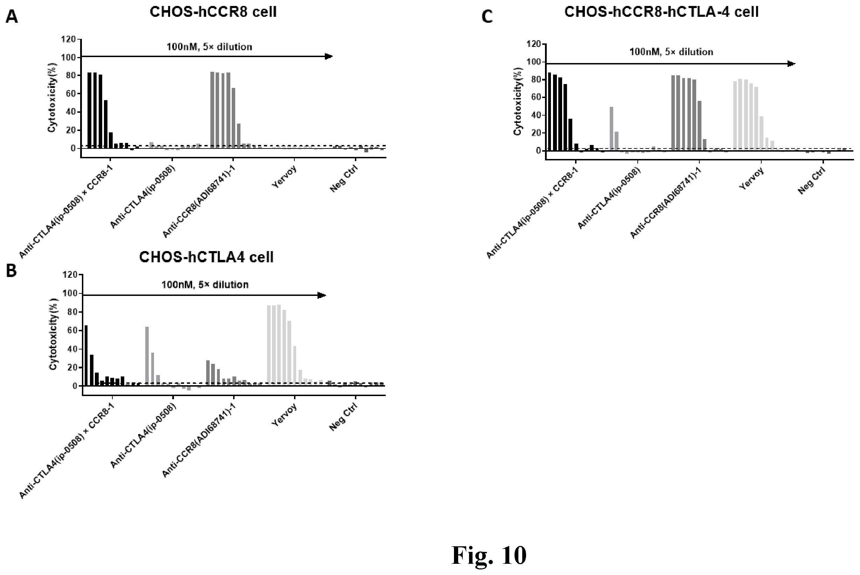

The experimental results are shown in Figs. 8-10. The CTLA4xCCR8 bispecific antibodies of the present disclosure could preferentially bind to CTLA4/CCR8 double-positive cells and induce elimination of target cells by PBMCs, with a killing effect comparable to that of the corresponding monoclonal antibodies. Furthermore, due to the weak binding of the CTLA4xCCR8 bispecific antibodies to CTLA4 or CCR8 single-positive cells, the mediated elimination effect on single-positive cells by PBMCs was significantly weaker than that of the corresponding CTLA4 or CCR8 monoclonal antibodies. In addition, compared to Yervoy, the affinity-optimized monoclonal antibody molecules and single domain molecules also showed varying degrees of reduced ADCC function against CTLA4-positive cells.

### Example 5. Internalization Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were adjusted to a cell density of 4×10⁶ cells/ml with culture medium, and 50 µl/well was added to a 96-well flow cytometry plate. The bispecific antibodies after gradient dilution (the antibodies were first incubated with a pH sensitive dye-labeled secondary antibody for 1 hour) were added to the cell wells at 50 µl/well, and placed in an incubator for 2 hours. After washing twice with PBS, the cells were finally resuspended by adding PBS at 100 µl/well, and detected on a CytoFlex (Beckman) flow cytometer, and the respective MFI was calculated.

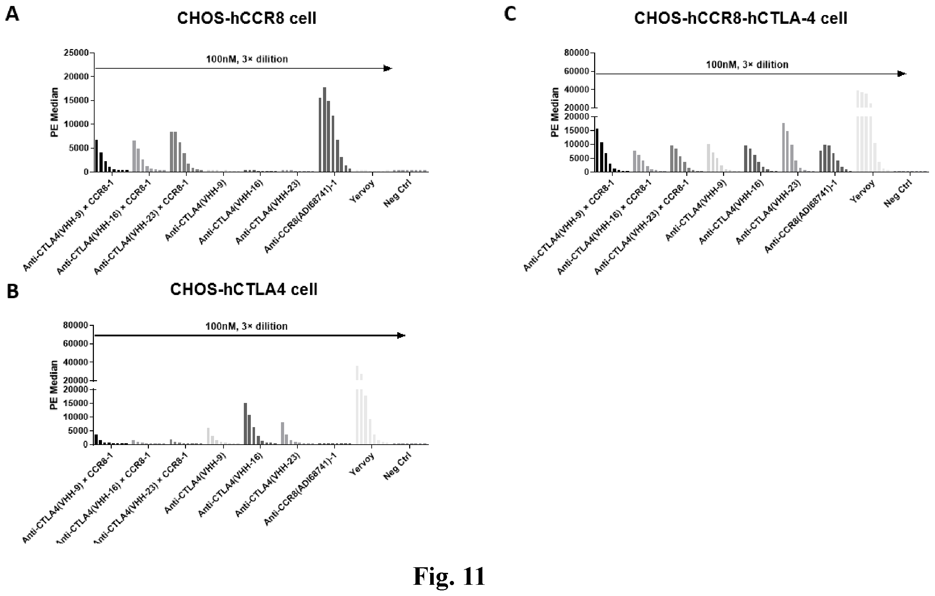

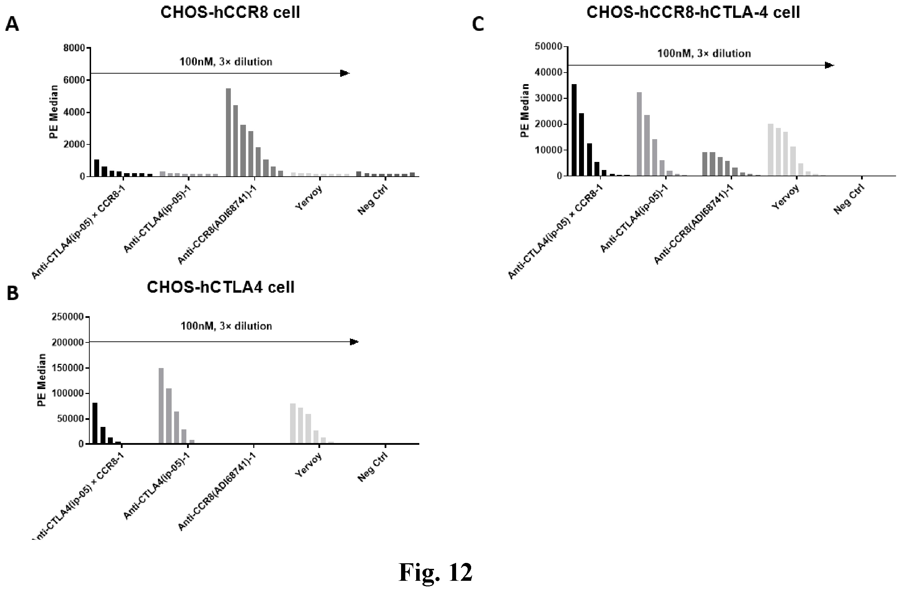

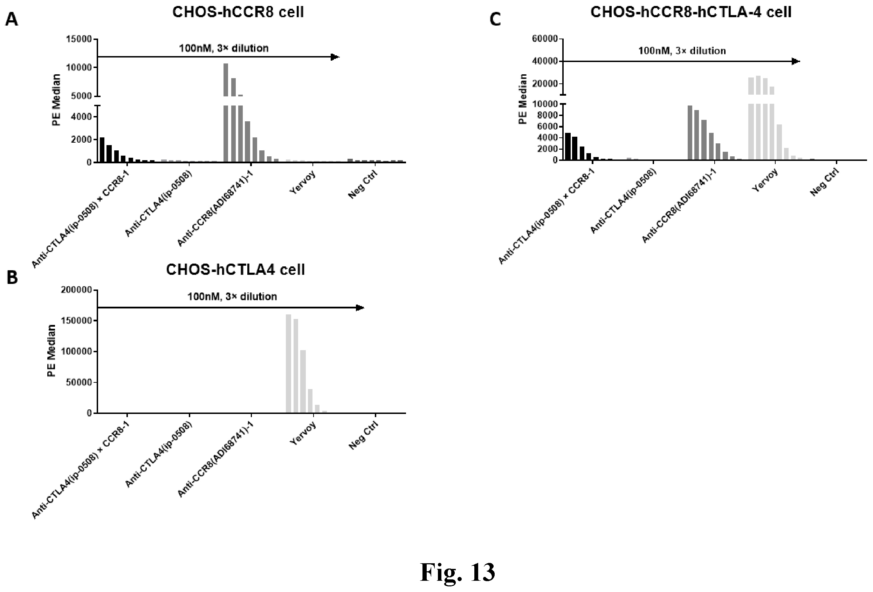

The experimental results are shown in Figs. 11-13. The CTLA4xCCR8 bispecific antibodies of the present disclosure could preferentially bind to CTLA4/CCR8 double-positive cells and mediate antibody internalization. Furthermore, due to the weak binding of the CTLA4xCCR8 bispecific antibodies to CTLA4 or CCR8 single-positive cells, the internalization effect in single-positive cells was significantly weaker than that of the corresponding CTLA4 or CCR8 monoclonal antibodies. In addition, compared to Yervoy, the affinity-optimized monoclonal antibody molecules and single domain molecules also showed varying degrees of reduced internalization function on CTLA4-positive cells.

### Example 6. Blocking Effect of CTLA4xCCR8 Bispecific Antibodies on CTLA4 Binding to CD80/CD86

In this experiment, expanded cultured CHOS-hCTLA4 (overexpressing human CTLA4) cells were adjusted to a cell density of 2×10⁶ cells/ml with 2% BSA solution, and 100 µl/well was added to a 96-well flow cytometry plate, and centrifuged for later use. The antibodies after gradient dilution were added to the 96-well flow cytometry plate containing the above cells at 100 µl/well, and incubated at 4°C for 60 min. After washing twice with PBS, human CD80/CD86 protein diluted with 2% BSA solution was added at 100 µl/well, and incubated at 4°C for 60 min. After washing twice with PBS, the cells were finally resuspended by adding PBS at 100 µl/well, and detected on a CytoFlex (Beckman) flow cytometer, and the respective MFI was calculated.

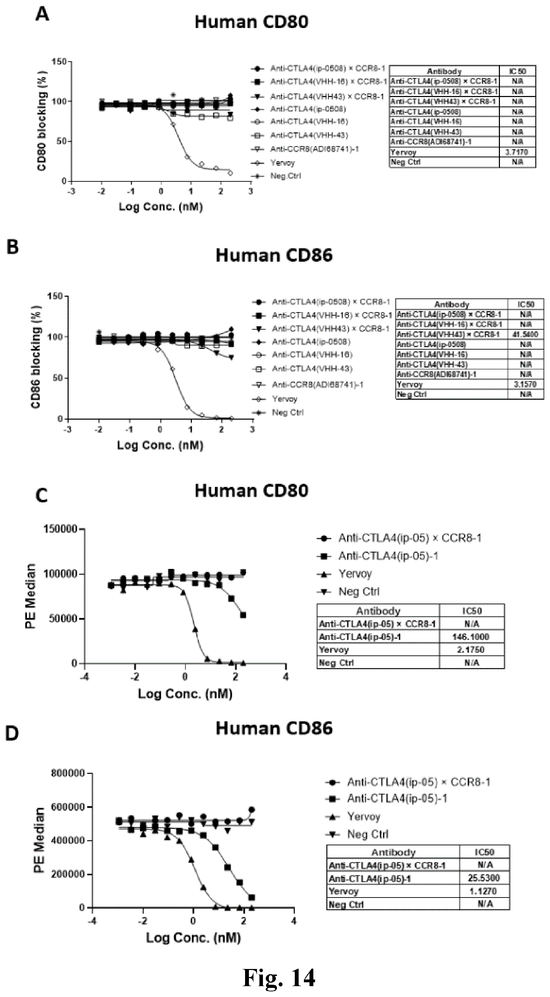

The experimental results are shown in Fig. 14. The CTLA4xCCR8 bispecific antibodies of the present disclosure could not block the binding of CTLA4 to human CD80/CD86 proteins. In addition, regarding CTLA4 monoclonal antibodies, Anti-CTLA4(ip-05)-1 and Anti-CTLA4(VHH-43) could block the binding of CTLA4 to human CD80/CD86 proteins, but the blocking activity was significantly weaker than that of the positive control antibody Yervoy.

### Example 7. PTM Analysis of CTLA4xCCR8 Bispecific Antibodies after High-Temperature Treatment

200 µg each of the high-temperature accelerated samples of CTLA4xCCR8 bispecific antibodies were taken in a 1.5 ml centrifuge tube, followed by the addition of 100 µl of protein denaturation solution (8 M guanidine hydrochloride, pH 6.0) and 2 µl of 1 M DTT, and a water bath at 37°C for 30 min for protein denaturation and reduction. After the water bath, the tube was spun for 30 sec, 4.4 µL of 1 mol/L IAM solution was added, vortexed for 30 sec to mix, spun for 30 sec, and then placed in the dark for 30 min. The sample after dark incubation was aspirated to a 10 kDa ultrafiltration tube, and 300 µl of 20 mmol/L His-HCl pH 6.0 digestion buffer was added. The tube was centrifuged at 13000 rpm/min for 15 min for desalting, then reverse spun at 3000g for 3 min, and the sample was recovered, and the protein concentration was determined. 40 µg of the desalted protein was taken, added with 2 µl of 0.5 mg/ml Trypsin/Lys-C mix enzyme solution (enzyme:protein ratio 1:40), and the digestion buffer was supplemented to a final volume of 40 µl. The mixture was gently vortexed to mix, spun briefly, taking care to avoid air bubbles, followed by a water bath at 37°C for 4 hours. After digestion, 2 µl of 20% formic acid aqueous solution was added to terminate the reaction. The mixture was mixed and centrifuged at 13000 rpm/min for 5 min, and the supernatant was taken for RP-UHPLC-MS analysis.

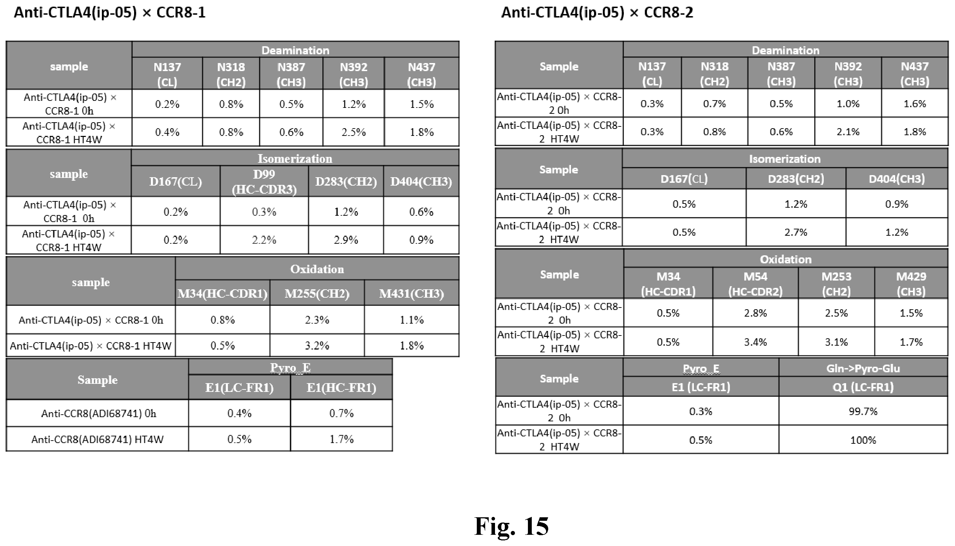

The experimental results are shown in Fig. 15. The isomerization of the D99 site in the HC-CDR3 of the Anti-CTLA4(ip-05)xCCR8-1 bispecific antibody of the present disclosure showed a certain degree of increase after high-temperature accelerated treatment. In addition, compared to the Anti-CTLA4(ip-05)xCCR8-1 antibody, the modified Anti-CTLA4(ip-05)xCCR8-2 bispecific antibody showed no detectable isomerization product at the D99 site, indicating better developability.

### Example 8. Hydrophilic and Hydrophobic Characteristic Analysis of CTLA4xCCR8 Bispecific Antibodies

In this study, sample HIC analysis was performed using a Sepax Proteomix HIC Butyl chromatography column. By comparing the retention times of the test sample and the control sample, the ΔRT value was obtained so as to analyze the hydrophilic and hydrophobic characteristics of the samples.

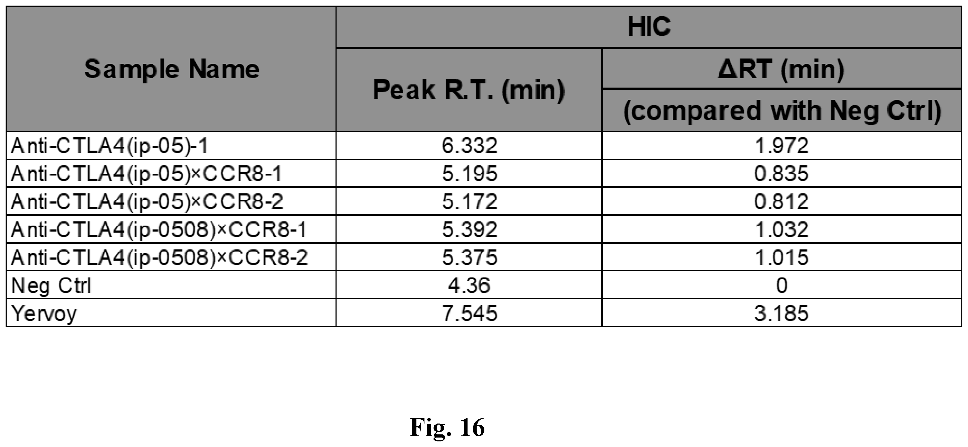

The experimental results are shown in Fig. 16. Compared to Yervoy, the hydrophobicity of the Anti-CTLA4(ip-05)-1 monoclonal antibody of the present disclosure was significantly improved. In addition, the CTLA4xCCR8 bispecific antibodies composed of Anti-CTLA4(ip-05)-1 or Anti-CTLA4(ip-0508), respectively, showed further optimized hydrophobicity compared to the monoclonal antibodies, and were significantly better than the Yervoy antibody. In summary, the CTLA4xCCR8 bispecific antibodies of the present application have better hydrophilicity compared to the monoclonal antibodies and the positive control antibody.

### Example 9. Binding of CTLA4xCCR8 Bispecific Antibodies to CTLA4/CCR8 Cells

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were adjusted to a cell density of 2×10⁶ cells/ml with 2% BSA solution, and 100 µl/well was added to a 96-well flow cytometry plate, and centrifuged for later use. The antibodies after gradient dilution were added to the 96-well flow cytometry plate containing the above cells at 100 µl/well, and incubated at 4°C for 60 min. After washing twice with PBS, 100 µl/well of Goat anti-human IgG-Fc (PE) (Abcam, ab98596) diluted 1000-fold with 2% BSA solution was added, and incubated at 4°C for 60 min. After washing twice with PBS, the cells were finally resuspended in 100 µl/well of PBS and detected on a CytoFlex (Beckman) flow cytometer, and the respective MFI was calculated.

Among them, the antibodies with "Afu" (i.e., Afucosylated hIgG) in the name (i.e., antibody without fucose modification) were obtained by transient expression and purification in a CHO cell expression system with the type VIII fucosyltransferase *fut-8* gene knockout. The specific structure of the antibodies is the same as described in Example 1. The specific preparation process is as follows: The pcDNA3.1 vector carrying the antibody heavy and light chains was transferred into *fut-8* gene knockout CHO cells by chemical transfection method, and the cells were cultured at 37°C, 8% CO₂ for 7 days. The cell culture fluid was collected and centrifuged at 13000 rpm for 20 minutes. The supernatant was taken, purified by Protein A and subjected to SEC for the antibody purity detection, while controlling the endotoxin content.

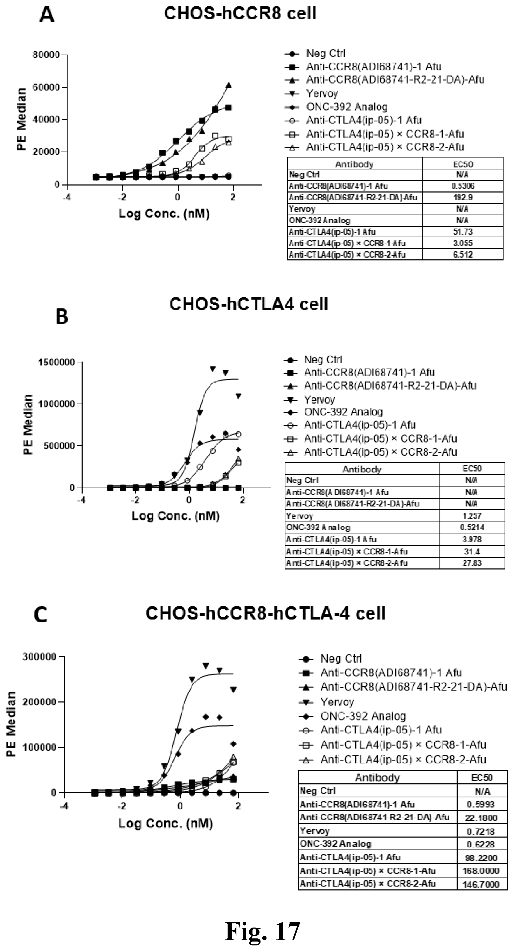

The experimental results are shown in Fig. 17. The cell binding activity of the Anti-CTLA4(ip-05)xCCR8-2-Afu bispecific antibody of the present disclosure was comparable to that of Anti-CTLA4(ip-05)xCCR8-1-Afu. They was able to preferentially bind to CCR8/CTLA4 double-positive cells, and its binding to CCR8 or CTLA4 single-positive cells was weaker, significantly weaker than that of the corresponding Anti-CCR8 or Anti-CTLA4 monoclonal antibodies.

### Example 10. ADCC Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells at 3×10⁴ cells/well, respectively mixed with NFAT Luciferase/Jurkat CD16a (overexpressing CD16a and NFAT-Luc) effector cells at 1.2×10⁵ cells/well, were seeded into a 96-well cell culture white bottom plate. Subsequently, the multispecific antibodies after gradient dilution were added to the 96-well plate and mixed, and incubated in a cell culture incubator for 6 hours. Chemiluminescence signals were collected by a microplate reader after color development using the Bio-Glo luciferase assay system (Promega, G7940) kit.

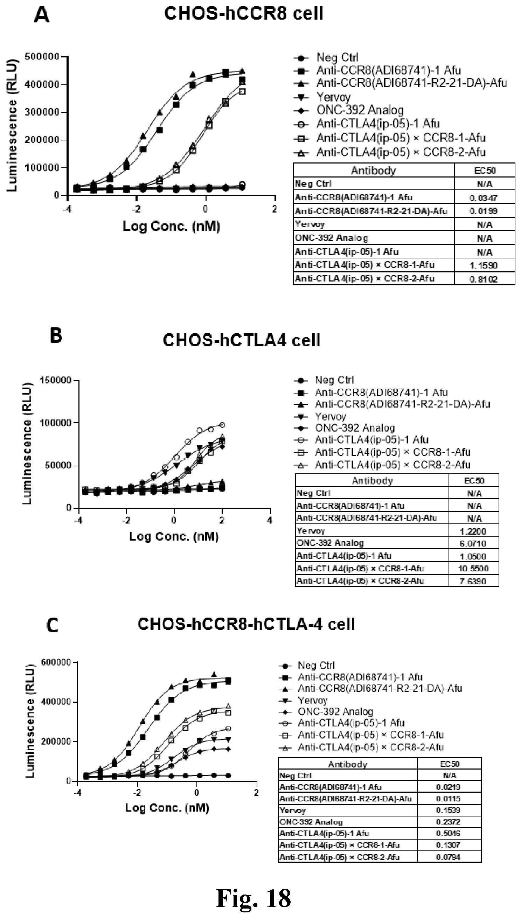

The experimental results are shown in Fig. 18. The ADCC activity of the Anti-CTLA4(ip-05)xCCR8-2-Afu bispecific antibody of the present disclosure was comparable to that of Anti-CTLA4(ip-05)xCCR8-1-Afu. That is, they could preferentially bind to CCR8/CTLA4 double-positive cells and mediate ADCC effects, thereby activating the CD16a-NFAT signaling pathway in Jurkat cells. However, due to the weak binding of the Anti-CTLA4(ip-05)xCCR8-1-Afu and Anti-CTLA4(ip-05)xCCR8-2-Afu bispecific antibodies to CCR8 or CTLA-4 single-positive cells, their ADCC effects on single-positive cells was significantly weaker than that of the corresponding monoclonal antibodies. In addition, since the Anti-CTLA4(ip-05)xCCR8-1-Afu and Anti-CTLA4(ip-05)xCCR8-2-Afu bispecific antibodies were subjected to fucose treatment to enhance ADCC function, their ADCC effects on CCR8/CTLA4 double-positive cells was stronger than Yervoy or ONC-392 Analog (a monoclonal antibody targeting CTLA4, Patent Publication No.: WO2024059833A1; the heavy chain of ONC-392 Analog is set forth in SEQ ID NO: 160, the light chain of ONC-392 Analog is set forth in SEQ ID NO: 161). The ADCC effect induced on CLTA4 single-positive cells was slightly weaker than that of Yervoy, comparable to that of ONC-392 Analog.

### Example 11. ADCC Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were stained using the CellTrace^{™} Violet kit and seeded into a 96-well clear-bottom black-walled cell culture plate at 1×10⁴ cells/well. PBMCs resuscitated one day in advance were collected and added to the target cell wells at 1×10⁵ cells/well. Subsequently, the bispecific antibodies after gradient dilution were added to the cell wells and co-incubated for 48 hours. After 48 hours, DAPI fluorescence signals were collected using a Cytation 5, and the respective killing intensity was calculated.

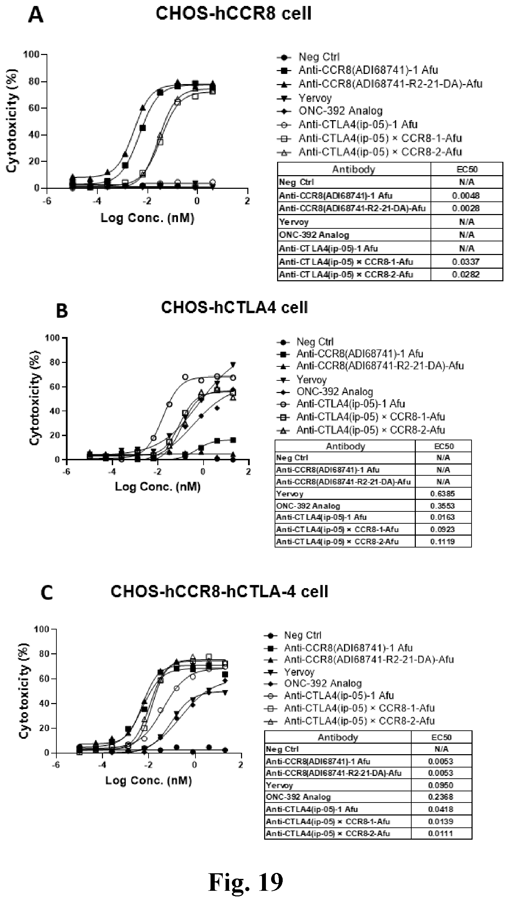

The experimental results are shown in Fig. 19. The ADCC activity induced by the Anti-CTLA4(ip-05)xCCR8-2-Afu bispecific antibody of the present disclosure was comparable to that of Anti-CTLA4(ip-05)xCCR8-1-Afu. That is, they could preferentially bind to CCR8/CTLA-4 double-positive cells and induce elimination of target cells by PBMCs, with a killing effect comparable to that of the corresponding monoclonal antibodies, and superior to that of Yervoy or ONC-392 Analog. In addition, due to the weak binding of the Anti-CTLA4(ip-05)xCCR8-1-Afu and Anti-CTLA4(ip-05)xCCR8-2-Afu bispecific antibodies to CCR8 or CTLA4 single-positive cells, the mediated elimination effect on single-positive cells by PBMCs was significantly weaker than that of the corresponding CCR8 or CTLA4 monoclonal antibodies, comparable to Yervoy or ONC-392 Analog.

### Example 12. Study on Tumor Suppression Activity of CTLA4xCCR8 Bispecific Antibodies

In this experiment, the anti-tumor activity of the CTLA4xCCR8 bispecific antibodies of the present disclosure was validated in a tumor model of h-CTLA4/CCR8 double KI BALB/c mice with CT26 subcutaneously inoculated.

First, a tumor-bearing mouse model was established by subcutaneous inoculation of CT26 cells. The mice were grouped once the tumor volume reached approximately 200 mm³, and were treated by intraperitoneal injection with G1: PBS, G2: 3mg/kg of Yervoy, G3: 3mg/kg of Anti-CCR8(ADI68741)-1-Afu, G4: 2.2mg/kg of Anti-CTLA4(VHH-43) x CCR8-1-Afu, and G5: 3mg/kg of Anti-CTLA4(ip-05) x CCR8-1-Afu, respectively (all groups received equal molar doses). The tumor volume and body weight changes of mice in each group were monitored 2-3 times per week for 2-3 weeks. The administration doses and methods are shown in Table 2.

Among them, the antibodies with "Afu" (i.e., Afucosylated hIgG) in the above names were obtained by transient expression and purification in a CHO cell expression system with *fut-8* gene knockout. The specific structure of the antibodies is the same as described in Example 1. The specific preparation process is as follows: The pcDNA3.1 vector carrying the antibody heavy and light chains was transferred into *fut-8* gene knockout CHO cells by chemical transfection method, and the cells were cultured at 37°C, 8% CO₂ for 7 days. The cell culture fluid was collected and centrifuged at 13000 rpm for 20 minutes. The supernatant was taken, purified by Protein A and subjected to SEC for the antibody purity detection, while controlling the endotoxin content.

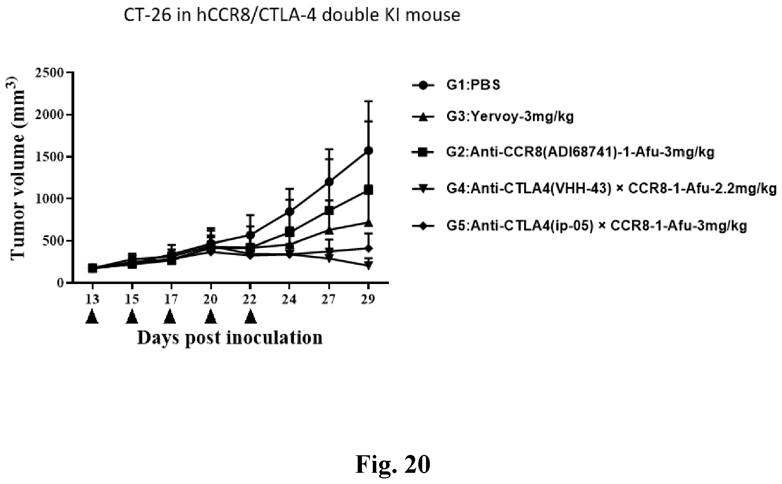

The experimental results are shown in Fig. 20. The anti-tumor activity of the CTLA4xCCR8 bispecific antibodies Anti-CTLA4(VHH-43)xCCR8-1-Afu (TGI: 87.04%) and Anti-CTLA4(ip-05)xCCR8-1-Afu (TGI: 73.73%) was significantly superior to that of Yervoy (TGI: 54.44%) or Anti-CCR8(ADI68741)-1-Afu (TGI: 30.02%).

**Table 2: Dosing Regimen for the Study on Tumor Suppression Activity of CTLA4xCCR8 Bispecific Antibodies**

| Group | Dose administered | Frequency of Administratio |
|---|---|---|
| | n | |
| G1: Negative control (PBS) | N/A | Q2d ×5 |
| G2: Yervoy | 2.5 mg/kg | Q2d ×5 |
| G3: Anti-CCR8(ADI68741)-1-Afu | 3 mg/kg | Q2d ×5 |
| G4: Anti-CTLA4(VHH-43) x CCR8-1-Afu | 2.2 mg/kg | Q2d ×5 |
| G5: Anti-CTLA4(ip-05) x CCR8-1-Afu | 3 mg/kg | Q2d ×5 |

### Example 13. Study on Tumor Suppression Activity of CTLA4xCCR8 Bispecific Antibodies

In this experiment, the anti-tumor activity of the Anti-CTLA4×CCR8 bispecific antibodies of the present disclosure was validated in a tumor model of h-CTLA4/CCR8 double KI BALB/c mice with CT26 subcutaneously inoculated. First, a tumor-bearing mouse model was established by subcutaneous inoculation of CT26 cells. The mice were grouped once the tumor volume reached approximately 150 mm³, and were treated by intraperitoneal injection with G1: PBS, G2: 3 mg/kg of Anti-CCR8 (ADI68741)-2, G3: 3 mg/kg of ONC-392 Analog, G4: 3 mg/kg of Anti-CTLA4(ip-05)-2, G5: 3 mg/kg of Anti-CTLA4 (ip-05)xCCR8-7, G6: 3 mg/kg of Anti-CTLA4 (ip-0508)xCCR8-7, and G7: 3 mg/kg of Anti-CTLA4 (ip-05)xCCR8-8, respectively (all groups received equal molar doses). The tumor volume and body weight changes of mice in each group were monitored 2-3 times per week for 2-3 weeks. The administration doses and methods are shown in Table 3.

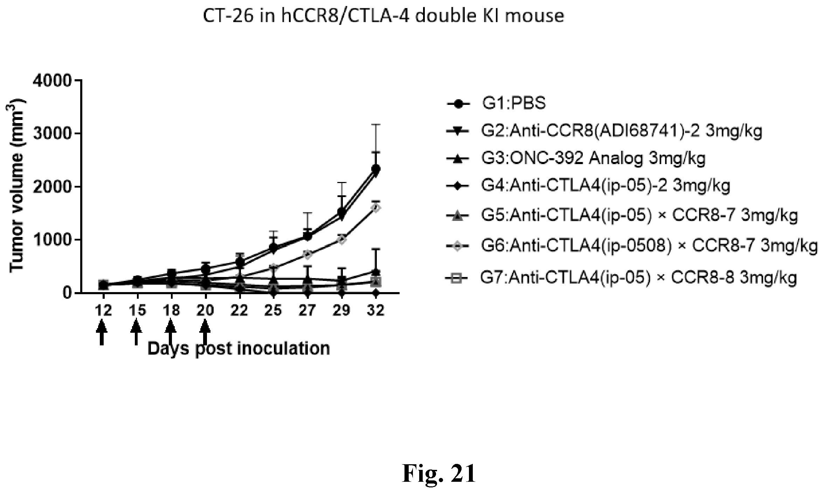

In order to better verify the importance of enhancing Fc function in this experiment, the Fc of the Anti-CTLA4xCCR8 bispecific antibody was changed to murine IgG2a, which may better simulate the Fc activity of defucosylated human IgG1 in the human body. The experimental results are shown in Fig. 21. The Anti-CTLA4(ip-05)xCCR8-7 (TGI: 89.54%) and Anti-CTLA4(ip-05)xCCR8-8 (TGI: 90.16%) bispecific antibodies both had very strong anti-tumor activity, superior to the ONC-392 Analog (TGI: 80.33%) molecule.

**Table 3: Dosing Regimen for the Study on Tumor Suppression Activity of CTLA4xCCR8 Bispecific Antibodies**

| Group | Dose administered | Frequency of Administration |
|---|---|---|
| G1: Negative control (PBS) | N/A | Q2d ×4 |
| G2: Anti-CCR8(ADI68741)-2 | 3 mg/kg | Q2d ×4 |
| G3: ONC-392 Analog | 3 mg/kg | Q2d ×4 |
| G4: Anti-CTLA4(ip-05)-2 | 3 mg/kg | Q2d ×4 |
| G5: Anti-CTLA4(ip-05)×CCR8-7 | 3 mg/kg | Q2d ×4 |
| G6: Anti-CTLA4(ip-0508)×CCR8-7 | 3 mg/kg | Q2d ×4 |
| G7: Anti-CTLA4(ip-05)×CCR8-8 | 3 mg/kg | Q2d ×4 |

### Example 14. Safety Study of CTLA4xCCR8 Bispecific Antibodies

In this experiment, the safety of the Anti-CTLA4×CCR8 bispecific antibodies of the present disclosure was validated using a juvenile h-CTLA4/CCR8 double KI BALB/c mouse model. The mice were treated by intraperitoneal injection with G1: 50 µg/mouse Anti-PD-1, G2: 50 µg/mouse Anti-PD-1 + 100 µg/mouse Yervoy, G3: 50 µg/mouse Anti-PD-1 + 100 µg/mouse ONC-392 Analog, G4: 50 µg/mouse Anti-PD-1 + 100 µg/mouse Anti-CTLA-4 (Ipi-05)-2, G5: 50 µg/mouse Anti-PD-1 + 100 µg/mouse Anti-CTLA4(ip-0508)xCCR8-7, G6: 50 µg/mouse Anti-PD-1 + 100 µg/mouse Anti-CTLA4(ip-05)xCCR8-7, respectively. The body weight changes of mice in each group were monitored 2-3 times per week for 2-3 weeks. The administration doses and methods are shown in Table 4.

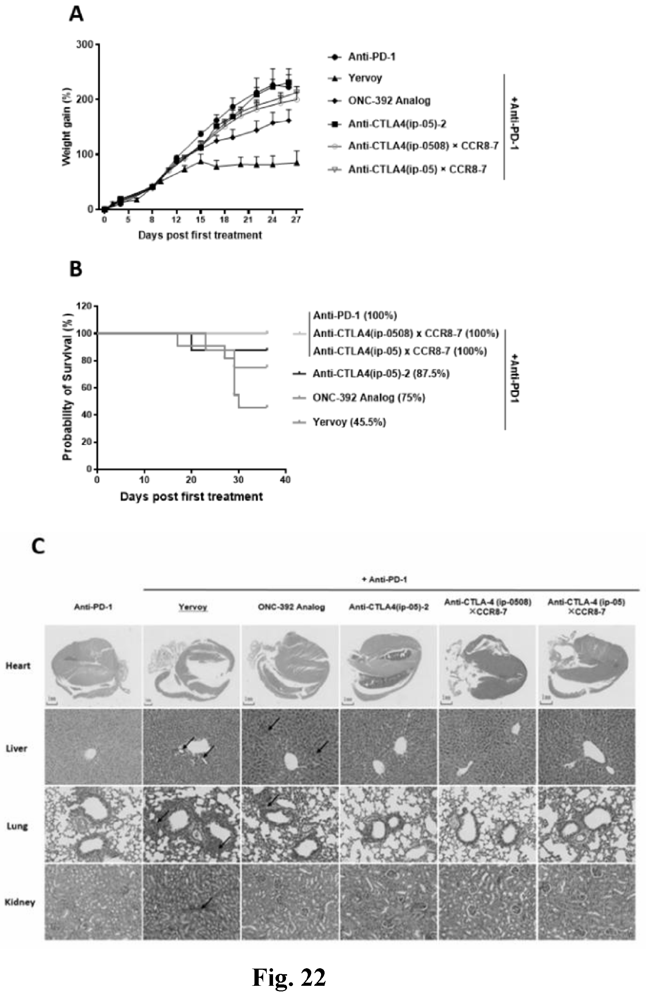

The experimental results are shown in Fig. 22. Under conditions of combination with the Anti-PD-1 antibody, the Anti-CTLA4(ip-05)xCCR8-7 and Anti-CTLA4(ip-0508)xCCR8-7 bispecific antibodies exhibited good safety profiles, with no significant change in mouse body weight compared to the control group, no obvious tissue damage, and no mouse death observed until the end of the experiment. In contrast, the combination of Yervoy and Anti-PD-1 showed significant toxicity, with mice exhibiting obvious weight loss, death, and tissue damage. In addition, ONC-392 Analog, a monoclonal antibody in clinical studies that has shown potential for better safety and a larger therapeutic window than Yervoy, also showed some degree of mouse body weight loss and death, but overall safety was better than Yervoy.

**Table 4: Dosing Regimen for the Safety Study of CTLA4xCCR8 Bispecific Antibodies**

| Group | Dose administered | Frequency of Administration |
|---|---|---|
| G1:Anti-PD-1 | 50 µ g | Q2d ×4 |
| G2: Anti-PD-1+Yervoy | 50 µ g +100 ug | Q2d ×4 |
| G3: Anti-PD-1+ONC-392 Analog | 50 µ g +100 ug | Q2d ×4 |
| G4: Anti-PD-1+ Anti-CTLA4(ip-05)-2 | 50 µ g +100 ug | Q2d ×4 |
| G5: Anti-PD-1+Anti-CTLA4(ip-05)×CCR8-7 | 50 µ g +100 ug | Q2d ×4 |
| G6: Anti-PD-1+Anti-CTLA4(ip-05)×CCR8-7 | 50 µ g +100 ug | Q2d ×4 |

### Example 15. Screening of Cell Lines for CTLA4xCCR8 Bispecific Antibodies

### Cell Line Construction

The vector pCHO2.0-GS-Puro-H1-L1 containing the heavy chain gene and the light chain gene of anti-CTLA4 antibody, and the vector pCHO2.0-GS-Puro-H2-L2 containing the heavy chain gene and the light chain gene of anti-CCR8 antibody were co-transferred into host cells CHOS-ADP using electroporation. The cells were screened by Puromycin and MSX selection pressure to obtain high-producing minipools. Then, through one round of limiting dilution and monoclonal identification, high-producing stable clonal cell lines were obtained.

### Cell Culture

The cells were cultured using Dynamis AGT Medium as the basal medium with a seeding density of (1.0 ± 0.2)×10⁶ cells/ml. On days 3, 5, 7, 9, and 11 of culture, fed-batch additions of 5.0 ± 0.5% (w/w) of the initial culture weight of feed 7a and 0.5 ± 0.05% (w/w) of the initial culture weight of 7b were performed, respectively. Dissolved oxygen was set at 40%. The initial culture temperature was 36.5°C, and it was reduced to 33.0°C on day 4. According to the daily glucose concentration measurements, 300 g/kg glucose concentrate was added to bring the glucose concentration in the culture solution to 6.0 g/L, except on the day of harvest. The culture was terminated on day 14 or when cell viability fell below 80%. During the culture process, cell density and viability were detected using a Vicell (Beckman). From day 7 onwards, antibody titer was measured daily using a Cedex (Roche).

### Product Quality Identification of Stable Cell Line

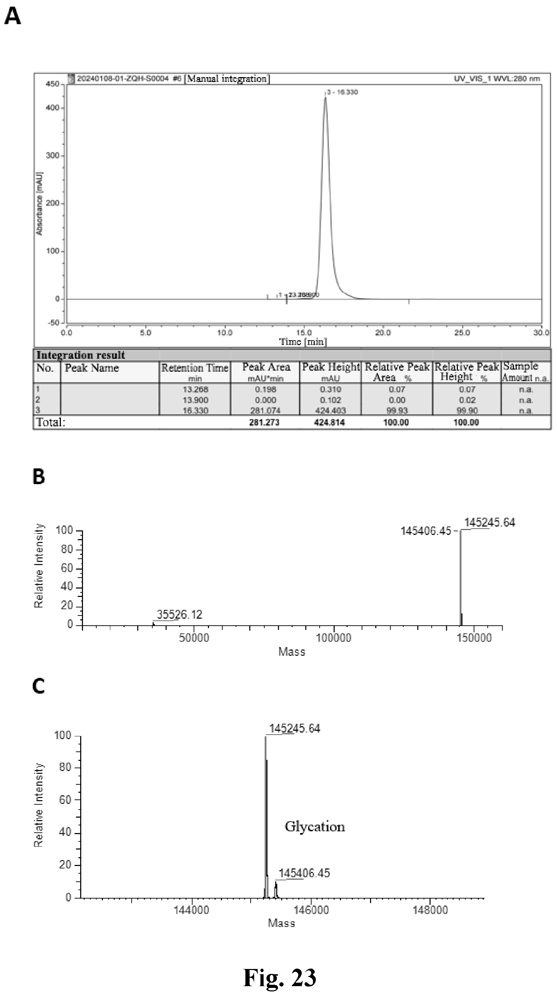

Antibodies were purified by one-step affinity chromatography, and the purity of the obtained protein was detected using HPLC. The HPLC method was as follows: mobile phase: 150 mM Na₂HPO₄•12H₂O, pH 7.0. Chromatographic conditions were: detection wavelength: 280 nm, column temperature: 25°C, flow rate: 0.5 ml/min, run time: 30 min, TSKgel G3000SWXL chromatography column. The SEC results, shown in A of Fig. 23, indicated that the bispecific antibody obtained by one-step affinity purification had a purity of 99.93%.

The heavy and light chain pairing of the obtained protein was analyzed by high-performance liquid chromatography-mass spectrometry using a Vanquish UHPLC (Thermo) liquid chromatography system, a Q Exactive (Thermo) mass spectrometer, and a Waters ACQUITY UPLC BEH C4 (2.1 mm × 100 mm) column. 50 µg of the sample was taken, diluted with ultrapure water to 25 µl and centrifuged, and 20 µl of the sample was transferred to a sample vial; 5 µl was injected for intact mass analysis by LC-MS. The chromatographic conditions were: column temperature: 80°C; UV detection wavelength: 280 nm; flow rate: 0.3 mL/min; mobile phase A: aqueous solution (containing 0.1% formic acid); mobile phase B: acetonitrile solution (containing 0.1% formic acid). Mass spectrometry parameters were: ESI ion source: ion transfer tube temperature 320°C, voltage 3.8 kV, gas flow 36 L/min; mode: positive ion Full MS; resolution: 17,500; scan range: 600-4000 m/z. The results showed that all eight purified bispecific antibodies Anti-CTLA4(ip-05)×CCR8 were correctly paired products. Data for Anti-CTLA4(ip-05)×CCR8-4 as shown in B of Fig. 23 and C of Fig. 23.

### Example 16. Binding of CTLA4xCCR8 Bispecific Antibodies to CTLA4/CCR8 Cells

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were adjusted with 2% BSA solution to a cell density of 2×10⁶ cells/ml, and 100 µl/well was added to a 96-well flow cytometry plate, and centrifuged for later use. The antibodies after gradient dilution were added to the 96-well flow cytometry plate containing the above cells at 100 µl/well, and incubated at 4°C for 60 min. After washing twice with PBS, 100 µl/well of Goat anti-human IgG-Fc (PE) (Abcam, ab98596) diluted 1000-fold with 2% BSA solution was added, and incubated at 4°C for 60 min. After washing twice with PBS, the cells were finally resuspended in 100 µl/well of PBS and analyzed on a CytoFlex (Beckman) flow cytometer, and the respective MFI was calculated.

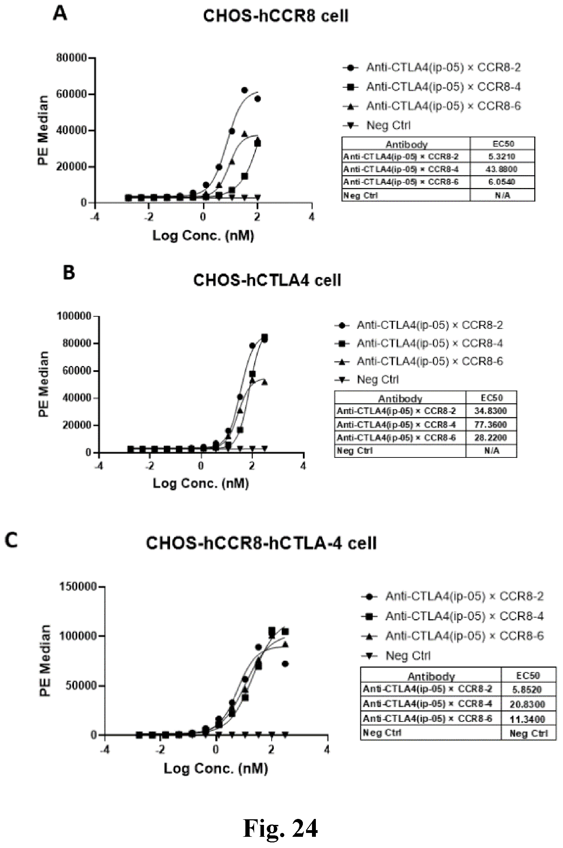

The experimental results are shown in Fig. 24. The binding activity of the Anti-CTLA4(ip-05)xCCR8-4 and Anti-CTLA4(ip-05)xCCR8-6 bispecific antibodies of the present disclosure to CCR8/CTLA4 double-positive cells was comparable to that of Anti-CTLA4(ip-05)xCCR8-2. In addition, their binding activity to CTLA4 or CCR8 single-positive cells was slightly weaker than that of the Anti-CTLA4(ip-05)xCCR8-2 bispecific antibody.

### Example 17. ADCC Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells at 3×10⁴ cells/well, respectively mixed with NFAT Luciferase/Jurkat CD16a (overexpressing CD16a and NFAT-Luc) effector cells at 1.2×10⁵ cells/well, were seeded into a 96-well cell culture white bottom plate. Subsequently, multispecific antibodies after gradient dilution were added to the 96-well plate and mixed, and incubated in a cell culture incubator for 6 hours. Chemiluminescence signals were collected by a microplate reader after color development using the Bio-Glo luciferase assay system (Promega, G7940) kit.

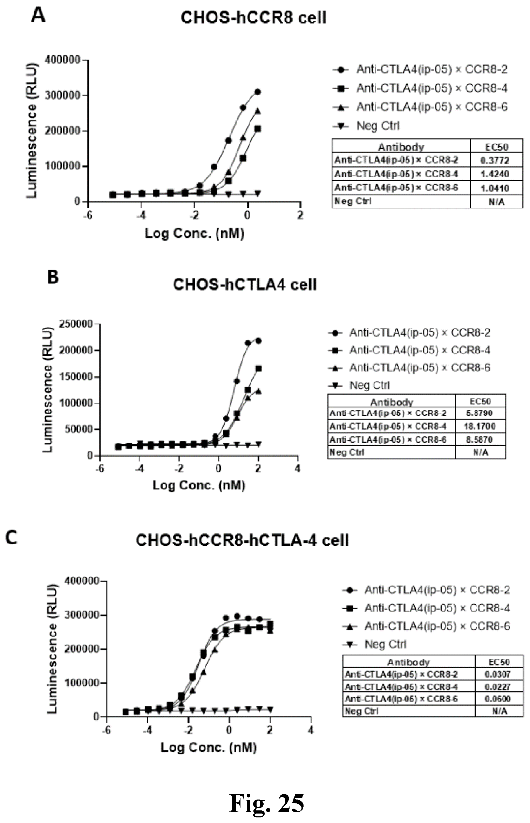

The experimental results are shown in Fig. 25. The activity of the Anti-CTLA4(ip-05)xCCR8-4 and Anti-CTLA4(ip-05)xCCR8-6 bispecific antibodies of the present disclosure in binding to CTLA4/CCR8 double-positive cells and mediating ADCC effects were comparable to that of Anti-CTLA4(ip-05)xCCR8-2. In addition, their ADCC activity mediated through binding to CTLA4 or CCR8 single-positive cells was slightly weaker than that of the Anti-CTLA4(ip-05)xCCR8-2 bispecific antibody.

### Example 18. ADCC Effect Induced by CTLA4xCCR8 Bispecific Antibodies

In this experiment, expanded cultured CHOS-hCTLA4 (overexpressing human CTLA4) cells, CHOS-hCCR8 (overexpressing human CCR8) cells, and CHOS-hCTLA4/hCCR8 (overexpressing human CTLA4 and human CCR8) double-positive cells were stained using the CellTrace^{™} Violet kit and seeded into a 96-well clear-bottom black-walled cell culture plate at 1×10⁴ cells/well. PBMCs resuscitated one day in advance were collected and added to the target cell wells at 1×10⁵ cells/well. Subsequently, the bispecific antibodies after gradient dilution were added to the cell wells and co-incubated for 48 hours. After 48 hours, DAPI fluorescence signals were collected using a Cytation 5, and the respective killing intensity was calculated.

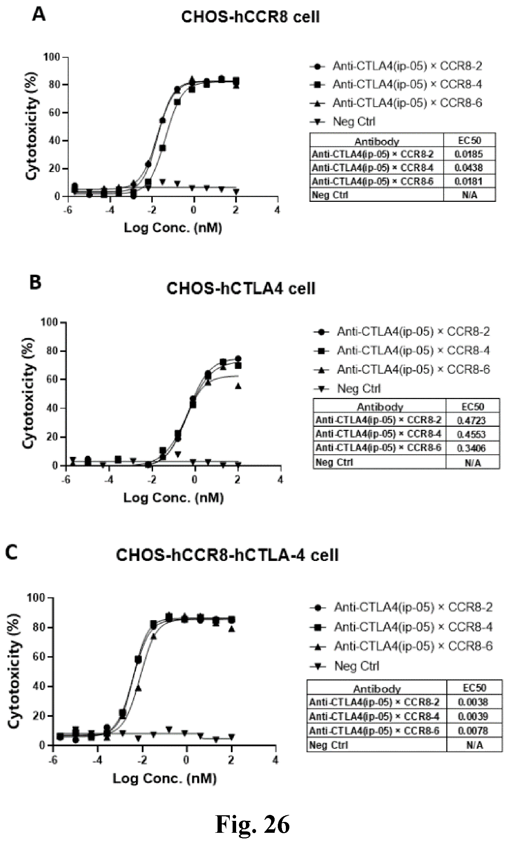

The experimental results are shown in Fig. 26. The activity of the Anti-CTLA4(ip-05)xCCR8-4 bispecific antibody of the present disclosure in binding to CTLA4/CCR8 double-positive cells and inducing elimination of target cells by PBMCs was comparable to that of Anti-CTLA4(ip-05)xCCR8-2, while the ADCC effect mediated by Anti-CTLA4(ip-05)xCCR8-6 was weaker than that of Anti-CTLA4(ip-05)xCCR8-2. Furthermore, regarding single-positive cells, the ADCC effect mediated by the Anti-CTLA4(ip-05)xCCR8-6 bispecific antibody was comparable to that of Anti-CTLA4(ip-05)xCCR8-2, while the PBMC killing effect induced by the Anti-CTLA4(ip-05)xCCR8-4 bispecific antibody through binding to CCR8 single-positive cells was slightly weaker than that of Anti-CTLA4(ip-05)xCCR8-2.

Although the specific embodiments of the present disclosure have been described in detail, those skilled in the art will appreciate that various modifications and changes may be made to the details based on all the teachings disclosed, and such changes are within the protection scope of the present disclosure. The entire scope of the present disclosure is given by the appended claims and any equivalents thereof.

## Claims

1. An antibody or antigen-binding fragment thereof capable of specifically binding to CTLA4, comprising:
(a) a heavy chain variable region (VH) comprising the following three complementarity determining regions (CDRs):
(i) a VH CDR1, consisting of the sequence set forth in SEQ ID NO: 48 or 54, or a sequence having one or several amino acid substitutions, deletions, or additions (e.g., 1, 2, or 3 amino acid substitutions, deletions, or additions) as compared to SEQ ID NO: 48 or 54,
(ii) a VH CDR2, consisting of the sequence set forth in SEQ ID NO: 49 or 55, or a sequence having one or several amino acid substitutions, deletions, or additions (e.g., 1, 2, or 3 amino acid substitutions, deletions, or additions) as compared to SEQ ID NO: 49 or 55, and
(iii) a VH CDR3, consisting of the sequence set forth in SEQ ID NO: 50 or 56, or a sequence having one or several amino acid substitutions, deletions, or additions (e.g., 1, 2, or 3 amino acid substitutions, deletions, or additions) as compared to SEQ ID NO: 50 or 56;
and/or,
(b) a light chain variable region (VL) comprising the following three complementarity determining regions (CDRs):
(iv) a VL CDR1, consisting of the sequence of SEQ ID NO: 51 or 57, or a sequence having one or several amino acid substitutions, deletions, or additions (e.g., 1, 2, or 3 amino acid substitutions, deletions, or additions) as compared to SEQ ID NO: 51 or 57,
(v) a VL CDR2, consisting of the sequence of SEQ ID NO: 52 or 58, or a sequence having one or several amino acid substitutions, deletions, or additions (e.g., 1, 2, or 3 amino acid substitutions, deletions, or additions) as compared to SEQ ID NO: 52 or 58, and
(vi) a VL CDR3, consisting of the sequence of SEQ ID NO: 53 or 59, or a sequence having one or several amino acid substitutions, deletions, or additions (e.g., 1, 2, or 3 amino acid substitutions, deletions, or additions) as compared to SEQ ID NO: 53 or 59;
preferably, the substitution in any one of (i)-(vi) is a conservative substitution;
preferably, the CDRs in any one of (i)-(vi) are defined according to the Kabat, IMGT, or Chothia numbering system;
preferably, the CDRs in any one of (i)-(vi) are defined according to the IMGT numbering system.

2. The antibody or antigen-binding fragment thereof of claim 1, comprising:
(1) the following three heavy chain CDRs: a VH CDR1 as set forth in SEQ ID NO: 48, a VH CDR2 as set forth in SEQ ID NO: 49, a VH CDR3 as set forth in SEQ ID NO: 50; and/or, the following three light chain CDRs: a VL CDR1 as set forth in SEQ ID NO: 51, a VL CDR2 as set forth in SEQ ID NO: 52, a VL CDR3 as set forth in SEQ ID NO: 53; or,
(2) the following three heavy chain CDRs: a VH CDR1 as set forth in SEQ ID NO: 54, a VH CDR2 as set forth in SEQ ID NO: 55, a VH CDR3 as set forth in SEQ ID NO: 56; and/or, the following three light chain CDRs: a VL CDR1 as set forth in SEQ ID NO: 57, a VL CDR2 as set forth in SEQ ID NO: 58, a VL CDR3 as set forth in SEQ ID NO: 59;
preferably, wherein the antibody or antigen-binding fragment thereof further comprises a framework region of a human immunoglobulin.

3. The antibody or antigen-binding fragment thereof of claim 1 or 2, wherein the antibody or antigen-binding fragment thereof comprises:
(a) a heavy chain variable region (VH) comprising an amino acid sequence selected from the group consisting of:
(i) the sequence set forth in SEQ ID NO: 3 or 5;
(ii) a sequence having one or several amino acid substitutions, deletions, or additions (e.g., 1, 2, 3, 4, or 5 amino acid substitutions, deletions, or additions) as compared to the sequence set forth in SEQ ID NO: 3 or 5; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 3 or 5;
and/or
(b) a light chain variable region (VL) comprising an amino acid sequence selected from the group consisting of:
(iv) the sequence set forth in SEQ ID NO: 4 or 6;
(v) a sequence having one or several amino acid substitutions, deletions, or additions (e.g., 1, 2, 3, 4, or 5 amino acid substitutions, deletions, or additions) as compared to the sequence set forth in SEQ ID NO: 4 or 6; or
(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 4 or 6;
preferably, the substitution in (ii) or (v) is a conservative substitution;
preferably, the antibody or antigen-binding fragment thereof comprises:
(1) a VH having the sequence set forth in SEQ ID NO: 3 and a VL having the sequence set forth in SEQ ID NO: 4; or,
(2) a VH having the sequence set forth in SEQ ID NO: 5 and a VL having the sequence set forth in SEQ ID NO: 6.

4. A single domain antibody or antigen-binding fragment thereof capable of specifically binding to CTLA4, comprising:
(a) a CDR1 having: the sequence set forth in any one of SEQ ID NOs: 60, 63, 66, or 69, or a sequence having one or several amino acid substitutions, deletions, or additions (e.g., 1, 2, or 3 amino acid substitutions, deletions, or additions) as compared to the sequence set forth in any one of SEQ ID NOs: 60, 63, 66, or 69;
(b) a CDR2 having: the sequence set forth in any one of SEQ ID NOs: 61, 64, 67, or 70, or a sequence having one or several amino acid substitutions, deletions, or additions (e.g., 1, 2, or 3 amino acid substitutions, deletions, or additions) as compared to the sequence set forth in any one of SEQ ID NOs: 61, 64, 67, or 70; and
(c) a CDR3 having: the sequence set forth in any one of SEQ ID NOs: 62, 65, 68, or 71, or a sequence having one or several amino acid substitutions, deletions, or additions (e.g., 1, 2, or 3 amino acid substitutions, deletions, or additions) as compared to the sequence set forth in any one of SEQ ID NOs: 62, 65, 68, or 71; preferably, the substitution is a conservative substitution.

5. The single domain antibody or antigen-binding fragment thereof of claim 4, comprising:
(1) a CDR1 as set forth in SEQ ID NO: 60; a CDR2 as set forth in SEQ ID NO: 61; and, a CDR3 as set forth in SEQ ID NO: 62;
(2) a CDR1 as set forth in SEQ ID NO: 63; a CDR2 as set forth in SEQ ID NO: 64; and, CDR3 as set forth in SEQ ID NO: 65;
(3) a CDR1 as set forth in SEQ ID NO: 66; a CDR2 as set forth in SEQ ID NO: 67; and, a CDR3 as set forth in SEQ ID NO: 68; or,
(4) a CDR1 as set forth in SEQ ID NO: 69; a CDR2 as set forth in SEQ ID NO: 70; and, a CDR3 as set forth in SEQ ID NO: 71.

6. The single domain antibody or antigen-binding fragment thereof of claim 4 or 5, comprising an amino acid sequence selected from the group consisting of:
(i) the sequence set forth in any one of SEQ ID NOs: 7, 8, 9, or 10;
(ii) a sequence having one or several amino acid substitutions, deletions, or additions (e.g., 1, 2, 3, 4, or 5 amino acid substitutions, deletions, or additions) as compared to the sequence set forth in any one of SEQ ID NOs: 7, 8, 9, or 10; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in any one of SEQ ID NOs: 7, 8, 9, or 10;
preferably, the substitution is a conservative substitution.

7. An antibody or antigen-binding fragment thereof capable of specifically binding to CCR8, comprising:
(a) a heavy chain variable region (VH) comprising the following three complementarity determining regions (CDRs):
(i) a VH CDR1, consisting of the sequence set forth in SEQ ID NO: 137, or a sequence having one or several amino acid substitutions, deletions, or additions (e.g., 1, 2, or 3 amino acid substitutions, deletions, or additions) as compared to SEQ ID NO: 137,
(ii) a VH CDR2, consisting of the sequence set forth in SEQ ID NO: 138, or a sequence having one or several amino acid substitutions, deletions, or additions (e.g., 1, 2, or 3 amino acid substitutions, deletions, or additions) as compared to SEQ ID NO: 138, and
(iii) a VH CDR3, consisting of the sequence set forth in SEQ ID NO: 139, or a sequence having one or several amino acid substitutions, deletions, or additions (e.g., 1, 2, or 3 amino acid substitutions, deletions, or additions) as compared to SEQ ID NO: 139;
and/or,
(b) a light chain variable region (VL) comprising the following three complementarity determining regions (CDRs):
(iv) a VL CDR1, consisting of the sequence of SEQ ID NO: 75, or a sequence having one or several amino acid substitutions, deletions, or additions (e.g., 1, 2, or 3 amino acid substitutions, deletions, or additions) as compared to SEQ ID NO: 75,
(v) a VL CDR2, consisting of the sequence of SEQ ID NO: 76, or a sequence having one or several amino acid substitutions, deletions, or additions (e.g., 1, 2, or 3 amino acid substitutions, deletions, or additions) as compared to SEQ ID NO: 76, and
(vi) a VL CDR3, consisting of the sequence of SEQ ID NO: 77, or a sequence having one or several amino acid substitutions, deletions, or additions (e.g., 1, 2, or 3 amino acid substitutions, deletions, or additions) as compared to SEQ ID NO: 77;
preferably, wherein the substitution in any one of (i)-(vi) is a conservative substitution;
preferably, wherein the CDRs in any one of (i)-(vi) are defined according to the Kabat, IMGT, or Chothia numbering system;
preferably, wherein the CDRs in any one of (i)-(vi) are defined according to the IMGT numbering system.

8. The antibody or antigen-binding fragment thereof of claim 7, comprising:
the following three heavy chain CDRs: a VH CDR1 as set forth in SEQ ID NO: 137, a VH CDR2 as set forth in SEQ ID NO: 138, a VH CDR3 as set forth in SEQ ID NO: 139; and/or, the following three light chain CDRs: VL CDR1 as set forth in SEQ ID NO: 75, VL CDR2 as set forth in SEQ ID NO: 76, VL CDR3 as set forth in SEQ ID NO: 77;
preferably, wherein the antibody or antigen-binding fragment thereof further comprises a framework region of a human immunoglobulin.

9. The antibody or antigen-binding fragment thereof of claim 7 or 8, wherein the antibody or antigen-binding fragment thereof comprises:
(a) a heavy chain variable region (VH) comprising an amino acid sequence selected from the group consisting of:
(i) the sequence set forth in SEQ ID NO: 78 or 1;
(ii) a sequence having one or several amino acid substitutions, deletions, or additions (e.g., 1, 2, 3, 4, or 5 amino acid substitutions, deletions, or additions) as compared to the sequence set forth in SEQ ID NO: 78 or 1; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 78 or 1;
and/or
(b) a light chain variable region (VL) comprising an amino acid sequence selected from the group consisting of:
(iv) the sequence set forth in SEQ ID NO: 2;
(v) a sequence having one or several amino acid substitutions, deletions, or additions (e.g., 1, 2, 3, 4, or 5 amino acid substitutions, deletions, or additions) as compared to the sequence set forth in SEQ ID NO: 2; or
(vi) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence set forth in SEQ ID NO: 2;
preferably, the substitution in (ii) or (v) is a conservative substitution;
preferably, the antibody or antigen-binding fragment thereof comprises a VH having the sequence set forth in SEQ ID NO: 78 or 1 and a VL having the sequence set forth in SEQ ID NO: 2.

10. The antibody or antigen-binding fragment thereof of any one of claims 1-9, wherein the antibody or antigen-binding fragment thereof further comprises a constant region derived from a human immunoglobulin;
preferably, the heavy chain of the antibody or antigen-binding fragment thereof comprises a heavy chain constant region derived from a human immunoglobulin (e.g., IgG1, IgG2, IgG3, or IgG4) or a variant thereof;
preferably, the heavy chain constant region has a LALA mutation and/or a knob-into-hole modification;
preferably, the heavy chain constant region has the sequence set forth in any one of SEQ ID NOs: 12-17, 79-82;
preferably, the light chain of the antibody or antigen-binding fragment thereof comprises a light chain constant region derived from a human immunoglobulin (e.g., κ or λ);
preferably, the light chain constant region has the sequence set forth in any one of SEQ ID NOs: 11, 81, 82;
preferably, the antibody or antigen-binding fragment thereof is hypofucosylated or afucosylated.

11. The antibody or antigen-binding fragment thereof of any one of claims 1-3 or 7-9, wherein the antigen-binding fragment is selected from Fab, Fab', (Fab')2, Fv, disulfide-linked Fv, scFv, diabody, and single domain antibody (sdAb); and/or, the antibody is a murine antibody, chimeric antibody, humanized antibody, or multispecific antibody.

12. An isolated nucleic acid molecule encoding the antibody or antigen-binding fragment thereof of any one of claims 1-3 or 7-9 or the single domain antibody or antigen-binding fragment thereof of any one of claims 4-6.

13. A vector comprising the nucleic acid molecule of claim 12; preferably, wherein the vector is a cloning vector or an expression vector.

14. A host cell comprising the nucleic acid molecule of claim 12 or the vector of claim 13;
preferably, wherein the host cell is a mammalian cell;
preferably, wherein the host cell has low or no fucosylation activity, for example the host cell is selected from a mammalian cell (e.g., CHO cell) lacking expression of a gene encoding fucosyltransferase.

15. A method of preparing the antibody or antigen-binding fragment thereof of any one of claims 1-3 or 7-9 or the single domain antibody or antigen-binding fragment thereof of any one of claims 4-6, comprising culturing the host cell of claim 14 under conditions that allow expression of the antibody or antigen-binding fragment thereof, and recovering the antibody or antigen-binding fragment thereof from the cultured host cell culture;
preferably, the host cell has low or no fucosylation activity, for example the host cell is selected from a mammalian cell (e.g., CHO cell) lacking expression of a gene encoding fucosyltransferase.

16. A multispecific antibody comprising the antibody or antigen-binding fragment thereof of any one of claims 1-3 or 7-9 or the single domain antibody or antigen-binding fragment thereof of any one of claims 4-6.

17. A multispecific antibody (e.g., a bispecific antibody) that specifically binds to CCR8 and CTLA4, comprising a first antigen-binding domain specific for CTLA4 and a second antigen-binding domain specific for CCR8.

18. The multispecific antibody of claim 17, wherein the first antigen-binding domain comprises: the antibody or antigen-binding fragment thereof of any one of claims 1-3 or the single domain antibody or antigen-binding fragment thereof of any one of claims 4-6.

19. The multispecific antibody of claim 17 or 18, wherein the second antigen-binding domain comprises: the antibody or antigen-binding fragment thereof of any one of claims 7-9.

20. The multispecific antibody of any one of claims 17-19, wherein the first antigen-binding domain is a Fab fragment, a scFv, or a VHH, and the second antigen-binding domain is a Fab fragment, a scFv, or a VHH;
preferably, the first antigen-binding domain is a Fab fragment or a VHH, and the second antigen-binding domain is a Fab fragment.

21. The multispecific antibody of any one of claims 17-20, further comprising an Fc domain comprising a first monomer and a second monomer;
preferably, wherein the first antigen-binding domain and the second antigen-binding domain are linked to the N-terminus of the first monomer and the second monomer of the Fc domain, respectively, optionally via a linker.

22. The multispecific antibody of claim 21, wherein the Fc domain comprises a modification to promote dimerization of the first monomer and the second monomer;
preferably, the modification comprises an amino acid substitution in the CH3 domain of the Fc domain.

23. The multispecific antibody of claim 21 or 22, wherein the Fc domain comprises a modification to promote controlled Fab-arm Exchange (cFAE);
preferably, the modification comprises an amino acid substitution in the CH3 domain of the Fc domain.

24. The multispecific antibody of any one of claims 21-23, wherein the modification comprises a "knob" modification in one of the two monomers of the Fc domain and a "hole" modification in the other of the two monomers of the Fc domain to form a "knob-into-hole" modification;
preferably, the two monomers of the Fc domain comprise the amino acid sequences set forth in SEQ ID NOs: 16 and 17, respectively, or comprise the amino acid sequences set forth in SEQ ID NOs: 79 and 80, respectively.

25. The multispecific antibody of any one of claims 21-23, wherein the modification comprises a substitution selected from F405L and/or R411T in one of the two monomers of the Fc domain and a substitution selected from T370K and/or K409R in the other of the two monomers of the Fc domain to form a modification that promotes cFAE;
preferably, the two monomers of the Fc domain comprise the amino acid sequences set forth in SEQ ID NOs: 13 and 14, respectively.

26. The multispecific antibody of any one of claims 17-25, which is hypofucosylated or afucosylated.

27. The multispecific antibody of any one of claims 17-26, wherein the first antigen-binding domain is a Fab fragment, the second antigen-binding domain is a Fab fragment, and the multispecific antibody comprises:
(i) a first peptide chain comprising a VL of the first antigen-binding domain and a first light chain constant region (CL); preferably, the first CL is kappa;
(ii) a second peptide chain comprising a VH of the first antigen-binding domain and a first heavy chain constant region (CH); preferably, the first CH is IgG, e.g., IgG1, IgG2, IgG3, or IgG4;
(iii) a third peptide chain comprising a VH of the second antigen-binding domain and a second heavy chain constant region (CH); preferably, the second CH is IgG, e.g., IgG1, IgG2, IgG3, or IgG4;
(iv) a fourth peptide chain comprising a VL of the second antigen-binding domain and a second light chain constant region (CL); preferably, the second CL is kappa;
preferably, the Fc domain monomers of the first CH and the second CH form a dimer; preferably, the Fc domain comprises the modification defined in claim 24 or 25;
preferably, the first CH and first CL, and the second CH and second CL are each capable of forming a dimer.

28. The multispecific antibody of claim 27, wherein the multispecific antibody comprises:
(i) a first peptide chain comprising a VL of the sequence set forth in SEQ ID NO: 4 and a CL of the sequence set forth in SEQ ID NO: 11, 82, 81, 143;
(ii) a second peptide chain comprising a VH of the sequence set forth in SEQ ID NO: 3 and a CH of the sequence set forth in SEQ ID NO: 13, 80, 79, 140;
(iii) a third peptide chain comprising a VH of the sequence set forth in SEQ ID NO: 1, 78 and a CH of the sequence set forth in SEQ ID NO: 14, 79, 80, 141;
(iv) a fourth peptide chain comprising a VL of the sequence set forth in SEQ ID NO: 2 and a CL of the sequence set forth in SEQ ID NO: 11, 81, 82, 143;
preferably, the multispecific antibody comprises a first peptide chain comprising the sequence set forth in SEQ ID NO: 29, a second peptide chain comprising the sequence set forth in SEQ ID NO: 28, a third peptide chain comprising the sequence set forth in SEQ ID NO: 30, and a fourth peptide chain comprising the sequence set forth in SEQ ID NO: 31;
preferably, the multispecific antibody comprises a first peptide chain comprising the sequence set forth in SEQ ID NO: 86, a second peptide chain comprising the sequence set forth in SEQ ID NO: 85, a third peptide chain comprising the sequence set forth in SEQ ID NO: 87, and a fourth peptide chain comprising the sequence set forth in SEQ ID NO: 88;
preferably, the multispecific antibody comprises a first peptide chain comprising the sequence set forth in SEQ ID NO: 96, a second peptide chain comprising the sequence set forth in SEQ ID NO: 95, a third peptide chain comprising the sequence set forth in SEQ ID NO: 93, and a fourth peptide chain comprising the sequence set forth in SEQ ID NO: 94;
preferably, the multispecific antibody comprises a first peptide chain comprising the sequence set forth in SEQ ID NO: 100, a second peptide chain comprising the sequence set forth in SEQ ID NO: 99, a third peptide chain comprising the sequence set forth in SEQ ID NO: 97, and a fourth peptide chain comprising the sequence set forth in SEQ ID NO: 98;
preferably, the multispecific antibody comprises a first peptide chain comprising the sequence set forth in SEQ ID NO: 110, a second peptide chain comprising the sequence set forth in SEQ ID NO: 109, a third peptide chain comprising the sequence set forth in SEQ ID NO: 111, and a fourth peptide chain comprising the sequence set forth in SEQ ID NO: 112;
preferably, the multispecific antibody comprises a first peptide chain comprising the sequence set forth in SEQ ID NO: 114, a second peptide chain comprising the sequence set forth in SEQ ID NO: 113, a third peptide chain comprising the sequence set forth in SEQ ID NO: 115, and a fourth peptide chain comprising the sequence set forth in SEQ ID NO: 116;
preferably, the multispecific antibody comprises a first peptide chain comprising the sequence set forth in SEQ ID NO: 150, a second peptide chain comprising the sequence set forth in SEQ ID NO: 148, a third peptide chain comprising the sequence set forth in SEQ ID NO: 149, and a fourth peptide chain comprising the sequence set forth in SEQ ID NO: 151;
preferably, the multispecific antibody comprises a first peptide chain comprising the sequence set forth in SEQ ID NO: 154, a second peptide chain comprising the sequence set forth in SEQ ID NO: 152, a third peptide chain comprising the sequence set forth in SEQ ID NO: 153, and a fourth peptide chain comprising the sequence set forth in SEQ ID NO: 155.

29. The multispecific antibody of claim 27, wherein the multispecific antibody comprises:
(i) a first peptide chain comprising a VL of the sequence set forth in SEQ ID NO: 6 and a CL of the sequence set forth in SEQ ID NO: 11, 82, 81, 143;
(ii) a second peptide chain comprising a VH of the sequence set forth in SEQ ID NO: 5 and a CH of the sequence set forth in SEQ ID NO: 13, 80, 79, 140;
(iii) a third peptide chain comprising a VH of the sequence set forth in SEQ ID NO: 1, 78 and a CH of the sequence set forth in SEQ ID NO: 14, 79, 80, 141;
(iv) a fourth peptide chain comprising a VL of the sequence set forth in SEQ ID NO: 2 and a CL of the sequence set forth in SEQ ID NO: 11, 81, 82, 143;
preferably, the multispecific antibody comprises a first peptide chain comprising the sequence set forth in SEQ ID NO: 33, a second peptide chain comprising the sequence set forth in SEQ ID NO: 32, a third peptide chain comprising the sequence set forth in SEQ ID NO: 34, and a fourth peptide chain comprising the sequence set forth in SEQ ID NO: 35;
preferably, the multispecific antibody comprises a first peptide chain comprising the sequence set forth in SEQ ID NO: 90, a second peptide chain comprising the sequence set forth in SEQ ID NO: 89, a third peptide chain comprising the sequence set forth in SEQ ID NO: 91, and a fourth peptide chain comprising the sequence set forth in SEQ ID NO: 92;
preferably, the multispecific antibody comprises a first peptide chain comprising the sequence set forth in SEQ ID NO: 104, a second peptide chain comprising the sequence set forth in SEQ ID NO: 103, a third peptide chain comprising the sequence set forth in SEQ ID NO: 101, and a fourth peptide chain comprising the sequence set forth in SEQ ID NO: 102;
preferably, the multispecific antibody comprises a first peptide chain comprising the sequence set forth in SEQ ID NO: 108, a second peptide chain comprising the sequence set forth in SEQ ID NO: 107, a third peptide chain comprising the sequence set forth in SEQ ID NO: 105, and a fourth peptide chain comprising the sequence set forth in SEQ ID NO: 106;
preferably, the multispecific antibody comprises a first peptide chain comprising the sequence set forth in SEQ ID NO: 118, a second peptide chain comprising the sequence set forth in SEQ ID NO: 117, a third peptide chain comprising the sequence set forth in SEQ ID NO: 119, and a fourth peptide chain comprising the sequence set forth in SEQ ID NO: 120;
preferably, the multispecific antibody comprises a first peptide chain comprising the sequence set forth in SEQ ID NO: 122, a second peptide chain comprising the sequence set forth in SEQ ID NO: 121, a third peptide chain comprising the sequence set forth in SEQ ID NO: 123, and a fourth peptide chain comprising the sequence set forth in SEQ ID NO: 124;
preferably, the multispecific antibody comprises a first peptide chain comprising the sequence set forth in SEQ ID NO: 158, a second peptide chain comprising the sequence set forth in SEQ ID NO: 156, a third peptide chain comprising the sequence set forth in SEQ ID NO: 157, and a fourth peptide chain comprising the sequence set forth in SEQ ID NO: 159.

30. The multispecific antibody of any one of claims 17-26, wherein the first antigen-binding domain is a VHH, the second antigen-binding domain is a Fab fragment, and the multispecific antibody comprises:
(i) a first peptide chain comprising a VL of the second antigen-binding domain and a light chain constant region (CL); preferably, the CL is a kappa light chain constant region;
(ii) a second peptide chain comprising a VH of the second antigen-binding domain and a heavy chain constant region (CH); preferably, the CH is IgG, e.g., IgG1, IgG2, IgG3, or IgG4;
and
(iii) a third peptide chain comprising the first antigen-binding domain and an Fc domain monomer; preferably, the Fc domain monomer is IgG, e.g., IgG1, IgG2, IgG3, or IgG4; preferably, the Fc domain monomer comprises a hinge region, CH2, and CH3; preferably, the first antigen-binding domain is linked to the N-terminus of the Fc domain monomer via a linker (e.g., a peptide linker comprising one or more glycine (G) and/or serine (S) residues);
preferably, the Fc domain monomer of the third peptide chain is capable of forming a dimer with the Fc domain of the heavy chain constant region (CH) of the second peptide chain; preferably, the Fc domain comprises the modification defined in claim 21;
preferably, the CL of the first peptide chain is capable of forming a dimer with the CH of the second peptide chain.

31. The multispecific antibody of claim 30, wherein the multispecific antibody comprises:
(i) a first peptide chain comprising a VL of the sequence set forth in SEQ ID NO: 2 and a CL of the sequence set forth in SEQ ID NO: 11;
(ii) a second peptide chain comprising a VH of the sequence set forth in SEQ ID NO: 1, 78 and a CH of the sequence set forth in SEQ ID NO: 17; and
(iii) a third peptide chain comprising a VHH of the sequence set forth in SEQ ID NO: 7, 8, 9, 10 and an Fc domain monomer of the sequence set forth in SEQ ID NO: 16.

32. The multispecific antibody of claim 30, wherein the multispecific antibody comprises:
(1) a first peptide chain comprising the sequence set forth in SEQ ID NO: 38, a second peptide chain comprising the sequence set forth in SEQ ID NO: 37, and a third peptide chain comprising the sequence set forth in SEQ ID NO: 36;
(2) a first peptide chain comprising the sequence set forth in SEQ ID NO: 41, a second peptide chain comprising the sequence set forth in SEQ ID NO: 40, and a third peptide chain comprising the sequence set forth in SEQ ID NO: 39;
(3) a first peptide chain comprising the sequence set forth in SEQ ID NO: 44, a second peptide chain comprising the sequence set forth in SEQ ID NO: 43, and a third peptide chain comprising the sequence set forth in SEQ ID NO: 42;
(4) a first peptide chain comprising the sequence set forth in SEQ ID NO: 47, a second peptide chain comprising the sequence set forth in SEQ ID NO: 46, and a third peptide chain comprising the sequence set forth in SEQ ID NO: 45;
(5) a first peptide chain comprising the sequence set forth in SEQ ID NO: 127, a second peptide chain comprising the sequence set forth in SEQ ID NO: 126, and a third peptide chain comprising the sequence set forth in SEQ ID NO: 125;
(6) a first peptide chain comprising the sequence set forth in SEQ ID NO: 130, a second peptide chain comprising the sequence set forth in SEQ ID NO: 129, and a third peptide chain comprising the sequence set forth in SEQ ID NO: 128;
(7) a first peptide chain comprising the sequence set forth in SEQ ID NO: 133, a second peptide chain comprising the sequence set forth in SEQ ID NO: 132, and a third peptide chain comprising the sequence set forth in SEQ ID NO: 131;
(8) a first peptide chain comprising the sequence set forth in SEQ ID NO: 136, a second peptide chain comprising the sequence set forth in SEQ ID NO: 135, and a third peptide chain comprising the sequence set forth in SEQ ID NO: 134.

33. An isolated nucleic acid molecule comprising a nucleotide sequence encoding the multispecific antibody of any one of claims 17-32 or at least one peptide chain thereof.

34. A vector comprising the isolated nucleic acid molecule of claim 33;
preferably, wherein the vector comprises nucleotide sequences encoding the respective peptide chains of the multispecific antibody, and the nucleotide sequences encoding the respective peptide chains are present on the same or different vectors.

35. A host cell comprising the isolated nucleic acid molecule of claim 32 or the vector of claim 30;
preferably, wherein the host cell has low or no fucosylation activity, for example, the host cell may be a mammalian cell such as a CHO cell lacking expression of a gene encoding fucosyltransferase (e.g., FUT8),.

36. A method of preparing the multispecific antibody of any one of claims 17-32, comprising culturing the host cell of claim 35 under conditions that allow protein expression, and recovering the multispecific antibody from the cultured host cell culture.

37. The method of claim 36, wherein the host cell has low or no fucosylation activity, thereby producing a hypofucosylated or afucosylated antibody; preferably, the host cell may be a mammalian cell such as a CHO cell lacking expression of a gene encoding fucosyltransferase (e.g., FUT8).

38. An immunoconjugate comprising the antibody or antigen-binding fragment thereof of any one of claims 1-3, 7-9 or the single domain antibody or antigen-binding fragment thereof of any one of claims 4-6 or the multispecific antibody of any one of claims 17-32, and a therapeutic agent linked to the antibody or antigen-binding fragment thereof or the multispecific molecule;
preferably, wherein the therapeutic agent is selected from a cytotoxic agent;
preferably, wherein the therapeutic agent is selected from an alkylating agent, a mitotic inhibitor, an anti-tumor antibiotic, an antimetabolite, a topoisomerase inhibitor, a tyrosine kinase inhibitor, a radionuclide agent, and any combination thereof;
preferably, wherein the immunoconjugate is an antibody-drug conjugate (ADC).

39. A pharmaceutical composition comprising the antibody or antigen-binding fragment thereof of any one of claims 1-3, 7-9, or the single domain antibody or antigen-binding fragment thereof of any one of claims 4-6, or the multispecific antibody of any one of claims 17-32, and a pharmaceutically acceptable carrier and/or excipient;
preferably, wherein the pharmaceutical composition further comprises an additional pharmaceutically active agent;
preferably, wherein the additional pharmaceutically active agent is a drug having anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeting drug, an immune checkpoint inhibitor, or an oncolytic virus; preferably, wherein the pharmaceutical composition further comprises an anti-PD-1 antibody;
preferably, wherein the pharmaceutical composition comprises the multispecific antibody of any one of claims 17-32 and an anti-PD-1 antibody.

40. A kit comprising the antibody or antigen-binding fragment thereof of any one of claims 1-3, 7-9 or the single domain antibody or antigen-binding fragment thereof of any one of claims 4-6 or the multispecific antibody of any one of claims 17-32;
preferably, wherein the antibody or antigen-binding fragment thereof has a detectable label, such as an enzyme (e.g., horseradish peroxidase), a radionuclide, a fluorescent dye, a luminescent substance (e.g., a chemiluminescent substance), or biotin;
preferably, wherein the multispecific antibody has a detectable label, such as an enzyme (e.g., horseradish peroxidase), a radionuclide, a fluorescent dye, a luminescent substance (e.g., a chemiluminescent substance), or biotin.

41. A method of inhibiting the growth of and/or killing a tumor cell expressing CCR8 and/or CTLA4, comprising contacting the tumor cell with an effective amount of the antibody or antigen-binding fragment thereof of any one of claims 1-3, 7-9, or the single domain antibody or antigen-binding fragment thereof of any one of claims 4-6, or the multispecific antibody of any one of claims 17-32, or the immunoconjugate of claim 38, or the pharmaceutical composition of claim 39.

42. Use of the antibody or antigen-binding fragment thereof of any one of claims 1-3, 7-9, or the single domain antibody or antigen-binding fragment thereof of any one of claims 4-6, or the multispecific molecule of any one of claims 17-32, or the immunoconjugate of claim 38, or the pharmaceutical composition of claim 39 for the manufacture of a medicament for:
(1) increasing immune cell activity *in vitro* or *in vivo* in a subject;
(2) enhancing an immune response in a subject;
(3) preventing and/or treating a tumor in a subject; or
(4) preventing and/or treating an infection in a subject;
preferably, wherein the tumor expresses CCR8 and/or CTLA4;
preferably, wherein the CCR8 and/or CTLA4 is expressed on the surface of the tumor cell;
preferably, wherein the tumor is selected from non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoides, Merkel cell carcinoma, and other hematologic malignancies, such as classical Hodgkin's lymphoma (CHL), primary mediastinal large B-cell lymphoma, T-cell/histiocyte rich B-cell lymphoma, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma;
preferably, wherein the infection is selected from a viral infection, a bacterial infection, a fungal infection, and a parasitic infection;
preferably, wherein the subject is a mammal, such as a human, a cynomolgus monkey, or a mouse.

43. Use of the antibody or antigen-binding fragment thereof of any one of claims 1-3, 7-9 or the single domain antibody or antigen-binding fragment thereof of any one of claims 4-6 or the multispecific antibody of any one of claims 17-32 for the manufacture of a kit for detecting whether a tumor may be treated by an anti-tumor therapy targeting CCR8 and/or CTLA4;
(1) contacting a sample containing the tumor cells with the antibody or antigen-binding fragment thereof of any one of claims 1-3, 7-9 or the single domain antibody or antigen-binding fragment thereof of any one of claims 4-6;
(2) detecting the formation of a complex between the antibody or antigen-binding fragment thereof and CCR8 and/or CTLA4;
preferably, wherein the antibody or antigen-binding fragment thereof has a detectable label;
preferably, wherein the CCR8 and/or CTLA4 is mammalian (e.g., human, monkey) CCR8 and/or CTLA4;
preferably, wherein the tumor is selected from non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoides, Merkel cell carcinoma, and other hematologic malignancies, such as classical Hodgkin's lymphoma (CHL), primary mediastinal large B-cell lymphoma, T-cell/histiocyte rich B-cell lymphoma, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin's lymphoma, central nervous system (CNS) tumors, such as primary CNS lymphoma, spinal axis tumor, brainstem glioma.
